(19) Europäisches Patentamt
European Patent Office
Office européen des brevets

(11) EP 2 592 094 A1

(12) EUROPEAN PATENT APPLICATION

(43) Date of publication:
15.05.2013 Bulletin 2013/20

(51) Int Cl.:
*C07K 16/42* (2006.01) *A61K 39/395* (2006.01)
*A61P 37/08* (2006.01)

(21) Application number: 13151217.0

(22) Date of filing: 15.02.2008

(84) Designated Contracting States:
AT BE BG CH CY CZ DE DK EE ES FI FR GB GR HR HU IE IS IT LI LT LU LV MC MT NL NO PL PT RO SE SI SK TR

(30) Priority: 15.02.2007 US 901305 P

(62) Document number(s) of the earlier application(s) in accordance with Art. 76 EPC:
08709415.7 / 2 121 762

(27) Previously filed application:
15.02.2008 PCT/GB2008/000524

(71) Applicants:
- **AstraZeneca AB**
  **151 85 Södertälje (SE)**
- **MEDIMMUNE LIMITED**
  **Cambridge**
  **Cambridgeshire CB21 6GH (GB)**

(72) Inventors:
- **Cochrane, Duncan**
  **Cambridge**
  **Cambridgeshire CB21 6GH (GB)**
- **Cohen, Suzanne**
  **Cambridge**
  **Cambridgeshire CB21 6GH (GB)**
- **Dobson, Claire Louise**
  **Cambridge**
  **Cambridgeshire CB21 6GH (GB)**
- **Eriksson, Per-Olof Fredrik**
  **S-221 87 Lund (SE)**
- **Monk, Phillip David**
  **Southampton**
  **Hampshire SO16 6YD (GB)**
- **Von Wachenfeldt, Karin**
  **S-221 87 Lund (SE)**

(74) Representative: **Bates, Rosica Florence**
**MedImmune Ltd.**
**Milstein Building**
**Granta Park**
**Cambridge CB21 6GH (GB)**

Remarks:
- The complete document including Reference Tables and the Sequence Listing can be downloaded from the EPO website
- This application was filed on 14-01-2013 as a divisional application to the application mentioned under INID code 62.

# (54) Binding members for IgE molecules

(57) This invention relates to binding members, especially antibody molecules, for IgE. The binding members are useful for, inter alia, treatment of disorders mediated by IgE including allergies and asthma.

EP 2 592 094 A1

## Description

### Field of the Invention

This invention relates to binding members, especially antibody molecules, for IgE. The binding members are useful for, *inter alia,* treatment of disorders mediated by IgE including allergies and asthma.

IgE is a member of the immunoglobulin family and mediates allergic responses such as asthma, food allergies, type 1 hypersensitivity and sinus inflammation.

IgE is secreted by, and expressed on the surface of, B-cells. Briefly, IgE is anchored in the B-cell membrane by a transmembrane domain that is linked to the mature IgE molecule through a short membrane-binding region. IgE may also be bound via its Fc region to B-cells, monocytes, eosinophils and platelets through a low affinity IgE receptor (FcεRII, also known as CD23). Upon exposure to an allergen, B-cells that produce allergen-specific IgE are clonally amplified. Allergen-specific IgE is then released info the circulation by the B-cells where it is in turn bound by B-cells through the FcεRII, as well as by mast cells and basophils through a high affinity receptor (FεFRI). Such mast cells and basophils are thereby sensitized for allergen. Subsequent exposure to the allergen cross-links the FcεRI on mast cells and basophils thereby activating their release of histamine and other factors responsible for clinical hypersensitivity and anaphylaxis.

Binding members that inhibit binding to and functional activity through *FcERI* with or without simultaneous inhibition of FcERII are useful for inhibiting IgE-mediated disease conditions, such as allergies and asthma.

It is generally understood that FcεRI and FcεRII bind to recognition site(s) in the IgE constant (Fc) domain. Various studies have been undertaken to identify these recognition sites. For example, peptides corresponding to specific portions of the IgE molecule have been used as either competitive inhibitors of IgE-receptor binding (Burt et al., Eur. J. Immun, 17:437-440 [1987]; Helm et al., Nature, 331:180-183 [1988]; Helm et al., Proc. Natl. Acad. Sci., 86:9465-9469 [1989]; Vercelli et al., Nature, 338:649-651 [1989]; Nio et al., Peptide Chemistry, 203-208 [1990]), or to elicit anti-IgE antibodies that might block IgE-receptor interaction (Burt et al., Molec. Immun. 24:379-389 [1987]; Robertson et al., Molec. Immun., 25:103-113 [1988]; Baniyash et al., Molec. Immun. 25:705-711 [1988]).

More recently, Xolair® (Omalizumab) has been produced and marketed for treating asthma patients. Xolair® is a humanized IgG1k monoclonal antibody that selectively binds to human IgE, thereby reducing the binding of IgE to at least FcεRI on the surface of mast cells and basophils. By reducing surface-bound IgE on FcεRI-bearing cells, Xolair® reduces somewhat the degree of release of mediators of the allergic response. Xolair® is disclosed in International patent application publication numbers: WO 93/04173 and WO 97/04807.

However, other binding members for IgE, such as those with a higher affinity and/or potency than Xolair®, are needed to improve this promising therapeutic strategy.

### The Invention

By utilising appropriately designed selection techniques and assays, we have developed binding members for IgE that inhibit binding to FcεRI, the high-affinity IgE receptor present on mast cells.

A binding member of the invention inhibits binding of IgE to FcεRI. The inhibition of binding may be by direct inhibition, for example, by neutralizing IgE. The binding member of the invention typically neutralizes human IgE with an IC50 of less than about 10 nM as determined by an RBL-ER51 calcium signalling assay, and in another embodiment, with an IC50 less than about 3.0 nM, or less than about 1 nM, or less than about 0.5 nM as determined by an RBL-ER51 calcium signalling assay.

In another embodiment of the invention there is provided an isolated binding member specific for immunoglobulin E which binding member has an IC50 geomean for inhibition of calcium signalling induced by 25ng/ml IgE in RBL-ER51 cells of less than 1nM, or alternatively less than 0.6nM, less than 0.5nM, less than 0.4nM, less than 0.3nM, less than 0.25nM or less than 0.2nM.

The binding members of the invention may also bind to and neutralize non-human IgE, meaning IgE orthologs that occur naturally in species other than human.

Binding members of the invention are normally specific for IgE over other immunoglobulins, and thus bind IgE selectively. Such selectivity may be determined or demonstrated, for example, in a standard competition assay.

The binding members are useful for treating and/or preventing disorders that are mediated by IgE, especially allergies and asthma.

The binding members are useful for reducing circulating free IgE in a mammal, and useful for inhibiting allergen-induced mast cell degranulation either in vivo or in vitro.

The binding members are further useful for inhibiting biological responses mediated by FcεR1 with or without simultaneous inhibition of FcERII, either in, vivo or in vitro.

The binding members of the invention also have diagnostic utility, such as for detecting the presence or amount of IgE, or the presence or amount of allergen-specific IgE, in a sample of interest, such as a sample from an asthmatic or allergic patient.

The binding member of the invention binds to an epitope of IgE that is distinct from that of Xolair®. Thus, when compared with Xolair®, the binding members of the invention differ in their abilities to compete with other anti-IgE antibodies for binding to IgE.

Any suitable method may be used to determine the sequence of residues bound by a binding member. For example, a peptide-binding scan may be used, such as a PEPSCAN-based enzyme linked immuno assay (ELISA) as described in detail elsewhere herein. In a peptide-binding scan, such as the kind provided by PEPSCAN Systems, short overlapping peptides derived from the antigen are systematically screened for binding to a binding member. The peptides may be covalently coupled to a support surface to form an array of peptides. Peptides may be in a linear or constrained conformation. A constrained conformation may be produced using peptides having a terminal Cys residue at each end of the peptide sequence. The Cys residues can be covalently coupled directly or indirectly to a a support surface such that the peptide is held in a looped conformation. Thus, peptides used in the method may have Cys residues added to each end of a peptide sequence corresponding to a fragment of the antigen. Double looped peptides may also be used, in which a Cys residue is additionally located at or near the middle of the peptide sequence. The Cys residues can be covalently coupled directly or indirectly to a support surface such that the peptides form a double-looped conformation, with one loop on each side of the central Cys residue. Peptides can be synthetically generated, and Cys residues can therefore be engineered at desired locations, despite not occurring naturally in the IgE sequence. Optionally, linear and constrained peptides may both be screened in a peptide-binding assay. A peptide-binding scan may involve identifying (e.g. using ELISA) a set of peptides to which the binding member binds, wherein the peptides have amino acid sequences corresponding to fragments of IgE (e.g. peptides of about 5, 10 or 15 contiguous residues of IgE), and aligning the peptides in order to determine a footprint of residues bound by the binding member, where the footprint comprises residues common to overlapping peptides.

Alternatively or additionally the peptide-binding scan method may involve identifying peptides to which the binding member binds with at least a given signal:noise ratio. Details of a suitable peptide-binding scan method for determining binding are known in the art. Other methods that are well known in the art and that could be used to determine the residues bound by an antibody, and/or to confirm peptide-binding scan results, include site directed mutagenesis, hydrogen deuterium exchange, mass spectrometry, NMR, and X-ray crystallography.

A binding member of the invention may or may not bind and/or neutralise IgE variants. Thus, a binding member of the invention may or may not inhibit binding of IgE variants to FcεRI with or without simultaneous inhibition of binding of IgE variants to FcERII.

Linear epitope sequences of IgE, e.g. as isolated peptide fragments or polypeptides comprising them, may be employed to identify, generate, isolate and/or test binding members of the present invention.

As described in more detail below, binding members according to the invention have been shown to neutralise IgE with high potency. Neutralisation means inhibition of a biological activity of IgE. Binding members of the invention may neutralise one or more biological activities of IgE, but typically inhibit IgE binding to FcεRI.

Neutralisation of IgE binding to FCεRI with or without simultaneous neutralisation of IgE binding to FcERII may optionally be measured as a function of the biological activity of the receptor, such as allergen-induced mast cell degranulation.

Suitable assays for measuring neutralisation of IgE by binding members of the invention include ligand receptor biochemical assays and surface plasmon resonance (SPR), e.g. BIACORE.

Inhibition of biological activity may be partial or total. Binding members may inhibit an IgE biological activity, such receptor binding or mast cell degranulation, by 100%, or alternatively by: at least 95 %, at least 90 %, at least 85 %, at least 80 %, at least 75 %, at least 70 %, at least 60 %, or at least 50 % of the activity in absence of the binding member.

The neutralising potency of a binding member is normally expressed as an $IC_{50}$ value, in nM unless otherwise stated. In functional assays, $IC_{50}$ is the concentration of a binding member that reduces a biological response by 50 % of its maximum. In ligand-binding studies, $IC_{50}$ is the concentration that reduces receptor binding by 50 % of maximal specific binding level. $IC_{50}$ may be calculated by plotting % of maximal biological response as a function of the log of the binding member concentration, and using a software program, such as Prism (GraphPad) to fit a sigmoidal function to the data to generate $IC_{50}$ values. Potency may be determined or measured using one or more assays known to the skilled person and/or as described or referred to herein.

The neutralising potency of a binding member can be expressed as a geomean. Geomean (also known as geometric mean), as used herein means the average of the logarithmic values of a data set, converted back to a base 10 number. This requires there to be at least two measurements, e.g. at least 2, preferably at least 5, more preferably at least 10 replicate. The person skilled in the art will appreciate that the greater the number of replicates the more robust the geomean value will be. The choice of replicate number can be left to the discretion of the person skilled in the art.

Neutralisation of IgE activity by a binding member in an assay described herein, indicates that the binding member binds to and neutralises IgE. Other methods that may be used for determining binding of a binding member to IgE include ELISA, Western blotting, immunoprecipitation, affinity chromatography and biochemical assays.

Neutralising potency of a binding member as calculated in an assay using IgE from a first species (e.g. human) may be compared with neutralising potency of the binding member in a similar assay under similar conditions using IgE from a second species (e.g. cynomolgus monkey), in order to assess the extent of cross-reactivity of the binding member for IgE of the two species. Alternatively, cross-reactivity may be assessed in a competition binding assay, as described in more detail elsewhere herein.

A binding member of the invention may have a greater neutralising potency in a human IgE binding or biological assay than in a similar assay with IgE from a species other than human. Thus, neutralising potency of a binding member in an assay with human IgE may be greater than in a similar assay with IgE from a species other than human. Potency in a human IgE binding or biological assay may, for example, be about 10-fold greater than in a similar assay employing IgE of cynomolgus monkey, or in other embodiments, about 25-fold or about 125-fold greater. More specifically, potency in the human RBL-ER51 calcium signalling assay may be determined for a concentration of human IgE of 25 ng/ml, and compared to the potency using 100ng/ml of cynomolgus IgE under otherwise similar conditions. Examples of data obtained in similar RBL-ER51 calcium signaling assays using human IgE and cynomolgus IgE are shown in Table 5b.

A binding member of the invention may have a stronger affinity for human IgE than for IgE of other species. Affinity of a binding member for human IgE may be, for example, about 5-fold stronger than for cynomolgus monkey IgE, and in other embodiments, may be about 10-fold stronger.

A binding member of the invention may have an IgE-neutralising potency or $IC_{50}$ of less than about about 10 nM, with a 25 ng/ml concentration of human IgE. Alternatively, the $IC_{50}$ is less than about 3 nM. In other embodiments, the $IC_{50}$ is less than about 1 nM, or less than about 0.5 nM, or less than about 0.2 nM.

Binding kinetics and affinity (expressed as the equilibrium dissociation constant KD) of IgE-binding members for human IgE may be determined, e.g. using surface plasmon resonance (BIACORE). Binding members of the invention normally have an affinity for human IgE (KD) of less than about 80 nM, and in some embodiments have a KD of less than about 10 nM, in other embodiments less than 5nM, in other embodiments less than 2nM, in other embodiments less than 1 nM.. Affinity for cynomolgus monkey IgE is normally less than about 15 nM.

*In vivo* endogenous IgE may be glycosylated and therefore glycosylated human IgE is a therapeutic target for human therapy. While recombinant human IgE, which may be bacterially-derived and not glycosylated, may be used in assays described herein, binding members of the invention may bind glycosylated human IgE, such as IgE produced by a myeloma cell line such as U266.B1. This represents a significant advantage of binding members of the invention, since glycosylated human IgE is the target antigen for *in* vivo human applications.

A binding member of the invention may comprise an antibody molecule, preferably a human antibody molecule or a humanized antibody molecule. In one aspect of the invention, the antibody molecule is a monoclonal antibody.

An antigen binding site is generally formed by the variable heavy (VH) and variable light (VL) immunoglobulin domains; with the antigen-binding interface formed by six surface polypeptide loops, termed complimentarity determining regions (CDRs). There are three CDRs in each VH (HCDR1, HCDR2, and HCDR3) and in each VL (LCDR1, LCDR2, and LCDR3), together with framework regions (FRs).

The binding member of the invention normally comprises an antibody VH and/or VL domain. A VH domain of the invention comprises a set of HCDRs, and a VL domain comprises a set of LCDRs. An antibody molecule may comprise an antibody VH domain comprising a VH CDR1, CDR2 and CDR3 and a framework. It may alternatively or also comprise an antibody VL domain comprising a VL CDR1, CDR2 and CDR3 and a framework. Examples of antibody VH domains (SEQ ID NOS: 2, 298, 338, 318, 328, 118, 309, 28, 68, 8, 48, 288, 158, 268, 168, 38, 128, 78, 138, 188, 198, 98, 18, 88, 58, 108, 218, 248, 228, 238, 178; 208, 278, 148, 426, 278 and 258) and VL domains (SEQ ID NOS: 353, 358, 360, 362, 364, 366, 368, 370, 372, 374, 376, 378, 380, 382; 384, 386, 388, 390, 392, 394, 396,398,400,402,404,406,408,410,412,414,416,418,420,422,431, and 424) and CDRs (SEQ ID NOS: 3-5, 9-11, 14-16, 19-21, 24-26, 29-31, 34-36, 39-41, 44-46, 49-51, 54-56, 59-61, 64-66, 69-71, 74-76, 79-81, 84-86, 89-91, 94-96, 99-101, 104-106, 109-111, 114-116, 119-121, 124-126, 129-131, 134-136, 139-141, 144-146, 149-151, 154-156, 159-161, 164-166, 169-171, 174-176, 179-181, 184-186, 189-191, 194-196, 199-201, 204-206, 209-211, 214-216, 219-221, 224-226, 229-231, 234-236, 239-241, 244-246, 249-251, 254-256, 259-261, 264-266, 269-271, 274-276, 279-281, 284-286, 289-291, 294-296, 299-301, 304-306, 309-311, 314-316, 319-321, 324-326, 329-331, 334-336, 339-341, 344-346, 350-352, 354-356 and 427-429) according to the present invention are as listed in the appended sequence listing that forms part of the present disclosure. Further CDRs are disclosed below and in Table 1. All VH and VL sequences, CDR sequences, sets of CDRs and sets of HCDRs and sets of LCDRs disclosed herein represent aspects and embodiments of the invention.

As described herein, a "set of CDRs" comprises CDR1, CDR2 and CDR3. Thus, a set of HCDRs refers to HCDR1, HCDR2 and HCDR3, and a set of LCDRs refers to LCDR1, LCDR2 and LCDR3. Unless otherwise stated, a "set of CDRs" includes HCDRs and LCDRs.

Alternatively, a binding member of the invention may comprise an antigen-binding site within a non-antibody molecule, normally provided by one or more CDRs e.g. a set of CDRs in a non-antibody protein scaffold, as discussed further below.

As described herein, a parent antibody molecule was isolated (see Antibody 1 of Table 1) having the set of CDR sequences as shown in Table 1. Through a process of optimisation we generated a panel of antibody clones numbered 2-36, with CDR sequences derived from the parent CDR sequences and having modifications at the positions indicated in Table 1. Thus, for example, it can be seen from Table 1 that Antibody 2 has the parent HCDR1, HCDR3, and LCDR3 sequences, and has: a parent HCDR2 sequence in which Kabat residue 56 is replaced with R, a parent LCDR1 in which Kabat residue 31 is replaced with T; and an LCDR2 in which Kabat residue 53 is replaced with R.

Described herein is a binding member comprising the parent set of CDRs as shown in Table 1 (Antibody 1), in which HCDR1 is SEQ ID NO: 3 (Kabat residues 31-35), HCDR2 is SEQ ID NO: 4 (Kabat residues 50-65), HCDR3 is SEQ ID NO: 5 (Kabat residues 95-102), LCDR1 is SEQ ID NO: 354 (Kabat residues 24-34), LCDR2 is SEQ ID NO: 355 (Kabat residues 50-56) and LCDR3 is SEQ ID NO: 356 (Kabat residues 89-97). The binding member according to the invention may also be the parent binding member as shown in Table 1, wherein one or more of the CDRs have one or more amino acid additions, substitutions, deletions, and/or insertions. In one embodiment the binding member has up to eight amino acid additions, substitutions, deletions, and/or insertions.

A binding member of the invention may comprise one or a combination of CDRs as described herein.

For example, a binding member or a VH domain according to the invention may comprise the parent HCDR1 with Kabat residue 31 replaced with G, or with Kabat residue 33 replaced with P, or with Kabat residue 34 replaced with V (see Table 1).

A binding member or a VH domain of the invention may comprise the parent HCDR2 with one or more of the following modifications:

Kabat residue 51 is replaced with a V;
G is at Kabat residue 52B;
Kabat residue 53 is replaced with G;
Kabat residue 56 is replaced by R;
Kabat residue 63 is replaced by A.

In certain embodiments, the binding member has G at Kabat residue 52B and R at Kabat residue 56 of the parent HCDR2, and these binding members tend to exhibit a lower KD for IgE and/or higher potency for inhibiting IgE-mediated biological activities. See Table 1, which shows several antibodies having G at Kabat residue 52B and R at Kabat residue' 56 of the parent HCDR2.

A binding member or a VH domain of the invention may comprise the parent HCDR3 with one or more of the following substitutions:

Kabat residue 95 replaced by L or S;
Kabat residue 96 replaced by S, T, W, or F;
Kabat residue 97 replaced by Y or F;
Kabat residue 98 replaced by P or F;
Kabat residue 99 replaced by Y;
Kabat residue 100 replaced by I or S.

A binding member or a VH domain of the invention may comprise the parent HCDR3 (SEQ ID No. 5) with one or more of the following substitutions:

Kabat residue 95 replaced by H, L or S;
Kabat residue 96 replaced by I, S, T, W, or F;
Kabat residue 97 replaced by A, Y or F;
Kabat residue 98 replaced by V, P or F;
Kabat residue 99 replaced by A or Y;
Kabat residue 100 replaced by G, I or S.

A binding member, or a VL domain thereof may comprise the parent LCDR1 with Kabat residue 31 and/or Kabat residue 33 replaced by T. For example, binding members in which Kabat residue 31 in LCDR1 was T tended to exhibit higher affinity for IgE and/or a higher potency in inhibiting IgE-mediated biological activities. As shown in Table 1, antibodies 2-9, 11-19, 22-30 and 32-36 all have T at Kabat residue 31 in LCDR1. Other antibodies or LCDR1 sequences can be engineered to have T at this residue, e.g. using site-directed mutagenesis.

A binding member, or a VL domain thereof may comprise the parent LCDR2 with Kabat residue 53 replaced by R and/or Kabat residue 56 replaced by P:

A binding member, or a VL domain thereof may also comprise the parent LCDR3 with one or more amino acid additions, substitutions, deletions, and/or insertions, such as from one to five substitutions.

In another embodiment, the invention is a binding member in which: HCDR1 has amino acid sequence SEQ ID NO: 279, HCDR2 has amino acid sequence SEQ ID NO: 280, HCDR3 has amino acid sequence SEQ ID NO: 281, LCDR1 has amino acid sequence SEQ ID NO: 284, LCDR2 has amino acid sequence SEQ ID NO: 285, and LCDR3 has amino acid sequence SEQ ID NO: 286. For example, see Antibody 33 of Table 1. Still other embodiments of the invention are binding members, such as antibody molecules, capable of competing with Antibody 33 of Table 1 for binding to human IgE.

The invention provides binding members comprising an HCDR1, HCDR2 and/or HCDR3 of any of antibodies 1 to 36 and/or an LCDR1, LCDR2 and/or LCDR3 of any of antibodies 1 to 36, e.g. a set of CDRs of any of antibodies 1 to 36 shown in Table 1. The binding member may comprise a set of VH CDRs of one of these antibodies. Optionally it may also comprise a set of VL CDRs of one of these antibodies, and the VL CDRs may be from the same or a different antibody as the VH CDRs. A VH domain comprising a set of HCDRs of any of antibodies 1 to 36, and/or a VL domain comprising a set of LCDRs of any of antibodies 1 to 36, are also provided by the invention.

Typically, a VH domain is paired with a VL domain to provide an antibody antigen-binding site, although as discussed further below a VH or VL domain alone may be used to bind antigen. The Antibody 1 VH domain (see Table 3) may be paired with the Antibody 1 VL domain (see Table 2), so that an antibody antigen-binding site is formed comprising both the antibody 1 VH and VL domains. Analogous embodiments are provided for the other VH and VL domains disclosed herein. In other embodiments, the Antibody 1 VH is paired with a VL domain other than the Antibody 1 VL. Light-chain promiscuity is well established in the art. Again, analogous embodiments are provided by the invention for the other VH and VL domains disclosed herein. Thus, the VH of the parent or of any of antibodies 2 to 36 may be paired with the VL of the parent or of any of antibodies 2 to 36.

A binding member may comprise a set of H and/or L CDRs of the parent antibody or any of antibodies 2 to 36 with as many as twenty, sixteen, ten, nine or fewer, e.g. one, two, three, four or five, amino acid additions, substitutions, deletions, and/or insertions within the disclosed set of H and/or L CDRs. Alternatively, a binding member may comprise a set of H and/or L CDRs of the parent antibody or any of antibodies 2 to 36 with as many as twenty, sixteen, ten, nine or fewer, e.g. one, two, three, four or five, amino acid substitutions within the disclosed set of H and/or L CDRs. Such modifications may potentially be made at any residue within the set of CDRs. For example, modifications may be made at the positions modified in any of Antibodies 2 to 36, as shown in Table 1. Thus, the one or more modifications, may comprise one or more substitutions at the following residues: Kabat residues 31, 33, 34, 51, 52B, 53, 56, 63, 95, 96, 97, 98, 99, and 100 in the HCDRs; and Kabat residues 31, 33, 53, and 56 in the LCDRs.

A binding member may comprise an antibody molecule having one or more CDRs, e.g. a set of CDRs, within an antibody framework. For example, one or more CDRs or a set of CDRs of an antibody may be grafted into a framework (e.g. human framework) to provide an antibody molecule. The framework regions may be of human germline gene sequences, or may be non-germlined. For example, the framework may be germlined where one or more residues within the framework are changed to match the residues at the equivalent position in the most similar human germline framework. Thus, a binding member of the invention may be an isolated human antibody molecule having a VH domain comprising a set of HCDRs in a human germline framework, e.g. human germline IgG VH framework. Normally the binding member also has a VL domain comprising a set of LCDRs, e.g. in a human germline IgG VL framework.

The VH and/or VL framework residues may be modified as discussed and exemplified herein e.g. using site-directed mutagenesis.

A VH domain according to the invention, or a binding member comprising such a VH domain, may have a VH domain of any of antibody no. 1-36 of Table 3. For example, a VH domain of the invention may comprise the parent framework (antibody 1 of Table 3) having one or more of the following modifications:

Kabat residue 19 is replaced with S;
Kabat residue 25 is replaced with P,
Kabat residue 26 is replaced with E;
Kabat residue 27 is replaced with L;
Kabat residue 29 is replaced with L;
Kabat residue 30 is replaced with G;
Kabat residue 68 is replaced with A;
Kabat residue 69 is replaced with V;
Kabat residue 71 is replaced with K;
Kabat residue 77 is replaced with M;
Kabat residue 82B is replaced with G;
Kabat residue 82C is replaced with P;
Kabat residue 107 is replaced with A;

Kabat residue 108 is replaced with P;
Kabat residue 110 is replaced with A;
Kabat residue 111 is replaced with I; and/or
Kabat residue 112 is replaced with P.

In certain embodiments, Kabat residue 19 is replaced with S, Kabat residue 25 is replaced with P, Kabat residue 52B is replaced with G, Kabat residue 56 is replaced with R, and Kabat residue 71 is replaced with K.

Generally, the binding member of the invention or VH domain may contain the VH framework of any of antibodies 1-36 of Table 3 with from one to ten substitutions in the VH framework regions.

In another embodiment, the binding member of the invention or VH domain may contain the VH framework of Antibody 1 of Table 3 with from one to seven substitutions in the VH region.

In another embodiment, the binding member of the invention or VH domain may contain the VH framework of Antibody 33 of Table 3 with from one to ten substitutions in the VH region.

A VL domain according to the invention, or a binding member comprising such a VL domain, may have a VL domain sequence of any of antibody no 1-36 of Table 2. For example, an antibody VL domain may have the sequence of antibody 1 of Table 2, with one or more of the following modifications in the framework regions:

Kabat residue 2 may be replaced with F;
Kabat residue 3 may be replaced with M or E;
Kabat residue 5 may be replaced with S;
Kabat residue 13 may be replaced with A;
Kabat residue 22 may be replaced with A;
Kabat residue 37 is replaced with Q;
Kabat residue 39 is replaced with K;
Kabat residue 40 is replaced with P;
Kabat residue 42 is replaced with L;
Kabat residue 45 is replaced with A;
Kabat residue 58 is replaced with V;
Kabat residue 69 is replaced with D;
Kabat residue 70 is replaced with A;
Kabat residue 76 is replaced with G or R;
Kabat residue 77 is replaced with R;
Kabat residue 79 is replaced with Q or R;
Kabat residue 80 is replaced with A;
Kabat residue 103 is replaced with E
Kabat residue 105 is replaced with S; and/or
Kabat residue 106 is replaced with A.

In certain embodiments Kabat residue 42 is replaced with L, and Kabat residue 45 is replaced with A.

Generally, the binding member of the invention or VL domain may contain the VL framework of any of antibodies 1-36 of Table 2 with from one to ten substitutions in the VL framework regions.

Generally, the binding member of the invention or VL domain may contain the VL framework of any of Antibody 1 of Table 2 with from one to nine substitutions in the VL regions.

Generally, the binding member of the invention or VL domain may contain the VL framework of any of Antibody 33 of Table 2 with from one to six substitutions in the VL regions.

A non-germlined antibody molecule has the same CDRs, but different frameworks, compared to a germlined antibody molecule. Germlined antibodies may be produced by germlining framework regions of the VH and VL domain sequences shown herein for these antibodies.

A binding member of the invention may be one which competes for binding to IgE with any other binding member of the invention. Such binding members, which are said to bind IgE competitively, bind to the same epitope. Competition between binding members may be assayed easily *in vitro,* for example using ELISA and/or by tagging a specific reporter molecule to one binding member which can be detected in the presence of one or more other untagged binding members, thereby enabling the identification of binding members that bind the same epitope, as well as binding members that bind overlapping epitopes. Such methods are readily known to one of ordinary skill in the art, and are described in more detail herein. Thus, a further aspect of the present invention provides a binding member comprising a human antibody antigen-binding site that competes with an antibody molecule, for example especially an antibody molecule comprising a VH and/or VL domain, CDR e.g. HCDR3 or set of CDRs of the parent antibody or any of antibodies 1 to 36, for binding to IgE. In one embodiment, the binding member of the invention competes with Antibody 33 of Table 2 and 3.

In further aspects the present invention provides a binding member comprising a human antibody antigen-binding site which competes with an antibody antigen-binding site for binding to IgE, wherein the antibody antigen-binding site is composed of a VH domain and a VL domain, and wherein the VH and VL domains comprise a set of CDRs of the parent, or of any of antibodies 1 to 36, as disclosed herein.

In further aspects, the invention provides an isolated nucleic acid which comprises a sequence encoding a binding member, VH domain and/or VL domain according to the present invention. Exemplary nucleic acids that encode a VH domain of the invention are SEQ ID NOS: 1, 7, 17, 27, 37, 47, 57, 67, 77, 87, 97, 107, 117, 127, 137, 147, 157, 167, 177, 187, 197, 207, 217, 227, 237, 247, 257, 267, 277, 287, 297, 307, 317, 327, 337 and 425 of the attached sequence listing. Exemplary nucleic acids that encode a VL domain of the invention are SEQ ID NOS: 6, 357, 359, 361, 363, 365, 367, 369, 371, 373, 375, 377, 379, 381, 383, 385, 387, 389, 391, 393, 395, 397, 399, 401, 403, 405, 407, 409, 411, 413, 415, 417, 419, 421, 430 and 423 of the attached sequence listing. The invention also includes methods of preparing a binding member, a VH domain and/or a VL domain of the invention, which comprise expressing said nucleic acid under conditions to bring about production of said binding member, VH domain and/or VL domain, and recovering it by isolating or purifying the binding member.

Another aspect of the present invention provides nucleic acid, generally isolated, encoding a VH CDR or VL CDR sequence disclosed herein.

A further aspect provides a host cell containing or transformed with nucleic acid of the invention.

Further aspects of the present invention provide for compositions containing binding members of the invention, and their use in methods of inhibiting and/or neutralising IgE, including methods of treatment of the human or animal body by therapy.

For example, binding members according to the invention may be used in a method of treatment and/or prevention, or used in a method of diagnosis, of a biological response, disease, disorder, or condition in the human or animal body (e.g. in a human patient), or in vitro.

The method of treatment and/or prevention may comprise administering to said patient a binding member of the invention in an amount sufficient to measurably neutralize IgE. Conditions treatable in accordance with the present invention include any in which IgE plays a role, such as allergies and asthma.

These and other aspects of the invention are described in further detail below.

It is convenient to point out here that "and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

IgE is immunoglobulin E. The amino acid sequence of the human IgE constant region is publicly available. In some embodiments IgE may be human or cynomolgus monkey IgE. As described elsewhere herein, IgE may be recombinant, and/or may be either glycosylated or unglycosylated. IgE is expressed naturally *in* vivo in glycosylated form. Glycosylated IgE may also be expressed in recombinant systems, e.g. using U266.B1 cells.

A binding member generally refers to one member of a pair of molecules that bind one another. The members of a binding pair may be naturally derived or wholly or partially synthetically produced. One member of the pair of molecules has an area on its surface, or a cavity, which binds to and is therefore complementary to a particular spatial and polar organization of the other member of the pair of molecules. Examples of types of binding pairs are antigen-antibody, biotin-avidin, hormone-hormone receptor, receptor-ligand, enzyme-substrate. The present invention is generally concerned with antigen-antibody type reactions.

A binding member normally comprises a molecule having an antigen-binding site. For example, a binding member may be an antibody molecule or a non-antibody protein that comprises an antigen-binding site.

An antigen binding site may be provided by means of arrangement of CDRs on non-antibody protein scaffolds, such as fibronectin or cytochrome B etc. [1, 2, 3], or by randomising or mutating amino acid residues of a loop within a protein scaffold to confer binding specificity for a desired target. Scaffolds for engineering novel binding sites in proteins have been reviewed in detail by *Nygren et al.* [3]. Protein scaffolds for antibody mimics are disclosed in WO/0034784, which is herein incorporated by reference in its entirety, in which the inventors describe proteins (antibody mimics) that include a fibronectin type III domain having at least one randomised loop. A suitable scaffold into which to graft one or more CDRs, e.g. a set of HCDRs, may be provided by any domain member of the immunoglobulin gene superfamily. The scaffold may be a human or non-human protein. An advantage of a non-antibody protein scaffold is that it may provide an antigen-binding site in a scaffold molecule that is smaller and/or easier to manufacture than at least some antibody molecules. Small size of a binding member may confer useful physiological properties, such as an ability to enter cells, penetrate deep into tissues or reach targets within other structures, or to bind within protein cavities of the target antigen. Use of antigen binding sites in non-antibody protein scaffolds is reviewed in Wess, 2004 [4]. Typical are proteins having a stable backbone and one or more variable loops, in which the amino acid sequence of the loop or loops is specifically or randomly mutated to create an antigen-binding site that binds the target antigen. Such proteins include the IgG-binding domains of protein A from S. aureus, transferrin, tetranectin, fibronectin (e.g. 10th fibronectin type III domain), lipocalins as well as gamma-crystalline and other Affilin™ scaffolds (Scil Proteins). Examples of other approaches include synthetic "Microbodies" based on cyclotides - small proteins having intramolecular disulphide bonds, Microproteins (Versabodies™, Amunix) and ankyrin repeat proteins (DARPins, Molecular Partners).

In addition to antibody sequences and/or an antigen-binding site, a binding member according to the present invention may comprise other amino acids, e.g. forming a peptide or polypeptide, such as a folded domain, or to impart to the molecule another functional characteristic in addition to ability to bind antigen. Binding members of the invention may carry a detectable label, or may be conjugated to a toxin or a targeting moiety or enzyme (e.g. via a peptidyl bond or linker). For example, a binding member may comprise a catalytic site (e.g. in an enzyme domain) as well as an antigen binding site, wherein the antigen binding site binds to the antigen and thus targets the catalytic site to the antigen. The catalytic site may inhibit biological function of the antigen, e.g. by cleavage.

Although, as noted, CDRs can be carried by non-antibody scaffolds, the structure for carrying a CDR or a set of CDRs of the invention will generally be an antibody heavy or light chain sequence or substantial portion thereof in which the CDR or set of CDRs is located at a location corresponding to the CDR or set of CDRs of naturally occurring VH and VL antibody variable domains encoded by rearranged immunoglobulin genes. The structures and locations of immunoglobulin variable domains may be determined by reference to Kabat, et al., 1987 [5], and updates thereof findable under "Kabat" using any internet search engine).

By CDR region or CDR, it is intended to indicate the hypervariable regions of the heavy and light chains of the immunoglobulin as defined by Kabat *et al.* 1991 [6], and later editions. An antibody typically contains 3 heavy chain CDRs and 3 light chain CDRs. The term CDR or CDRs is used here in order to indicate, according to the case, one of these regions or several, or even the whole, of these regions which contain the majority of the amino acid residues responsible for the binding by affinity of the antibody for the antigen or the epitope which it recognizes.

Among the six short CDR sequences, the third CDR of the heavy chain (HCDR3) has a greater size variability (greater diversity essentially due to the mechanisms of arrangement of the genes which give rise to it). It may be as short as 2 amino acids although the longest size known is 26. CDR length may also vary according to the length that can be accommodated by the particular underlying framework. Functionally, HCDR3 plays a role in part in the determination of the specificity of the antibody [see references 7, 8, 9, 10, 11, 12, 13, 14].

Antibody molecule refers to an immunoglobulin whether natural or partly or wholly synthetically produced. The term also covers any polypeptide or protein comprising an antibody antigen-binding site. It must be understood here that the invention does not relate to the antibodies in natural form, that is to say they are not in their natural environment but have been isolated or obtained by purification from natural sources, or else obtained by genetic recombination, or by chemical synthesis, including modification with unnatural amino acids. Antibody fragments that comprise an antibody antigen-binding site include, but are not limited to, molecules such as Fab, Fab', Fab'-SH, scFv, Fv, dAb and Fd. Various other antibody molecules including one or more antibody antigen-binding sites have been engineered, including for example $Fab_2$, $Fab_3$, diabodies, triabodies, tetrabodies and minibodies. Antibody.molecules and methods for their construction and use are described in [15].

It is possible to take monoclonal and other antibodies and use techniques of recombinant DNA technology to produce other antibodies or chimeric molecules that bind the target antigen. Such techniques may involve introducing DNA encoding the immunoglobulin variable region, or the CDRs, of an antibody to the constant regions, or constant regions plus framework regions, of a different immunoglobulin. See, for instance, EP-A-184187, GB 2188638A or EP-A-239400, and a large body of subsequent literature. A hybridoma or other cell producing an antibody may be subject to genetic mutation or other changes, which may or may not alter the binding specificity of antibodies produced.

As antibodies can be modified in a number of ways, the term "antibody molecule" should be construed as covering any binding member or substance having an antibody antigen-binding site with the required specificity and/or binding to antigen. Thus, this term covers antibody fragments and derivatives, including any polypeptide comprising an antibody antigen-binding site, whether natural or wholly or partially synthetic. Chimeric molecules comprising an antibody antigen-binding site, or equivalent, fused to another polypeptide (e.g. derived from another species or belonging to another antibody class or subclass) are therefore included. Cloning and expression of chimeric antibodies are described in EP-A-0120694 and EP-A-0125023, and a large body of subsequent literature.

Further techniques available in the art of antibody engineering have made it possible to isolate human and humanised antibodies. For example, human hybridomas can be made as described by Kontermann & Dubel [16]. Phage display, another established technique for generating binding members has been described in detail in many publications, such as Kontermann & Dubel [16] and WO92/01047 (discussed further below), and US patents US 5,969,108, US 5,565,332, US 5,733,743, US 5,858,657, US 5,871,907, US 5,872,215, US 5,885,793, US 5,962,255, US 6,140,471, US 6,172,197, US 6,225,447, US 6,291,650, US 6,492,160, US 6,521,404.

Transgenic mice in which the mouse antibody genes are inactivated and functionally replaced with human antibody genes while leaving intact other components of the mouse immune system, can be used for isolating human antibodies [17]. Humanised antibodies can be produced using techniques known in the art such as those disclosed in for example WO91/09967, US 5,585,089, EP592106, US 5,565,332 and WO93/17105. Further, WO2004/006955 describes methods for humanising antibodies, based on selecting variable region framework sequences from human antibody genes by comparing canonical CDR structure types for CDR sequences of the variable region of a non-human antibody to canonical CDR structure types for corresponding CDRs from a library of human antibody sequences, e.g. germline antibody gene segments. Human antibody variable regions having similar canonical CDR structure types to the non-human CDRs form a subset of member human antibody sequences from which to select human framework sequences. The subset members may be further ranked by amino acid similarity between the human and the non-human CDR sequences. In the method of WO2004/006955, top ranking human sequences are selected to provide the framework sequences for constructing a chimeric antibody that functionally replaces human CDR sequences with the non-human CDR counterparts using the selected subset member human frameworks, thereby providing a humanized antibody of high affinity and low immunogenicity without need for comparing framework sequences between the non-human and human antibodies. Chimeric antibodies made according to the method arc also disclosed.

Synthetic antibody molecules may be created by expression from genes generated by means of oligonucleotides synthesized and assembled within suitable expression vectors, for example as described by Knappik *et al.* [18] or *Krebs et al.* [19].

It has been shown that fragments of a whole antibody can perform the function of binding antigens. Examples of binding fragments are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment [20, 21, 22], which consists of a VH or a VL domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site [23, 24]; (viii) bispecific single chain Fv dimers (PCT/US92/09965) and (ix) "diabodies", multivalent or multispecific fragments constructed by gene fusion (WO94/13804; [25]). Fv, scFv or diabody molecules may be stabilized by the incorporation of disulphide bridges linking the VH and VL domains [26]. Minibodies comprising a scFv joined to a CH3 domain may also be made [27]. Other examples of binding fragments are Fab', which differs from Fab fragments by the addition of a few residues at the carboxyl terminus of the heavy chain CH1 domain, including one or more cysteines from the antibody hinge region, and Fab'-SH, which is a Fab' fragment in which the cysteine residue(s) of the constant domains bear a free thiol group.

Antibody fragments of the invention can be obtained starting from a parent antibody molecule or any of the antibody molecules 1 to 36, by methods such as digestion by enzymes e.g. pepsin or papain and/or by cleavage of the disulfide bridges by chemical reduction. In another manner, the antibody fragments comprised in the present invention can be obtained by techniques of genetic recombination likewise well known to the person skilled in the art or else by peptide synthesis by means of, for example, automatic peptide synthesizers, such as those supplied by the company Applied Biosystems, etc., or by nucleic acid synthesis and expression.

Functional antibody fragments according to the present invention include any functional fragment whose half-life is increased by a chemical modification, especially by PEGylation, or by incorporation in a liposome.

A dAb (domain antibody) is a small monomeric antigen-binding fragment of an antibody, namely the variable region of an antibody heavy or light chain [22]. VH dAbs occur naturally in camelids (e.g. camel, llama) and may be produced by immunizing a camelid with a target antigen, isolating antigen-specific B cells and directly cloning dAb genes from individual B cells. dAbs are also producible in cell culture. Their small size, good solubility and temperature stability makes them particularly physiologically useful and suitable for selection and affinity maturation. Camelid VH dAbs are being developed for therapeutic use under the name "nanobodies™". A binding member of the present invention may be a dAb comprising a VH or VL domain substantially as set out herein, or a VH or VL domain comprising a set of CDRs substantially as set out herein.

Bispecific or bifunctional antibodies form a second generation of monoclonal antibodies in which two different variable regions are combined in the same molecule [28]. Their use has been demonstrated both in the diagnostic field and in the therapy field from their capacity to recruit new effector functions or to target several molecules on the surface of tumour cells. Where bispecific antibodies are to be used, these may be conventional bispecific antibodies, which can be manufactured in a variety of ways [29], e.g. prepared chemically or from hybrid hybridomas, or may be any of the bispecific antibody fragments mentioned above. These antibodies can be obtained by chemical methods [30, 31] or somatic methods [32, 33] but likewise and preferentially by genetic engineering techniques which allow the heterodimerization to be forced and thus facilitate the process of purification of the antibody sought [34]. Examples of bispecific antibodies include those of the BiTE™ technology in which the binding domains of two antibodies with different specificity can be used and directly linked via short flexible peptides. This combines two antibodies on a short single polypeptide chain. Diabodies and scFv can be constructed without an Fc region, using only variable domains, potentially reducing the effects of anti-idiotypic reaction.

Bispecific antibodies can be constructed as entire IgG, as bispecific Fab'2, as Fab'PEG, as diabodies or else as bispecific scFv. Further, two bispecific antibodies can be linked using routine methods known in the art to form tetravalent antibodies.

Bispecific diabodies, as opposed to bispecific whole antibodies, may also be particularly useful because they can be readily constructed and expressed in E. coli. Diabodies (and many other polypeptides, such as antibody fragments) of appropriate binding specificities can be readily selected using phage display (WO94/13804) from libraries. If one arm of the diabody is to' be kept constant, for instance, with a specificity directed against IgE, then a library can be made where the other arm is varied and an antibody of appropriate specificity selected. Bispecific whole antibodies may be made by alternative engineering methods as described in Ridgeway et al., 1996 [35].

Various methods are available in the art for obtaining antibodies against IgE. The antibodies may be monoclonal antibodies, especially of human, murine, chimeric or humanized origin, which can be obtained according to the standard methods well known to the person skilled in the art.

In general, for the preparation of monoclonal antibodies or their functional fragments, especially of murine origin, it is possible to refer to techniques which are described in particular in the manual "Antibodies" [36] or to the technique of preparation from hybridomas described by Köhler and Milstein [37].

Monoclonal antibodies can be obtained, for example, from an animal cell immunized against IgE, or one of its fragments containing the epitope recognized by said monoclonal antibodies. Suitable fragments and peptides or polypeptides comprising them are described herein, and may be used to immunise animals to generate antibodies against IgE. Said IgE, or one of its fragments, can especially be produced according to the usual working methods, by genetic recombination starting with a nucleic acid sequence contained in the cDNA sequence coding for IgE or fragment thereof, by peptide synthesis starting from a sequence of amino acids comprised in the peptide sequence of the IgE and/or fragment thereof.

The monoclonal antibodies can, for example, be purified on an affinity column on which IgE or one of its fragments containing the epitope recognized by said monoclonal antibodies, has previously been immobilized. More particularly, the monoclonal antibodies can be purified by chromatography on protein A and/or G, followed or not followed by ionexchange chromatography aimed at eliminating the residual protein contaminants as well as the DNA and the LPS, in itself, followed or not followed by exclusion chromatography on Sepharose gel in order to eliminate the potential aggregates due to the presence of dimers or of other multimers. In one embodiment, the whole of these techniques can be used simultaneously or successively.

An antigen-binding site is the part of a molecule that binds to and is complementary to all or part of the target antigen. In an antibody molecule it is referred to as the antibody antigen-binding site, and comprises the part of the antibody that binds to and is complementary to all or part of the target antigen. Where an antigen is large, an antibody may only bind to a particular part of the antigen, which part is termed an epitope. An antibody antigen-binding site may be provided by one or more antibody variable domains. An antibody antigen-binding site may comprise an antibody light chain variable region (VL) and an antibody heavy chain variable region (VH).

Isolated refers to the state in which binding members of the invention, or nucleic acid encoding such binding members, will generally be in accordance with the present invention. Thus, binding members, VH and/or VL domains, and encoding nucleic acid molecules and vectors according to the present invention may be provided isolated and/or purified, e.g. from their natural environment, in substantially pure or homogeneous form, or, in the case of nucleic acid, free or substantially free of nucleic acid or genes of origin other than the sequence encoding a polypeptide with the required function. Isolated members and isolated nucleic acid will be free or substantially free of material with which they are naturally associated, such as other polypeptides or nucleic acids with which they are found in their natural environment, or the environment in which they are prepared (e.g. cell culture) when such preparation is by recombinant DNA technology practised in vitro or in vivo. Members and nucleic acid may be formulated with diluents or adjuvants and still for practical purposes be isolated - for example the members will normally be mixed with gelatin or other carriers if used to coat microtitre plates for use in immunoassays, or will be mixed with pharmaceutically acceptable carriers or diluents when used in diagnosis or therapy. Binding members may be glycosylated, either naturally or by systems of heterologous eukaryotic cells (e.g. CHO or NS0 (ECACC 85110503) cells, or they may be (for example if produced by expression in a prokaryotic cell) unglycosylated.

Heterogeneous preparations comprising anti-IgE antibody molecules also form part of the invention. For example, such preparations may be mixtures of antibodies with full-length heavy chains and heavy chains lacking the C-terminal lysine, with various degrees of glycosylation and/or with derivatized amino acids, such as cyclization of an N-terminal glutamic acid to form a pyroglutamic acid residue.

As used herein, the phrase "substantially as set out" refers to the characteristic(s) of the relevant CDRs of the VH or VL domain of binding members described herein will be either identical or highly similar to the specified regions of which the sequence is set out herein. As described herein, the phrase "highly similar" with respect to specified region(s) of one or more variable domains, it is contemplated that from 1 to about 5, e.g. from 1 to 4, including 1 to 3, or 1 or 2, or 3 or 4, amino acid substitutions may be made in the CDR and/or VH or VL domain.

## Detailed Description

As noted above, a binding member in accordance with the present invention modulates and may neutralise a biological activity of IgE. As described herein, IgE-binding members of the present invention may be optimised for neutralizing potency: Generally, potency optimisation involves mutating the sequence of a selected binding member (normally the variable domain sequence of an antibody) to generate a library of binding members, which are then assayed for potency and the more potent binding members are selected. Thus selected "potency-optimised" binding members tend to have a higher potency than the binding member from which the library was generated. Nevertheless, high potency binding members may also be obtained without optimisation, for example a high potency binding member may be obtained directly from an initial screen e.g. a biochemical neutralization assay. A "potency optimized" binding member refers to a binding member with an optimized potency of neutralization of a particular activity or downstream function: Assays and potencies are described in more detail elsewhere herein. The present invention provides both potency-optimized and non-optimized binding members, as well as methods for potency optimization from a selected binding member. The present invention thus allows the skilled person to generate binding members having high potency.

Although potency optimization may be used to generate higher potency binding members from a given binding member, it is also noted that high potency binding members may be obtained even without potency optimization.

In a further aspect, the present invention provides a method of obtaining one or more binding members able to bind the antigen, the method including bringing into contact a library of binding members according to the invention and said antigen, and selecting one or more binding members of the library able to bind said antigen.

The library may be displayed on particles or molecular complexes, e.g. replicable genetic packages, such as yeast, bacterial or bacteriophage (e.g. T7) particles, viruses, cells or covalent, ribosomal or *other in vitro* display systems, each particle or molecular complex containing nucleic acid encoding the antibody VH variable domain displayed on it, and optionally also a displayed VL domain if present. Phage display is described in WO92/01047 and e.g. US patents US 5,969,108, US 5,565,332, US 5,733,743, US 5,858,657, US 5,871,907, US 5,872,215, US 5,885,793, US 5,962,255, US 6,140,471, US 6,172,197, US 6,225,447, US 6,291,650, US 6,492,160 and US 6,521,404, each of which is herein incorporated by reference in their entirety.

Following selection of binding members able to bind the antigen and displayed on bacteriophage or other library particles or molecular complexes, nucleic acid may be taken from a bacteriophage or other particle or molecular complex displaying a selected binding member. Such nucleic acid may be used in subsequent production of a binding member or an antibody VH or VL variable domain by expression from nucleic acid with the sequence of nucleic acid taken from a bacteriophage or other particle or molecular complex displaying a said selected binding member.

An antibody VH variable domain with the amino acid sequence of an antibody VH variable domain of a said selected binding member may be provided in isolated form, as may a binding member comprising such a VH domain.

Ability to bind IgE may be further tested, also ability to compete with e.g. a parent antibody molecule or an antibody molecule 1 to 36 (e.g. in scFv format and/or IgG format, e.g. IgG1) for binding to IgE. Ability to neutralize IgE may be tested, as discussed further elsewhere herein.

A binding member according to the present invention may bind IgE with the affinity of a parent or other antibody molecule, e.g. scFv, or one of antibodies 1 to 36, e.g. IgG1, or with an affinity that is better.

A binding member according to the present invention may neutralise a biological activity of IgE with the potency of a parent or other antibody molecule, one of antibodies 1 to 36 e.g. scFv, or IgG1, or with a potency that is better.

Binding affinity and neutralization potency of different binding members can be compared under appropriate conditions.

Variants of the VH and VL domains and CDRs of the present invention, including those for which amino acid sequences are set out herein, and which can be employed in binding members for IgE can be obtained by means of methods of sequence alteration or mutation and screening for antigen binding members with desired characteristics. Examples of desired characteristics include but are not limited to:

- Increased binding affinity for antigen relative to known antibodies which are specific for the antigen
- Increased neutralization of an antigen activity relative to known antibodies which are specific for the antigen if the activity is known
- Specified competitive ability with a known antibody or ligand to the antigen at a specific molar ratio
- Ability to immunoprecipitate complex
- Ability to bind to a specified epitope
    - o Linear epitope, e.g. peptide sequence identified using peptide-binding scan as described herein, e.g. using peptides screened in linear and/or constrained conformation
    - o Conformational epitope, formed by non-continuous residues
- Ability to modulate a new biological activity of IgE, or downstream molecule.

Such methods are also provided herein.

Variants of antibody molecules disclosed herein may be produced and used in the present invention. Following the lead of computational chemistry in applying multivariate data analysis techniques to the structure/property-activity relationships [38] quantitative activity-property relationships of antibodies can be derived using well-known mathematical techniques, such as statistical regression, pattern recognition and classification [39, 40, 41, 42, 43, 44]. The properties of antibodies can be derived from empirical and theoretical models (for example, analysis of likely contact residues or calculated physicochemical property) of antibody sequence, functional and three-dimensional structures and these properties can be considered singly and in combination.

An antibody antigen-binding site composed of a VH domain and a VL domain is typically formed by six loops of polypeptide: three from the light chain variable domain (VL) and three from the heavy chain variable domain (VH). Analysis of antibodies of known atomic structure has elucidated relationships between the sequence and three-dimensional structure of antibody combining sites [45, 46]. These relationships imply that, except for the third region (loop) in VH domains, binding site loops have one of a small number of main-chain conformations: canonical structures. The canonical structure formed in a particular loop has been shown to be determined by its size and the presence of certain residues at key sites in both the loop and in framework regions [45, 46].

This study of sequence-structure relationship can be used for prediction of those residues in an antibody of known sequence, but of an unknown three-dimensional structure, which are important in maintaining the three-dimensional structure of its CDR loops and hence maintain binding specificity. These predictions can be backed up by comparison of the predictions to the output from lead optimization experiments. In a structural approach, a model can be created of the antibody molecule [47] using any freely available or commercial package, such as WAM [48]. A protein visualisation and analysis software package, such as Insight II (Accelrys, Inc.) or Deep View [49] may then be used to evaluate possible substitutions at each position in the CDR. This information may then be used to make substitutions likely to have a minimal or beneficial effect on activity.

The techniques required to make substitutions within amino acid sequences of CDRs, antibody VH or VL domains and binding members generally are available in the art. Variant sequences may be made, with substitutions that may or may not be predicted to have a minimal or beneficial effect on activity, and tested for ability to bind and/or neutralize IgE and/or for any other desired property.

Variable domain amino acid sequence variants of any of the VH and VL domains whose sequences are specifically disclosed herein may be employed in accordance with the present invention, as discussed.

A further aspect of the invention is an antibody molecule comprising a VH domain that has at least 60, 70, 80, 85, 90, 95, 98 or 99 % amino acid sequence identity with a VH domain of any of antibodies 1 to 36 shown in Table 3 and the appended sequence listing, or with an HCDR (e.g., HCDR1, HCDR2, or HCDR3) shown in Table 1. The antibody molecule may optionally also comprise a VL domain that has at least 60, 70, 80, 85, 90, 95, 98 or 99 % amino acid sequence identity with a VL domain of any of the antibodies 1 to 36, or with an LCDR (e.g., LCDR1, LCDR2, or LCDR3) shown in Table 2. Algorithms that can be used to calculate % identity of two amino acid sequences include e.g. BLAST [50], FASTA [51], or the Smith-Waterman algorithm [52], e.g. employing default parameters.

Particular variants may include one or more amino acid sequence alterations (addition, deletion, substitution and/or insertion of an amino acid residue). In certain embodiments, the variants have less than about 20 such alterations.

Alterations may be made in one or more framework regions and/or one or more CDRs. The alterations normally do not result in loss of function, so a binding member comprising a thus-altered amino acid sequence may retain an ability to bind and/or neutralize IgE. It may retain the same quantitative binding and/or neutralizing ability as a binding member in which the alteration is not made, e.g. as measured in an assay described herein. The binding member comprising a thus-altered amino acid sequence may have an improved ability to bind and/or neutralize IgE.

Alteration may comprise replacing one or more amino acid residue(s) with a non-naturally occurring or non-standard amino acid, modifying one or more amino acid residue into a non-naturally occurring or non-standard form, or inserting one or more non-naturally occurring or non-standard amino acid into the sequence. Examples of numbers and locations of alterations in sequences of the invention are described elsewhere herein. Naturally occurring amino acids include the 20 "standard" L-amino acids identified as G, A, V, L, I, M, P, F, W, S, T, N, Q, Y, C, K, R, H, D, E by their standard single-letter codes. Non-standard amino acids include any other residue that may be incorporated into a polypeptide backbone or result from modification of an existing amino acid residue. Non-standard amino acids may be naturally occurring or non-naturally occurring. Several naturally occurring non-standard amino acids are known in the art, such as 4-hydroxyproline, 5-hydroxylysine, 3-methylhistidine, N-acetylserine, etc. [53]. Those amino acid residues that are derivatised at their N-alpha position will only be located at the N-terminus of an amino-acid sequence. Normally in the present invention an amino acid is an L-amino acid, but it may be a D-amino acid. Alteration may therefore comprise modifying an L-amino acid into, or replacing it with, a D-amino acid. Methylated, acetylated and/or phosphorylated forms of amino acids are also known, and amino acids in the present invention may be subject to such modification.

Amino acid sequences in antibody domains and binding members of the invention may comprise non-natural or non-standard amino acids described above. Non-standard amino acids (e.g. D-amino, acids) may be incorporated into an amino acid sequence during synthesis, or by modification or replacement of the "original" standard amino acids after synthesis of the amino acid sequence.

Use of non-standard and/or non-naturally occurring amino acids increases structural and functional diversity, and can thus increase the potential for achieving desired IgE-binding and neutralizing properties in a binding member of the invention. Additionally, D-amino acids and analogues have been shown to have better pharmacokinetic profiles compared with standard L-amino acids, owing to *in vivo* degradation of polypeptides having L-amino acids after administration to an animal e.g. a human.

Novel VH or VL regions carrying CDR-derived sequences of the invention may be generated using random mutagenesis of one or more selected VH and/or VL genes to generate mutations within the entire variable domain. Such a technique is described by Gram *et al.* [54], who used error-prone PCR. In some embodiments one or two amino acid substitutions are made within an entire variable domain or set of CDRs.

Another method that may be used is to direct mutagenesis to CDR regions of VH or VL genes. Such techniques are disclosed by Barbas *et al.* [55] and *Schier et al.* [56].

All the above-described techniques are known as such in the art and the skilled person will be able to use such techniques to provide binding members of the invention using routine methodology in the art.

A further aspect of the invention provides a method for obtaining an antibody antigen-binding site for IgE, the method comprising providing by way of addition, deletion, substitution or insertion of one or more amino acids in the amino acid sequence of a VH domain set out herein a VH domain which is an amino acid sequence variant of the VH domain, optionally combining the VH domain thus provided with one or more VL domains, and testing the VH domain or VH/VL combination or combinations to identify a binding member or an antibody antigen-binding site for IgE and optionally with one or more desired properties, e.g. ability to neutralize IgE activity. Said VL domain may have an amino acid sequence which is substantially as set out herein. An analogous method may be employed in which one or more sequence variants of a VL domain disclosed herein are combined with one or more VH domains.

As noted above, a CDR amino acid sequence substantially as set out herein may be carried as a CDR in a human antibody variable domain or a substantial portion thereof. The HCDR3 sequences substantially as set out herein represent embodiments of the present invention and each of these may be carried as a HCDR3 in a human heavy chain variable domain or a substantial portion thereof.

Variable domains employed in the invention may be obtained or derived from any germline or rearranged human variable domain, or may be a synthetic variable domain based on consensus or actual sequences of known human variable domains. A variable domain can be derived from a non-human antibody. A CDR sequence of the invention (e.g. CDR3) may be introduced into a repertoire of variable domains lacking a CDR (e.g. CDR3), using recombinant DNA technology. For example, *Marks et al.* [57] describe methods of producing repertoires of antibody variable domains in which consensus primers directed at or adjacent to the 5' end of the variable domain area are used in conjunction with consensus primers to the third framework region of human VH genes to provide a repertoire of VH variable domains lacking a CDR3. *Marks et al.* further describe how this repertoire may be combined with a CDR3 of a particular antibody. Using analogous techniques, the CDR3-derived sequences of the present invention may be shuffled with repertoires of VH or VL domains lacking a CDR3, and the shuffled complete VH or VL domains combined with a cognate VL or VH domain to provide binding members of the invention. The repertoire may then be displayed in a suitable host system, such as the phage display system of WO92/01047, which is herein incorporated by reference in its entirety, or any of a subsequent large body of literature, including Kay, Winter & McCafferty [58], so that suitable binding members may be selected. A repertoire may consist of from anything from $10^4$ individual members upwards, for example at least $10^5$, at least $10^6$, at least $10^7$, at least $10^8$, at least $10^9$ or at least $10^{10}$ members or more. Other suitable host systems include, but are not limited to yeast display, bacterial display, T7 display, viral display, cell display, ribosome display and covalent display.

A method of preparing a binding member for IgE antigen is provided, which method comprises:

(a) providing a starting repertoire of nucleic acids encoding a VH domain which either include a CDR3 to be replaced or lack a CDR3 encoding region;
(b) combining said repertoire with a donor nucleic acid encoding an amino acid sequence substantially as set out herein for a VH CDR3 such that said donor nucleic acid is inserted into the CDR3 region in the repertoire, so as to provide a product repertoire of nucleic acids encoding a VH domain;
(c) expressing the nucleic acids of said product repertoire;
(d) selecting a binding member for IgE; and
(e) recovering said binding member or nucleic acid encoding it.

Again, an analogous method may be employed in which a VL CDR3 of the invention is combined with a repertoire of nucleic acids encoding a VL domain that either include a CDR3 to be replaced or lack a CDR3 encoding region.

Similarly, one or more, or all three CDRs may be grafted into a repertoire of VH or VL domains that are then screened for a binding member or binding members for IgE.

For example, one or more of the parent or antibody 1 to 36 HCDR1, HCDR2 and HCDR3 or the parent or antibody 1 to 36 set of HCDRs may be employed, and/or one or more of the parent or antibody 1 to 36 LCDR1, LCDR2 and LCDR3 or the parent or antibody 1 to 36 set of LCDRs may be employed.

Similarly, other VH and VL domains, sets of CDRs and sets of HCDRs and/or sets of LCDRs disclosed herein may be employed.

A substantial portion of an immunoglobulin variable domain may comprise at least the three CDR regions, together with their intervening framework regions. The portion may also include at least about 50 % of either or both of the first and fourth framework regions, the 50 % being the C-terminal 50 % of the first framework region and the N-terminal 50 % of the fourth framework region. Additional residues at the N-terminal or C-terminal end of the substantial part of the variable domain may be those not normally associated with naturally occurring variable domain regions. For example, construction of binding members of the present invention made by recombinant DNA techniques may result in the introduction of Nor C-terminal residues encoded by linkers introduced to facilitate cloning or other manipulation steps. Other manipulation steps include the introduction of linkers to join variable domains of the invention to further protein sequences including antibody constant regions, other variable domains (for example in the production of diabodies) or detectable/functional labels as discussed in more detail elsewhere herein.

Although in some aspects of the invention, binding members comprise a pair of VH and VL domains, single binding domains based on either VH or VL domain sequences form further aspects of the invention. It is known that single immunoglobulin domains, especially VH domains, are capable of binding target antigens in a specific manner. For example, see the discussion of dAbs above.

In the case of either of the single binding domains, these domains may be used to screen for complementary domains capable of forming a two-domain binding member able to bind IgE. This may be achieved by phage display screening methods using the so-called hierarchical dual combinatorial approach as disclosed in WO92/01047, herein incorporated by reference in its entirety, in which an individual colony containing either an H or L chain clone is used to infect a complete library of clones encoding the other chain (L or H) and the resulting two-chain binding member is selected in accordance with phage display techniques, such as those described in that reference. This technique is also disclosed in Marks et al, ibid.

Binding members of the present invention may further comprise antibody constant regions or parts thereof, e.g. human antibody constant regions or parts thereof. For example, a VL domain may be attached at its C-terminal end to antibody light chain constant domains including human $C\kappa$ or $C\lambda$ chains. Similarly, a binding member based on a VH domain may be attached at its C-terminal end to all or part (e.g. a CH1 domain) of an immunoglobulin heavy chain derived from any antibody isotype, e.g. IgG, IgA, IgE and IgM and any of the isotype sub-classes, particularly IgG1 and IgG2. IgG1 is advantageous due to its ease of manufacture and stability, e.g., half-life. Any synthetic or other constant region variant that has these properties and stabilizes variable regions may also be useful in the present invention.

Binding members of the invention may be labelled with a detectable or functional label. Thus, a binding member or antibody molecule can be present in the form of an immunoconjugate so as to obtain a detectable and/or quantifiable signal. An immunoconjugate may comprise an antibody molecule of the invention conjugated with detectable or functional label. A label can be any molecule that produces or can be induced to produce a signal, including but not limited to fluorescers, radiolabels, enzymes, chemiluminescers or photosensitizers. Thus, binding may be detected and/or measured by detecting fluorescence or luminescence, radioactivity, enzyme activity or light absorbance.

Suitable labels include, by way of illustration and not limitation,

- enzymes, such as alkaline phosphatase, glucose-6-phosphate dehydrogenase ("G6PDH"), alpha-D-galactosidase, glucose oxydase, glucose amylase, carbonic anhydrase, acetylcholinesterase, lysozyme, malate dehydrogenase and peroxidase e.g. horseradish peroxidase;
- dyes;
- fluorescent labels or fluorescers, such as fluorescein and its derivatives, fluorochrome, rhodamine compounds and derivatives, GFP (GFP for "Green Fluorescent Protein"), dansyl, umbelliferone, phycoerythrin, phycocyanin, allophycocyanin, o-phthaldehyde, and fluorescamine; fluorophores such as lanthanide cryptates and chelates e.g. Europium etc (Perkin Elmer and Cis Biointernational),
- chemoluminescent labels or chemiluminescers, such as isoluminol, luminol and the dioxetanes;
- bio-luminescent labels, such as luciferase and luciferin;
- sensitizers;
- coenzymes;
- enzyme substrates;
- radiolabels including but not limited to bromine77, carbon14, cobalt57, fluorine8, gallium67, gallium 68, hydrogen3 (tritium), indium111, indium 113m, iodine123m; iodine125, iodine126, iodine131, iodine133, mercury107, mercury203, phosphorous32, rhenium99m, rhenium101, rhenium105, ruthenium95, ruthenium97, ruthenium103, ruthenium105, scandium47, selenium75, sulphur35, technetium99, technetium99m, tellurium121m, tellurium122m, tellurium125m, thulium165, thulium167, thulium168, yttrium199 and other radiolabels mentioned herein;

- particles, such as latex or carbon particles; metal sol; crystallite; liposomes; cells, etc., which may be further labelled with a dye, catalyst or other detectable group;
- molecules such as biotin, digoxygenin or 5-bromodeoxyuridine;
- toxin moieties, such as for example a toxin moiety selected from a group of Pseudomonas exotoxin (PE or a cytotoxic fragment or mutant thereof), Diptheria toxin or a cytotoxic fragment or mutant thereof, a botulinum toxin A, B, C, D, E or F, ricin or a cytotoxic fragment thereof e.g. ricin A, abrin or a cytotoxic fragment thereof, saporin or a cytotoxic fragment thereof, pokeweed antiviral toxin or a cytotoxic fragment thereof and bryodin 1 or a cytotoxic fragment thereof.

Suitable enzymes and coenzymes are disclosed in Litman, et al., US 4,275,149, and Boguslaski, et al., US 4,318,980, each of which are herein incorporated by reference in their entireties. Suitable fluorescers and chemiluminescers are disclosed in Litman, et al., US4275149, which is incorporated herein by reference in its entirety. Labels further include chemical moieties, such as biotin that may be detected via binding to a specific cognate detectable moiety, e.g. labelled avidin or streptavidin. Detectable labels may be attached to antibodies of the invention using conventional chemistry known in the art.

Immunoconjugates or their functional fragments can be prepared by methods known to the person skilled in the art. They can be coupled to enzymes or to fluorescent labels directly or by the intermediary of a spacer group or of a linking group, such as a polyaldehyde, like glutaraldehyde, ethylenediaminetetraacetic acid (EDTA), diethylenetriaminepentaacetic acid (DPTA), or in the presence of coupling agents, such as those mentioned above for the therapeutic conjugates. Conjugates containing labels of fluorescein type can be prepared by reaction with an isothiocyanate.

The methods known to the person skilled in the art existing for coupling the therapeutic radioisotopes to the antibodies either directly or via a chelating agent, such as EDTA, DTPA mentioned above can be used for the radioelements which can be used in diagnosis. It is likewise possible to perform labelling with sodium125 by the chloramine T method [59] or else with technetium99m by the technique of Crockford et al., (US 4,424,200, herein incorporated by reference in its entirety) or attached via DTPA as described by Hnatowich (US 4,479,930, herein incorporated by reference in its entirety).

There are numerous methods by which the label can produce a signal detectable by external means, for example, by visual examination, electromagnetic radiation, heat, and chemical reagents. The label can also be bound to another binding member that binds the antibody of the invention, or to a support.

The label can directly produce a signal, and therefore, additional components are not required to produce a signal. Numerous organic molecules, for example fluorescers, are able to absorb ultraviolet and visible light, where the light absorption transfers energy to these molecules and elevates them to an excited energy state. This absorbed energy is then dissipated by emission of light at a second wavelength. This second wavelength emission may also transfer energy to a labelled acceptor molecule, and the resultant energy dissipated from the acceptor molecule by emission of light for example fluorescence resonance energy transfer (FRET). Other labels that directly produce a signal include radioactive isotopes and dyes.

Alternately, the label may need other components to produce a signal, and the signal producing system would then include all the components required to produce a measurable signal, which may include substrates, coenzymes, enhancers, additional enzymes, substances that react with enzymic products, catalysts, activators, cofactors, inhibitors, scavengers, metal ions, and a specific binding substance required for binding of signal generating substances. A detailed discussion of suitable signal producing systems can be found in Ullman, et al. US 5,185,243, which is herein incorporated herein by reference in its entirety.

The present invention provides a method comprising causing or allowing binding of a binding member as provided herein to IgE. As noted, such binding may take *place in vivo,* e.g. following administration of a binding member or encoding nucleic acid to a human or animal (e.g., a mammal), or it may take place *in vitro,* for example in ELISA, Western blotting, immunocytochemistry, immunoprecipitation, affinity chromatography, and biochemical or cell-based assays.

Generally, complexes between the binding member of the invention and IgE may be detected by, *inter alia,* enzyme-linked immunoassay, radioassay, immunoprecipitation, fluorescence immunoassay, chemiluminescent assay, immunoblot assay, lateral flow assay, agglutination assay and particulate-based assay.

The present invention also provides for measuring levels of antigen directly, by employing a binding member according to the invention for example in a biosensor system. For instance, the present invention comprises a method of detecting and/or measuring binding to IgE, comprising, (i) exposing said binding member to IgE and (ii) detecting binding of said binding member to IgE, wherein binding is detected using any method or detectable label described herein. This, and any other binding detection method described herein, may be interpreted directly by the person performing the method, for instance, by visually observing a detectable label. Alternatively, this method, or any other binding detection method described herein, may produce a report in the form of an autoradiograph, a photograph, a computer printout, a flow cytometry report, a graph, a chart, a test tube or container or well containing the result, or any other visual or physical representation of a result of the method.

The amount of binding of binding member to IgE may be determined. Quantitation may be related to the amount of the antigen in a test sample, which may be of diagnostic interest. Screening for IgE binding and/or the quantitation thereof may be useful, for instance, in screening patients for diseases or disorders referred to herein and/or any other disease or disorder involving aberrant IgE production, expression and/or activity.

A diagnostic method of the invention may comprise (i) obtaining a tissue or fluid sample from a subject, (ii) exposing said tissue or fluid sample to one or more binding members of the present invention; and (iii) detecting bound IgE as compared with a control sample, wherein an increase in the amount of IgE binding as compared with the control may indicate an aberrant level of IgE production, expression or activity. Tissue or fluid samples to be tested include blood, serum, urine, biopsy material, tumours, or any tissue suspected of containing aberrant IgE levels. Subjects testing positive for aberrant IgE levels or activity may also benefit from the treatment methods disclosed later herein.

The diagnostic method of the invention may further comprise capturing a complex of the binding member and IgE via an immobilized antigen. For example, an antigen may be immobilized on a lateral strip assay for capturing antigen-specific IgE in a sample of interest.

Those skilled in the art are able to choose a suitable mode of determining binding of the binding member to an antigen according to their preference and general knowledge, in light of the methods disclosed herein.

The reactivities of binding members in a sample may be determined by any appropriate means. Radioimmunoassay (RIA) is one possibility. Radioactive labelled antigen is mixed with unlabelled antigen (the test sample) and allowed to bind to the binding member. Bound antigen is physically separated from unbound antigen and the amount of radioactive antigen bound to the binding member determined. The more antigen there is in the test sample the less radioactive antigen will bind to the binding member. A competitive binding assay may also be used with non-radioactive antigen, using antigen or an analogue linked to a reporter molecule. The reporter molecule may be a fluorochrome, phosphor or laser dye with spectrally isolated absorption or emission characteristics. Suitable fluorochromes include fluorescein, rhodamine, phycoerythrin and Texas Red, and lanthanide chelates or cryptates. Suitable chromogenic dyes include diaminobenzidine.

Other reporters include macromolecular colloidal particles or particulate material, such as latex beads that are colored, magnetic or paramagnetic, and biologically or chemically active agents that can directly or indirectly cause detectable signals to be visually observed, electronically detected or otherwise recorded. These molecules may be enzymes, which catalyze reactions that develop, or change colours or cause changes in electrical properties, for example. They may be molecularly excitable, such that electronic transitions between energy states result in characteristic spectral absorptions or emissions. They may include chemical entities used in conjunction with biosensors. Biotin/avidin or biotin/streptavidin and alkaline phosphatase detection systems may be employed.

The signals generated by individual binding member-reporter conjugates may be used to derive quantifiable absolute or relative data of the relevant binding member binding in samples (normal and test).

A kit comprising a binding member according to any aspect or embodiment of the present invention is also provided as an aspect of the present invention. In the kit, the binding member may be labelled to allow its reactivity in a sample to be determined, e.g. as described further below. Further the binding member may or may not be attached to a solid support. Components of a kit are generally sterile and in sealed vials or other containers. Kits may be employed in diagnostic analysis or other methods for which binding members are useful. A kit may contain instructions for use of the components in a method, e.g. a method in accordance with the present invention. Ancillary materials to assist in or to enable performing such a method may be included within a kit of the invention. The ancillary materials include a second, different binding member which binds to the first binding member and is conjugated to a detectable label (e.g., a fluorescent label, radioactive isotope or enzyme): Antibody-based kits may also comprise beads for conducting an immunoprecipitation. Each component of the kits is generally in its own suitable container. Thus, these kits generally comprise distinct containers suitable for each binding member. Further, the kits may comprise instructions for performing the assay and methods for interpreting and analyzing the data resulting from the performance of the assay.

The present invention also provides the use of a binding member as above for measuring antigen levels in a competition assay, that is to say a method of measuring the level of antigen in a sample by employing a binding member as provided by the present invention in a competition assay. This may be where the physical separation of bound from unbound antigen is not required. Linking a reporter molecule to the binding member so that a physical or optical change occurs on binding is one possibility. The reporter molecule may directly or indirectly generate detectable signals, which may be quantifiable. The linkage of reporter molecules may be directly or indirectly, covalently, e.g. via a peptide bond or non-covalently. Linkage via a peptide bond may be as a result of recombinant expression of a gene fusion encoding antibody and reporter molecule.

In various aspects and embodiments, the present invention extends to a binding member that competes for binding to IgE with any binding member defined herein, e.g. the parent antibody or any of antibodies 1 to 36, e.g. in IgG1 format. Competition between binding members may be assayed easily *in vitro,* for example by tagging a specific reporter molecule to one binding member which can be detected in the presence of other untagged binding member(s), to enable identification of binding members which bind the same epitope or an overlapping epitope. Competition may be determined for example using ELISA in which IgE is immobilized to a plate and a first tagged or labelled binding member along with one or more other untagged or unlabelled binding members is added to the plate. Presence of an untagged binding member that competes with the tagged binding member is observed by a decrease in the signal emitted by the tagged binding member.

For example, the present invention includes a method of identifying an IgE binding compound, comprising (i) immobilizing IgE to a support, (ii) contacting said immobilized IgE simultaneously or in a step-wise manner with at least one tagged or labelled binding member according to the invention and one or more untagged or unlabelled test binding compounds, and (iii) identifying a new IgE binding compound by observing a decrease in the amount of bound tag from the tagged binding member. Such methods can be performed in a high-throughput manner using a multiwell or array format. Such assays may be also be performed in solution. See, for instance, U.S. 5,814,468, which is herein incorporated by reference in its entirety. As described above, detection of binding may be interpreted directly by the person performing the method, for instance, by visually observing a detectable label, or a decrease in the presence thereof. Alternatively, the binding methods of the invention may produce a report in the form of an autoradiograph, a photograph, a computer printout, a flow cytometry report, a graph, a chart, a test tube or container or well containing the result, or any other visual or physical representation of a result of the method.

Competition assays can also be used in epitope mapping. In one instance epitope mapping may be used to identify the epitope bound by an IgE-binding member which optionally may have optimized neutralizing and/or modulating characteristics. Such an epitope can be linear or conformational. A conformational epitope can comprise at least two different fragments of IgE, wherein said fragments are positioned in proximity to each other when IgE is folded in its tertiary or quaternary structure to form a conformational epitope which is recognized by an inhibitor of IgE, such as an IgE- binding member. In testing for competition a peptide fragment of the antigen may be employed, especially a peptide including or consisting essentially of an epitope of interest. A peptide having the epitope sequence plus one or more amino acids at either end may be used. Binding members according to the present invention may be such that their binding for antigen is inhibited by a peptide with or including the sequence given.

The present invention further provides an isolated nucleic acid encoding a binding member of the present invention. Nucleic acid may include DNA and/or RNA. In one, the present invention provides a nucleic acid that codes for a CDR or set of CDRs or VH domain or VL domain or antibody antigen-binding site or antibody molecule, e.g. scFv or IgG1, of the invention as defined above.

The present invention also provides constructs in the form of plasmids, vectors, transcription or expression cassettes which comprise at least one polynucleotide as above.

The present invention also provides a recombinant host cell that comprises one or more constructs as above. A nucleic acid encoding any CDR or set of CDRs or VH domain or VL domain or antibody antigen-binding site or antibody molecule, e.g. scFv or IgG1 as provided, itself forms an aspect of the present invention, as does a method of production of the encoded product, which method comprises expression from encoding nucleic acid therefor. Expression may conveniently be achieved by culturing under appropriate conditions recombinant host cells containing the nucleic acid. Following production by expression a VH or VL domain, or binding member may bc isolated and/or purified using any suitable technique, then used as appropriate.

Nucleic acid according to the present invention may comprise DNA or RNA and may be wholly or partially synthetic. Reference to a nucleotide sequence as set out herein encompasses a DNA molecule with the specified sequence, and encompasses a RNA molecule with the specified sequence in which U is substituted for T, unless context requires otherwise.

A yet further aspect provides a method of production of an antibody VH variable domain, the method including causing expression from encoding nucleic acid. Such a method may comprise culturing host cells under conditions for production of said antibody VH variable domain.

Analogous methods for production of VL variable domains and binding members comprising a VH and/or VL domain are provided as further aspects of the present invention.

A method of production may comprise a step of isolation and/or purification of the product. A method of production may comprise formulating the product into a composition including at least one additional component, such as a pharmaceutically acceptable excipient.

Systems for cloning and expression of a polypeptide in a variety of different host cells are well known. Suitable host cells include bacteria, mammalian cells, plant cells, filamentous fungi, yeast and baculovirus systems and transgenic plants and animals. The expression of antibodies and antibody fragments in prokaryotic cells is well established in the art. For a review, see for example Plückthun [60]. A common bacterial host is *E. coli.*

Expression in eukaryotic cells in culture is also available to those skilled in the art as an option for production of a binding member [61, 62, 63]. Mammalian cell lines available in the art for expression of a heterologous polypeptide include Chinese hamster ovary (CHO) cells, HeLa cells, baby hamster kidney cells, NS0 mouse melanoma cells, YB2/0 rat myeloma cells, human embryonic kidney cells, human embryonic retina cells and many others.

Suitable vectors can be chosen or constructed, containing appropriate regulatory sequences, including promoter sequences, terminator sequences, polyadenylation sequences, enhancer sequences, marker genes and other sequences as appropriate. Vectors may be plasmids e.g. phagemid, or viral e.g. 'phage, as appropriate [64]. Many known techniques and protocols for manipulation of nucleic acid, for example in preparation of nucleic acid constructs, mutagenesis, sequencing, introduction of DNA into cells and gene expression, and analysis of proteins, are described in detail in Ausubel *et al.* [65].

A further aspect of the present invention provides a host cell containing nucleic acid as disclosed herein. Such a host cell may *be in vitro* and may be in culture. Such a host cell may be *in vivo. In vivo* presence of the host cell may allow intra-cellular expression of the binding members of the present invention as "intrabodies" or intra-cellular antibodies. Intrabodies may be used for gene therapy.

A still further aspect provides a method comprising introducing nucleic acid of the invention into a host cell. The introduction may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. Introducing nucleic acid in the host cell, in particular a eukaryotic cell may use a viral or a plasmid based system. The plasmid system may be maintained episomally or may be incorporated into the host cell or into an artificial chromosome. Incorporation may be either by random or targeted integration of one or more copies at single or multiple loci. For bacterial cells, suitable techniques may include calcium chloride transformation, electroporation and transfection using bacteriophage.

The introduction may be followed by causing or allowing expression from the nucleic acid, e.g. by culturing host cells under conditions for expression of the gene. The purification of the expressed product may be achieved by methods known to one of skill in the art.

Nucleic acid of the invention may be integrated into the genome (e.g. chromosome) of the host cell. Integration may be promoted by inclusion of sequences that promote recombination with the genome, in accordance with standard techniques.

The present invention also provides a method that comprises using a construct as stated above in an expression system in order to express a binding member or polypeptide as above.

Binding members of the present invention may be used in methods of diagnosis or treatment in human or animal subjects, especially human. Binding members for IgE may be used to treat disorders characterized by biological effects mediated by IgE, particularly allergies and asthma. For example, binding members of the invention may be used to treat allergic rhinitis, allergic contact dermatitis, atopic dermatitis, anaphylactic reaction, food allergy, urticaria, inflammatory bowel disease, eosinophilic gastroenteritis, drug-induced rash, allergic opthalmopathy, allergic conjunctivitis, asthma bronchiale, airway hyperresponsiveness, cosmetic allergy, drug-induced allergy, drug-induced hypersensitivity syndrome, metal allergy, occupational hypersensitivity pneumonitis, chronic hypersensitivity pneumonitis, cold hypersensitivity, helminthic infection induced hypersensitivity, latex allergy or hay fever.

Binding members for IgE may be used to inhibit allergen-induced mast-cell degranulation in vivo or in vitro, reduce Fc$\varepsilon$R1-mediated biological responses in vivo or in vitro, as well as to reduce circulating IgE in a human or animal patient.

Accordingly, the invention provides a method for inhibiting allergen-induced mast cell degranulation in a mammal, comprising administering to said mammal a binding member, antibody, VH domain, or VL domain of the invention, in an amount sufficient to neutralize IgE.

The invention further provides a method for reducing Fc$\varepsilon$R1-mediated biological responses; comprising, contacting a cell expressing the Fc$\varepsilon$R1 with a binding member, antibody, VH domain, or VL domain of the invention, in the presence of IgE.

When test cells are contacted with the binding member of the invention in vitro, a control cell(s) may also be used for positive controls (e.g., reactions containing no binding member) and/or negative controls (e.g., reactions containing no IgE and/or antigen).

When cells are contacted by the binding member in vivo, for example, by administering the binding member of the invention to a mammal exhibiting Fc$\varepsilon$R1-mediated biological responses, the binding member of the invention is administered in amounts sufficient to neutralize IgE.

Still further, the invention provides a method for reducing circulating IgE in a mammal, such as a human, comprising administering a binding member, antibody, VH domain, or VL domain of the invention, in an amount sufficient to neutralize and reduce circulating free IgE.

Binding members of the invention may be used in the diagnosis or treatment of diseases or disorders including but not limited to any one or more of the following: allergic rhinitis, allergic contact dermatitis, atopic dermatitis, anaphylactic reaction, food allergy, urticaria, inflammatory bowel disease, eosinophilic gastroenteritis, drug-induced rash, allergic opthalmopathy, rhino-conjunctivitis, and allergic conjunctivitis.

Evidence for involvement of IgE in the above disorders is known in the art.

The data presented herein with respect to binding and neutralization of IgE thus indicate that binding members of the invention can be used to treat or prevent such disorders, including the reduction of severity of the disorders. Accordingly, the invention provides a method of treating or reducing the severity of at least one symptom of any of the disorders mentioned herein, comprising administering to a patient in need thereof an effective amount of one or more binding members of the present invention alone or in a combined therapeutic regimen with another appropriate medicament known in the art or described herein such that the severity of at least one symptom of any of the above disorders is reduced.

Binding members of the invention may be used in appropriate animals and in animal models of disease, especially monkeys.

Thus, the binding members of the present invention are useful as therapeutic agents in the treatment of diseases or disorders involving IgE, e.g. IgE production, expression and/or activity, especially aberrant production, expression, or activity. A method of treatment may comprise administering an effective amount of a binding member of the invention to a patient in need thereof, wherein aberrant production, expression and/or activity of IgE is thereby decreased. A method of treatment may comprise (i) identifying a patient demonstrating aberrant IgE levels or activity, for instance using the diagnostic methods described above, and (ii) administering an effective amount of a binding member of the invention to the patient, wherein aberrant production, expression and/or activity of IgE is decreased. An effective amount according to the invention is an amount that decreases the aberrant production, expression and/or activity of IgE so as to decrease or lessen the severity of at least one symptom of the particular disease or disorder being treated, but not necessarily cure the disease or disorder.

The invention also provides a method of antagonising at least one effect of IgE comprising contacting with or administering an effective amount of one or more binding members of the present invention such that said at least one effect of IgE is antagonised. Effects of IgE that may be antagonised by the methods of the invention include biological responses mediated by FcεR1, and any downstream effects that arise as a consequence of these binding reactions.

Accordingly, further aspects of the invention provide the use of an isolated binding member, antibody, VH domain or VL domain of the invention for the manufacture of a medicament for treating a disorder associated with, or mediated by, IgE as discussed herein. Such use of, or methods of making, a medicament or pharmaceutical composition comprise formulating the binding member with a pharmaceutically acceptable excipient.

A pharmaceutically acceptable excipient may be a compound or a combination of compounds entering into a pharmaceutical composition not provoking secondary reactions and which allows, for example, facilitation of the administration of the active compound(s), an increase in its lifespan and/or in its efficacy in the body, an increase in its solubility in solution or else an improvement in its conservation. These pharmaceutically acceptable vehicles are well known and will be adapted by the person skilled in the art as a function of the nature and of the mode of administration of the active compound(s) chosen.

Binding members of the present invention will usually be administered in the form of a pharmaceutical composition, which may comprise at least one component in addition to the binding member. Thus pharmaceutical compositions according to the present invention, and for use in accordance with the present invention, may comprise, in addition to active ingredient, a pharmaceutically acceptable excipient, carrier, buffer, stabilizer or other materials well known to those skilled in the art. Such materials should be non-toxic and should not interfere with the efficacy of the active ingredient. The precise nature of the carrier or other material will depend on the route of administration, which may be oral, inhaled, intra-tracheal, topical, intra-vesicular or by injection, as discussed below.

Pharmaceutical compositions for oral administration, such as for example single domain antibody molecules (e.g. "nanobodies™") etc are also envisaged in the present invention. Such oral formulations may be in tablet, capsule, powder, liquid or semi-solid form. A tablet may comprise a solid carrier, such as gelatin or an adjuvant. Liquid pharmaceutical compositions generally comprise a liquid carrier, such as water, petroleum, animal or vegetable oils, mineral oil or synthetic oil. Physiological saline solution, dextrose or other saccharide solution or glycols, such as ethylene glycol, propylene glycol or polyethylene glycol may be included.

For intra-venous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles, such as Sodium Chloride Injection, Ringer's Injection, Lactated Ringer's Injection. Preservatives, stabilizers, buffers, antioxidants and/or other additives may be employed as required including buffers such as phosphate, citrate and other organic acids; antioxidants, such as ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3'-pentanol; and m-cresol); low molecular weight polypeptides; proteins, such as serum albumin, gelatin or immunoglobulins; hydrophilic polymers, such as polyvinylpyrrolidone; amino acids, such as glycine, glutamine, asparagines, histidine, arginine, or lysine; monosaccharides, disaccharides and other carbohydrates including glucose, mannose or dextrins; chelating agents, such as EDTA; sugars, such as sucrose, mannitol, trehalose or sorbitol; salt-forming counter-ions, such as sodium; metal complexes (e.g. Zn-protein complexes); and/or non-ionic surfactants, such as TWEEN™, PLURONICS™ or polyethylene glycol (PEG).

Binding members of the present invention may be formulated in liquid, semi-solid or solid forms depending on the physicochemical properties of the molecule and the route of delivery. Formulations may include excipients, or combinations of excipients, for example: sugars, amino acids and surfactants. Liquid formulations may include a wide range of antibody concentrations and pH. Solid formulations may be produced by lyophilisation, spray drying, or drying by supercritical fluid technology, for example. Formulations of anti-IgE will depend upon the intended route of delivery: for example, formulations for pulmonary delivery may consist of particles with physical properties that ensure penetration into the deep lung upon inhalation; topical formulations (e.g. for treatment of scarring, e.g. dermal scarring) may include viscosity modifying agents, which prolong the time that the drug is resident at the site of action. A binding member may be prepared with a carrier that will protect the binding member against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are known to those skilled in the art [66].

Anti-IgE treatment may be given orally (such as for example single domain antibody molecules (e.g. "nanobodies™")) by injection (for example, subcutaneously, intra-articular, intra-venously, intra-peritoneal, intra-arterial or intra-muscularly), by inhalation, intra-tracheal, by the intra-vesicular route (instillation into the urinary bladder), or topically (for example intra-ocular, intra-nasal, rectal, into wounds, on skin). The treatment may be administered by pulse infusion, particularly with declining doses of the binding member. The route of administration can be determined by the physicochemical characteristics of the treatment, by special considerations for the disease or by the requirement to optimize efficacy or to minimize side-effects. One particular route of administration is intra-venous. Another route of administering pharmaceutical compositions of the present invention is subcutaneously. It is envisaged that anti-IgE treatment will not be restricted to use in the clinic. Therefore, subcutaneous injection using a needle-free device is also advantageous.

A composition may be administered alone or in combination with other treatments, either simultaneously or sequentially dependent upon the condition to be treated.

A binding member for IgE may be used as part of a combination therapy in conjunction with an additional medicinal component. Combination treatments may be used to provide significant synergistic effects, particularly the combination of an anti-IgE binding member with one or more other drugs. A binding member for IgE may be administered concurrently or sequentially or as a combined preparation with another therapeutic agent or agents, for the treatment of one or more of the conditions listed herein.

A binding member of the invention may be formulated and/or used in combination with other available treatments for asthma and allergic disorders, or other disorders involving IgE mediated effects.

A binding member according to the present invention may be provided in combination or addition with one or more of the following agents:

- a cytokine or agonist or antagonist of cytokine function (e.g. an agent which acts on cytokine signalling pathways, such as a modulator of the SOCS system), such as an alpha-, beta- and/or gamma-interferon; insulin-like growth factor type I (IGF-1), its receptors and associated binding proteins; interleukins (IL), e.g. one or more of IL-1 to -33, and/or an interleukin antagonist or inhibitor, such as anakinra; inhibitors of receptors of interleukin family members or inhibitors of specific subunits of such receptors, a tumour necrosis factor alpha (TNF-$\alpha$) inhibitor, such as an anti-TNF monoclonal antibodies (for example infliximab, adalimumab and/or CDP-870) and/or a TNF receptor antagonist, e.g. an immunoglobulin molecule (such as etanercept) and/or a low-molecular-weight agent, such as pentoxyfylline;
- a modulator of B cells, e.g. a monoclonal antibody targeting B-lymphocytes (such as CD20 (rituximab) or MRA-aIL16R) or T-lymphocytes (e.g. CTLA4-Ig, HuMax Il-15 or Abatacept);
- a modulator that inhibits osteoclast activity, for example an antibody to RANKL;
- a modulator of chemokine or chemokine receptor function, such as an antagonist of CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 and CXCR6 (for the C-X-C family) and $CX_3CR1$ for the C-$X_3$-C family;
- an inhibitor of matrix metalloproteases (MMPs), i.e. one or more of the stromelysins, the collagenases and the gelatinases as well as aggrecanase, especially collagenase-1 (MMP-1), collagenase-2 (MMP-8), collagenase-3 (MMP-13), stromelysin-1 (MMP-3), stromelysin-2 (MMP-10) and/or stromelysin-3 (MMP-11) and/or MMP-9 and/or MMP-12, e.g. an agent such as doxycycline;
- a leukotriene biosynthesis inhibitor, 5-lipoxygenase (5-LO) inhibitor or 5-lipoxygenase activating protein (FLAP) antagonist, such as zileuton; ABT-761; fenleuton; tepoxalin; Abbott-79175; Abbott-85761; N-(5-substituted)-thiophene- 2-alkylsulfonamides; 2,6-di-tert-butylphenolhydrazones; methoxytetrahydropyrans such as Zeneca ZD-2138; the compound SB-210661; a pyridinyl-substituted 2-cyanonaphthalene compound, such as L-739,010; a 2-cyanoquinoline compound, such as L-746,530; indole and/or a quinoline compound, such as MK-591, MK-886 and/or BAY x 1005;
- a receptor antagonist for leukotrienes (LT) B4, LTC4, LTD4, and LTE4, selected from the group consisting of the phenothiazin-3-1s, such as L-651,392; amidino compounds, such as CGS-25019c; benzoxalamines, such as ontazolast; benzenecarboximidamides, such as BIIL 284/260; and compounds, such as zafirlukast, ablukast, montelukast, pranlukast, verlukast (MK-679), RG-12525, Ro-245913, iralukast (CGP 45715A) and BAY x 7195;

- a phosphodiesterase (PDE) inhibitor, such as a methylxanthanine, e.g. theophylline and/or aminophylline; and/or a selective PDE isoenzyme inhibitor, e.g. a PDE4 inhibitor and/or inhibitor of the isoform PDE4D and/or an inhibitor of PDE5;
- a histamine type 1 receptor antagonist, such as cetirizine, loratadine, desloratadine, fexofenadine, acrivastine, terfenadine, astemizole, azelastine, levocabastine, chlorphenirainine, promethazine, cyclizine, and/or mizolastine (generally applied orally, topically or parenterally);
- a proton pump inhibitor (such as omeprazole) or gastroprotective histamine type 2 receptor antagonist;
- an antagonist of the histamine type 4 receptor;
- an alpha-1/alpha-2 adrenoceptor agonist vasoconstrictor sympathomimetic agent, such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride and ethylnorepinephrine hydrochloride;
- an anticholinergic agent, e.g. a muscarinic receptor (M1, M2, and M3) antagonist, such as atropine, hyoscine, glycopyrrrolate, ipratropium bromide, tiotropium bromide, oxitropium bromide, pirenzepine and telenzepine;
- a beta-adrenoceptor agonist (including beta receptor subtypes 1-4), such as isoprenaline, salbutamol, formoterol, salmeterol, terbutaline, orciprenaline, bitolterol mesylate and/or pirbuterol, e.g. a chiral enantiomer thereof;
- a chromone, e.g. sodium cromoglycate and/or nedocromil sodium;
- a glucocorticoid, such as flunisolide, triamcinolone acetonide, beclomethasone dipropionate, budesonide, fluticasone propionate, ciclesonide, and/or mometasone furoate;
- an agent that modulate nuclear hormone receptors, such as a PPAR;
- an immunoglobulin (Ig) or Ig preparation or an antagonist or antibody modulating Ig function, such as anti-IgE that binds to the same or a different epitope as the binding member of the invention;
- other systemic or topically-applied anti-inflammatory agent, e.g. thalidomide or a derivative thereof, a retinoid, dithranol and/or calcipotriol;
- combinations of aminosalicylates and sulfapyridine, such as sulfasalazine, mesalazine, balsalazide, and olsalazine; and immunomodulatory agents, such as the thiopurines; and corticosteroids, such as budesonide;
- an antibacterial agent, e.g. a penicillin derivative, a tetracycline, a macrolide, a betalactam, a fluoroquinolone, metronidazole and/or an inhaled aminoglycoside; and/or an antiviral agent, e.g. acyclovir, famciclovir, valaciclovir, ganciclovir, cidofovir; amantadine, rimantadine; ribavirin; zanamavir and/or oseltamavir; a protease inhibitor, such as indinavir, nelfinavir, ritonavir and/or saquinavir; a nucleoside reverse transcriptase inhibitor, such as didanosine, lamivudine, stavudine, zalcitabine, zidovudine; a non-nucleoside reverse transcriptase inhibitor, such as nevirapine, efavirenz;
- a cardiovascular agent, such as a calcium channel blocker, beta-adrenoceptor blocker, angiotensin-converting enzyme (ACE) inhibitor, angiotensin-2 receptor antagonist; lipid lowering agent, such as a statin and/or fibrate; a modulator of blood cell morphology, such as pentoxyfylline; a thrombolytic and/or an anticoagulant, e.g. a platelet aggregation inhibitor;
- a CNS agent, such as an antidepressant (such as sertraline), anti-Parkinsonian drug (such as deprenyl, L-dopa, ropinirole, pramipexole; MAOB inhibitor, such as selegine and rasagiline; comP inhibitor, such as tasmar; A-2 inhibitor; dopamine reuptake inhibitor, NMDA antagonist, nicotine agonist, dopamine agonist and/or inhibitor of neuronal nitric oxide synthase) and an anti-Alzheimer's drug, such as donepezil, rivastigmine, tacrine, COX-2 inhibitor, propentofylline or metrifonate;
- an agent for the treatment of acute and chronic pain, e.g. a centrally or peripherally-acting analgesic, such as an opioid analogue or derivative, carbamazepine, phenytoin, sodium valproate, amitryptiline or other antidepressant agent, paracetamol, or non-steroidal anti-inflammatory agent;
- a parenterally or topically-applied (including inhaled) local anaesthetic agent, such as lignocaine or an analogue thereof;
- an anti-osteoporosis agent, e.g. a hormonal agent, such as raloxifene, or a biphosphonate, such as alendronate;
- (i) a tryptase inhibitor; (ii) a platelet activating factor (PAF) antagonist; (iii) an interleukin converting enzyme (ICE) inhibitor; (iv) an IMPDH inhibitor; (v) an adhesion molecule inhibitors including VLA-4 antagonist; (vi) a cathepsin; (vii) a kinase inhibitor, e.g. an inhibitor of tyrosine kinases (such as Btk, Itk, Jak3 MAP examples of inhibitors might include Gefitinib, Imatinib mesylate), a serine / threonine kinase (e.g. an inhibitor of MAP kinase, such as p38, JNK, protein kinases A, B and C and IKK), or a kinase involved in cell cycle regulation (e.g. a cylin dependent kinase); (viii) a glucose-6 phosphate dehydrogenase inhibitor; (ix) a kinin-B.subl. - and/or B.sub2. -receptor antagonist; (x) an anti-gout agent, e.g. colchicine; (xi) a xanthine oxidase inhibitor, e.g: allopurinol; (xii) a uricosuric agent, e.g. probenecid, sulfinpyrazone, and/or benzbromarone; (xiii) a growth hormone secretagogue; (xiv) transforming growth factor (TGFβ); (xv) platelet-derived growth factor (PDGF); (xvi) fibroblast growth factor, e.g. basic fibroblast growth factor (bFGF); (xvii) granulocyte macrophage colony stimulating factor (GM-CSF); (xviii) capsaicin cream; (xix) a tachykinin NK.sub1. and/or NK.sub3. receptor antagonist, such as NKP-608C, SB-233412 (talnetant) and/or D-4418; (xx) an elastase inhibitor, e.g. UT-77 and/or ZD-0892; (xxi) a TNF-alpha converting enzyme inhibitor (TACE); (xxii) induced nitric oxide synthase (iNOS) inhibitor or (xxiii) a chemoattractant receptor-homologous molecule expressed on TH2 cells (such as a CRTH2 antagonist); (xxiv) an inhibitor of a P38 (xxv) agent modulating the function of Toll-like receptors (TLR) and (xxvi) an agent modulating the activity of purinergic receptors, such as P2X7; (xxvii) an inhibitor of transcription factor activation, such as NFkB, API, and/or STATS.

An inhibitor may be specific or may be a mixed inhibitor, e.g. an inhibitor targeting more than one of the molecules (e.g. receptors) or molecular classes mentioned above.

The binding member could also be used in association with a chemotherapeutic agent or another tyrosine kinase inhibitor in co-administration or in the form of an immunoconjugate. Fragments of said antibody could also be use in bispecific antibodies obtained by recombinant mechanisms or biochemical coupling and then associating the specificity of the above described antibody with the specificity of other antibodies able to recognize other molecules involved in the activity for which IgE is associated.

For treatment of an inflammatory disease, e.g. rheumatoid arthritis, osteoarthritis, asthma, allergic rhinitis, chronic obstructive pulmonary disease (COPD), or psoriasis, a binding member of the invention may be combined with one or more agents, such as non-steroidal anti-inflammatory agents (hereinafter NSAIDs) including non-selective cyclo-oxygenase (COX)-1 / COX-2 inhibitors whether applied topically or systemically, such as piroxicam, diclofenac, propionic acids, such as naproxen, flurbiprofen, fenoprofen, ketoprofen and ibuprofen, fenamates, such as mefenamic acid, indomethacin, sulindac, azapropazone, pyrazolones, such as phenylbutazone, salicylates, such as aspirin); selective COX-2 inhibitors (such as meloxicam, celecoxib, rofecoxib, valdecoxib, lumarocoxib, parecoxib and etoricoxib); cyclo-oxygenase inhibiting nitric oxide donors (CINODs); glucocorticosteroids (whether administered by topical, oral, intramuscular, intra-venous or intra-articular routes); methotrexate, leflunomide; hydroxychloroquine, d-penicillamine, auranofin or other parenteral or oral gold preparations; analgesics; diacerein; intra-articular therapies, such as hyaluronic acid derivatives; and nutritional supplements, such as glucosamine.

A binding member of the invention and one or more of the above additional medicinal components may be used in the manufacture of a medicament. The medicament may be for separate or combined administration to an individual, and accordingly may comprise the binding member and the additional component as a combined preparation or as separate preparations. Separate preparations may be used to facilitate separate and sequential or simultaneous administration, and allow administration of the components by different routes e.g. oral and parenteral administration.

In accordance with the present invention, compositions provided may be administered to mammals. Administration is normally in a "therapeutically effective amount", this being sufficient to show benefit to a patient. Such benefit may be at least amelioration of at least one symptom. The actual amount administered, and rate and time-course of administration, will depend on the nature and severity of what is being treated, the particular mammal being treated, the clinical condition of the individual patient, the cause of the disorder, the site of delivery of the composition, the type of binding member, the method of administration, the scheduling of administration and other factors known to medical practitioners. Prescription of treatment, e.g. decisions on dosage etc, is within the responsibility of general practitioners and other medical doctors and may depend on the severity of the symptoms and/or progression of a disease being treated. Appropriate doses of antibody are well known in the art [67, 68]. Specific dosages indicated herein or in the Physician's Desk Reference (2003) as appropriate for the type of medicament being administered may be used. A therapeutically effective amount or suitable dose of a binding member of the invention can be determined by comparing its *in vitro* activity and *in vivo* activity in an animal model. Methods for extrapolation of effective dosages in mice and other test animals to humans are known. The precise dose will depend upon a number of factors, including whether the antibody is for diagnosis, prevention or for treatment, the size and location of the area to be treated, the precise nature of the antibody (e.g. whole antibody, fragment or diabody) and the nature of any detectable label or other molecule attached to the antibody. A typical antibody dose will be in the range 100 μg to 1 g for systemic applications, and 1 μg to 1 mg for topical applications. An initial higher loading dose, followed by one or more lower doses, may be administered. Typically, the antibody will be a whole antibody, e.g. the IgG1 isotype. This is a dose for a single treatment of an adult patient, which may be proportionally adjusted for children and infants, and also adjusted for other antibody formats in proportion to molecular weight. Treatments may be repeated at daily, twice-weekly, weekly or monthly intervals, at the discretion of the physician. Treatments may be every two to four weeks for subcutaneous administration and every four to eight weeks for intra-venous administration. Treatment may be periodic, and the period between administrations is about two weeks or more, e.g. about three weeks or more, about four weeks or more, or about once a month. Treatment may be given before, and/or after surgery, and/or may be administered or applied directly at the anatomical site of surgical treatment.

**Brief Description of the Tables and Figures**

| | |
|---|---|
| Table 1a-f | lists the amino acid sequences of the heavy chain CDRs and the light chain CDRs of each of antibodies 1-36. |
| Table 2a-f | lists the amino acid sequences of the light chain of each of antibodies 1-36. |
| Table 3a-h | lists the amino acid sequences of the heavy chain of each of antibodies 1-36. |
| Table 4 | shows sequences of exemplary binding members of the invention as shown in the appended sequence listing, in which SEQ ID NOS correspond as shown in Table 4. |
| Table 4b | shows the VL DNA and VL amino sequences of exemplary binding members of the invention from the provisional applicaton which are shown in the appended sequence listing, in which SEQ ID NOS correspond as shown in Table 4b. |
| Table 5a | shows example potencies of clones identified from the targeted mutagenesis libraries when tested in the Receptor-ligand binding HTRF® Assays. |
| Table 5b | shows the binding affinity (KD) for exemplary binding members of the invention to human IgE and cynomolgus monkey IgE, using SPR (BIACORE). Table 5b further shows the potency, expressed as IC50, for exemplary binding members of the invention, in an RBL-ER51 calcium signalling assay (at 4 hours with 25 ng/ml human or 100ng/ml cynomolgus monkey IgE). |
| Table 6 | shows example binding affinity calculation using BIAcore and potency measurements using RBL-ER51 calcium signalling assay for germlined antibodies. |
| Table 7. | shows summary study design for Assessment of the general safety and capacity of anti-IgE monoclonal antibodies (mAbs) |
| Figure 1: | relates to Example 2.6 and shows the molar concentration of antibody expressed as a log on the x-axis and the peak height in an RBL-ER51 calcium signalling assay on the y axis. The open squares relate to antibody 11, the crosses an irrelevant IgG1 control antibody and the inverted open triangles a anti-IgE cross-linking antibody (Biosource). Note that the open squares and crosses are superimposed on one another in this figure. |
| Figure 2: | shows the sequence of Cynomolgus Cε3-4 FLAG His10. |
| Figure 3: | shows the sequence of variable heavy chain that encodes human anti-oestradiaol scFv (D12_VH) and one of cynomologous IgHE gene haplotype, (cyIgHE TQ). |
| Figure 4: | shows the sequence of variable heavy chain that encodes human anti-oestradiaol scFv (D12_VH) and one of cynomologous IgHE gene haplotype,cyIgHE ME. |
| Figure 5: | shows the sequence of the variable light chain of Human anti-eostradiol scFv (D12_VL) and cynomolgus lambda constant region genes cyIGLC 4, Sequence Range: 1 to 708. |
| Figure 6: | shows the sequence of the variable light chain of Human anti-eostradiol scFv (D12_VL) and cynomolgus lambda constant region genes D12_VL cyIGLC 7. |
| Figure 7. | relates to Example 4 and shows the percentage inhibition of maximum IgE expression in B cells not treated with blocking anti-IgE wherein the x axis is the concentration of human interleukin 4 in ng/ml and the y axis is the percentage of cells which are both CD19 and IgE positive. The crosses relate to the control with no Antibody 33 present, the open circles relates to the shift induced by Antibody 3 3 at 0.5 μg/ml and the open squares the shift induced by Antibody 33 at 5μg/ml. |
| Figure 8: | Relates to Example 5 and shows mean toxicokinetics profiles of E85_50 $IgG_1$ E85_50 $IgG_2$ and Antibody 33 following 1 mg/kg (Day 1), 30 mg/kg (Day 8), and 100 mg/kg (Day 16 and beyond) doses in cynomolgus monkeys. Error bars represent standard deviations. The y-axis is serum concentration of antibody and the x axis is time in days following the first dose. Group 1 (E85_50 IgG1) is shown as filled circles, Group 2 (E85_50 IgG2) is shown as open triangles and Group 3 (Antibody 33) is shown as filed squares. |
| Figure 9: | Relates to Example 5 and shows mean free IgE profiles in cynomolgus monkeys receiving weekly doses of E85_50 $IgG_1$, E85_50 $IgG_2$ and Antibody 33 (1 mg/kg on Day 1, 30 mg/kg on Day 8, and 100 mg/kg on Day 16 and beyond). Error bars are standard deviations. The y-axis is IgE concentration in ng/ml and the x axis is time in days. Group 1 (E85_50 IgG1) is shown as filled circles, Group 2 (E85_50 IgG2) is shown as open triangles and Group 3 (Antibody 33) is shown as filed squares. |
| Figure 10: | Relates to Example 5 and shows a plot of platelet numbers ($x10^9$/L) expressed as a percentage change from the mean of the 2 pre-dose values versus plasma concentration from an animal in Group 1 (Antibody 33-treated). This plot is representative of the other 16 animals across the 3 groups that showed no significant effect on platelets. The x-axis shows time in hours, the left y axis the level of platelets as a percentage change from the mean level pre-treatment and the left y-ax is the concentration of anti-IgE antibody in nmol/L. The closed squares show the platelet concentration and the filled diamonds the con- |

centration of anti-IgE antibody. The closed triangles show the dosing of the anti-IgE antibody in mg/kg.

## Examples

Naïve human single chain Fv (scFv) phage display libraries cloned in to a phagemid vector based on the filamentous phage M13 were used for selections [69, 70]).

Anti-IgE specific scFv antibodies are isolated from the phage display libraries using a series of selection cycles on recombinant human IgE.

Selected scFv antibodies are optimized for binding to human IgE and/or for potency, and are reformatted as IgG antibodies.

## SEQUENCES

Sequences of exemplary binding members of the invention are shown in the appended sequence listing, in which SEQ ID NOS correspond as follows, wherein:

i) where an antibody number is followed by GL, for example 33GL this refers to the antibody wherein one or more of the residues have been mutated back to the germline configuration, in general where GL is used all non-germline residues which can be mutated back to germline without appreciable loss of activity have been germlined;

Table 4a

| Antibody | SEQ ID No. | Description |
|---|---|---|
| 1 | 1 | VH/DNA |
| 1 | 2 | VH/amino acid |
| 1 | 3 | HCDR1 |
| 1 | 4 | HCDR2 |
| 1 | 5 | HCDR3 |
| 1 | 6 | VL/DNA |
| 1 | 353 | VL/amino acid |
| 1 | 354 | LCDR1 |
| 1 | 355 | LCDR2 |
| 1 | 356 | LCDR3 |
| 2 | 297 | VH/DNA |
| 2 | 298 | VH/amino acid |
| 2 | 299 | HCDR1 |
| 2 | 300 | HCDR2 |
| 2 | 301 | HCDR3 |
| 2 | 357 | VL/DNA |
| 2 | 358 | VL/amino acid |
| 2 | 304 | LCDR1 |
| 2 | 305 | LCDR2 |
| 2 | 306 | LCDR3 |
| 3 | 337 | VH/DNA |
| 3 | 338 | VH/amino acid |
| 3 | 339 | HCDR1 |

(continued)

| Antibody | SEQ ID No. | Description |
|---|---|---|
| 3 | 340 | HCDR2 |
| 3 | 341 | HCDR3 |
| 3 | 359 | VL/DNA |
| 3 | 360 | VL/amino acid |
| 3 | 344 | LCDR1 |
| 3 | 345 | LCDR2 |
| 3 | 346 | LCDR3 |
| 4 | 317 | VH/DNA |
| 4 | 318 | VH/amino acid |
| 4 | 319 | HCDR1 |
| 4 | 320 | HCDR2 |
| 4 | 321 | HCDR3 |
| 4 | 361 | VL/DNA |
| 4 | 362 | VL/amino acid |
| 4 | 324 | LCDR1 |
| 4 | 325 | LCDR2 |
| 4 | 326 | LCDR3 |
| 5 | 327 | VH/DNA |
| 5 | 328 | VH/amino acid |
| 5 | 329 | HCDR1 |
| 5 | 330 | HCDR2 |
| 5 | 331 | HCDR3 |
| 5 | 363 | VL/DNA |
| 5 | 364 | VL/amino acid |
| 5 | 334 | LCDR1 |
| 5 | 335 | LCDR2 |
| 5 | 336 | LCDR3 |
| 6 | 117 | VH/DNA |
| 6 | 118 | VH/amino acid |
| 6 | 119 | HCDR1 |
| 6 | 120 | HCDR2 |
| 6 | 121 | HCDR3 |
| 6 | 365 | VL/DNA |
| 6 | 366 | VL/amino acid |
| 6 | 124 | LCDR1 |
| 6 | 125 | LCDR2 |
| 6 | 126 | LCDR3 |
| 7 | 307 | VH/DNA |

(continued)

| Antibody | SEQ ID No. | Description |
|---|---|---|
| 7 | 308 | VH/amino acid |
| 7 | 309 | HCDR1 |
| 7 | 310 | HCDR2 |
| 7 | 311 | HCDR3 |
| 7 | 367 | VL/DNA |
| 7 | 368 | VL/amino acid |
| 7 | 314 | LCDR1 |
| 7 | 315 | LCDR2 |
| 7 | 316 | LCDR3 |
| 8 | 27 | VH/DNA |
| 8 | 28 | VH/amino acid |
| 8 | 29 | HCDR |
| 8 | 30 | HCDR2 |
| 8 | 31 | HCDR3 |
| 8 | 369 | VL/DNA |
| 8 | 370 | VL/amino acid |
| 8 | 34 | LCDR1 |
| 8 | 35 | LCDR2 |
| 8 | 36 | LCDR3 |
| 9 | 67 | VH/DNA |
| 9 | 68 | VH/amino acid |
| 9 | 69 | CDR1 |
| 9 | 70 | HCDR2 |
| 9 | 71 | HCDR3 |
| 9 | 371 | VL/DNA |
| 9 | 372 | VL/amino acid |
| 9 | 74 | LCDR1 |
| 9 | 75 | LCDR2 |
| 9 | 76 | LCDR3 |
| 10 | 7 | VH/DNA |
| 10 | 8 | VH/amino acid |
| 10 | 9 | HCDR1 |
| 10 | 10 | HCDR2 |
| 10 | 11 | HCDR3 |
| 10 | 373 | VL/DNA |
| 10 | 374 | VL/amino acid |
| 10 | 14 | LCDR1 |
| 10 | 15 | LCDR2 |

(continued)

| Antibody | SEQ ID No. | Description |
|---|---|---|
| 10 | 16 | LCDR3 |
| 11 | 47 | VH/DNA |
| 11 | 48 | VH/amino acid |
| 11 | 49 | HCDR1 |
| 11 | 50 | HCDR2 |
| 11 | 51 | HCDR3 |
| 11 | 375 | VL/DNA |
| 11 | 376 | VL/amino acid |
| 11 | 54 | LCDR1 |
| 11 | 55 | LCDR2 |
| 11 | 56 | LCDR3 |
| 12 | 287 | VH/DNA |
| 12 | 288 | VH/amino acid |
| 12 | 289 | HCDR1 |
| 12 | 290 | HCDR2 |
| 12 | 291 | HCDR3 |
| 12 | 377 | VL/DNA |
| 12 | 378 | VL/amino acid |
| 12 | 294 | LCDR1 |
| 12 | 295 | LCDR2 |
| 12 | 296 | LCDR3 |
| 13 | 157 | VH/DNA |
| 13 | 158 | VH/amino acid |
| 13 | 159 | HCDR1 |
| 13 | 160 | HCDR2 |
| 13 | 161 | HCDR3 |
| 13 | 379 | VL/DNA |
| 13 | 380 | VL/amino acid |
| 13 | 164 | LCDR1 |
| 13 | 165 | LCDR2 |
| 13 | 166 | LCDR3 |
| 14 | 267 | VH/DNA |
| 14 | 268 | VH/amino acid |
| 14 | 269 | HCDR1 |
| 14 | 270 | HCDR2 |
| 14 | 271 | HCDR3 |
| 14 | 381 | VL/DNA |
| 14 | 382 | VL/amino acid |

(continued)

| Antibody | SEQ ID No. | Description |
|---|---|---|
| 14 | 274 | LCDR1 |
| 14 | 275 | LCDR2 |
| 14 | 276 | LCDR3 |
| 15 | 167 | VH/DNA |
| 15 | 168 | VH/amino acid |
| 15 | 169 | HCDR1 |
| 15 | 170 | HCDR2 |
| 15 | 171 | HCDR3 |
| 15 | 383 | VL/DNA |
| 15 | 384 | VL/amino acid |
| 15 | 174 | LCDR1 |
| 15 | 175 | LCDR2 |
| 15 | 176 | LCDR3 |
| 16 | 37 | VH/DNA |
| 16 | 38 | VH/amino acid |
| 16 | 39 | HCDR1 |
| 16 | 40 | HCDR2 |
| 16 | 41 | HCDR3 |
| 16 | 385 | VL/DNA |
| 16 | 386 | VL/amino acid |
| 16 | 44 | LCDR1 |
| 16 | 45 | LCDR2 |
| 16 | 46 | LCDR3 |
| 17 | 127 | VH/DNA |
| 17 | 128 | VH/amino acid |
| 17 | 129 | HCDR1 |
| 17 | 130 | HCDR2 |
| 17 | 131 | HCDR3 |
| 17 | 387 | VL/DNA |
| 17 | 388 | VL/amino acid |
| 17 | 134 | LCDR1 |
| 17 | 135 | LCDR2 |
| 17 | 136 | LCDR3 |
| 18 | 77 | VH/DNA |
| 18 | 78 | VH/amino acid |
| 18 | 79 | HCDR1 |
| 18 | 80 | HCDR2 |
| 18 | 81 | HCDR3 |

(continued)

| Antibody | SEQ ID No. | Description |
|---|---|---|
| 18 | 389 | VL/DNA |
| 18 | 390 | VL/amino acid |
| 18 | 84 | LCDR1 |
| 18 | 85 | LCDR2 |
| 18 | 86 | LCDR3 |
| 19 | 137 | VH/DNA |
| 19 | 138 | VH/amino acid |
| 19 | 139 | HCDR1 |
| 19 | 140 | HCDR2 |
| 19 | 141 | HCDR3 |
| 19 | 391 | VL/DNA |
| 19 | 392 | VL/amino acid |
| 19 | 144 | LCDR1 |
| 19 | 145 | LCDR2 |
| 19 | 146 | LCDR3 |
| 20 | 187 | VH/DNA |
| 20 | 188 | VH/amino acid |
| 20 | 189 | HCDR1 |
| 20 | 190 | HCDR2 |
| 20 | 191 | HCDR3 |
| 20 | 393 | VL/DNA |
| 20 | 394 | VL/amino acid |
| 20 | 194 | LCDR1 |
| 20 | 195 | LCDR2 |
| 20 | 196 | LCDR3 |
| 21 | 197 | VH/DNA |
| 21 | 198 | VH/amino acid |
| 21 | 199 | HCDR1 |
| 21 | 200 | HCDR2 |
| 21 | 201 | HCDR3 |
| 21 | 395 | VL/DNA |
| 21 | 396 | VL/amino acid |
| 21 | 204 | LCDR1 |
| 21 | 205 | LCDR2 |
| 21 | 206 | LCDR3 |
| 22 | 97 | VH/DNA |
| 22 | 98 | VH/amino acid |
| 22 | 99 | HCDR1 |

(continued)

| Antibody | SEQ ID No. | Description |
|---|---|---|
| 22 | 100 | HCDR2 |
| 22 | 101 | HCDR3 |
| 22 | 397 | VL/DNA |
| 22 | 398 | VL/amino acid |
| 22 | 104 | LCDR1 |
| 22 | 105 | LCDR2 |
| 22 | 106 | LCDR3 |
| 23 | 17 | VH/DNA |
| 23 | 18 | VH/amino acid |
| 23 | 19 | HCDR1 |
| 23 | 20 | HCDR2 |
| 23 | 21 | HCDR3 |
| 23 | 399 | VL/DNA |
| 23 | 400 | VL/amino acid |
| 23 | 24 | LCDR1 |
| 23 | 25 | LCDR2 |
| 23 | 26 | LCDR3 |
| 24 | 87 | VH/DNA |
| 24 | 88 | VH/amino acid |
| 24 | 89 | HCDR1 |
| 24 | 90 | HCDR2 |
| 24 | 91 | HCDR3 |
| 24 | 401 | VL/DNA |
| 24 | 402 | VL/amino acid |
| 24 | 94 | LCDR1 |
| 24 | 95 | LCDR2 |
| 24 | 96 | LCDR3 |
| 25 | 57 | VH/DNA |
| 25 | 58 | VH/amino acid |
| 25 | 59 | CDR1 |
| 25 | 60 | HCDR2 |
| 25 | 61 | HCDR3 |
| 25 | 403 | VL/DNA |
| 25 | 404 | VL/amino acid |
| 25 | 64 | LCDR1 |
| 25 | 65 | LCDR2 |
| 25 | 66 | LCDR3 |
| 26 | 107 | VH/DNA |

(continued)

| Antibody | SEQ ID No. | Description |
|---|---|---|
| 26 | 108 | VH/amino acid |
| 26 | 109 | HCDR1 |
| 26 | 110 | HCDR2 |
| 26 | 111 | HCDR3 |
| 26 | 405 | VL/DNA |
| 26 | 406 | VL/amino acid |
| 26 | 114 | LCDR1 |
| 26 | 115 | LCDR2 |
| 26 | 116 | LCDR3 |
| 27 | 217 | VH/DNA |
| 27 | 218 | VH/amino acid |
| 27 | 219 | HCDR1 |
| 27 | 220 | HCDR2 |
| 27 | 221 | HCDR3 |
| 27 | 407 | VL/DNA |
| 27 | 408 | VL/amino acid |
| 27 | 224 | LCDR1 |
| 27 | 225 | LCDR2 |
| 27 | 226 | LCDR3 |
| 28 | 247 | VH/DNA |
| 28 | 248 | VH/amino acid |
| 28 | 249 | HCDR1 |
| 28 | 250 | HCDR2 |
| 28 | 251 | HCDR3 |
| 28 | 409 | VL/DNA |
| 28 | 410 | VL/amino acid |
| 28 | 254 | LCDR1 |
| 28 | 255 | LCDR2 |
| 28 | 256 | LCDR3 |
| 29 | 227 | VH/DNA |
| 29 | 228 | VH/amino acid |
| 29 | 229 | HCDR1 |
| 29 | 230 | HCDR2 |
| 29 | 231 | HCDR3 |
| 29 | 411 | VL/DNA |
| 29 | 412 | VL/amino acid |
| 29 | 234 | LCDR1 |
| 29 | 235 | LCDR2 |

(continued)

| Antibody | SEQ ID No. | Description |
|---|---|---|
| 29 | 236 | LCDR3 |
| 30 | 237 | VH/DNA |
| 30 | 238 | VH/amino acid |
| 30 | 239 | HCDR1 |
| 30 | 240 | HCDR2 |
| 30 | 241 | HCDR3 |
| 30 | 413 | VL/DNA |
| 30 | 414 | VL/amino acid |
| 30 | 244 | LCDR1 |
| 30 | 245 | LCDR2 |
| 30 | 246 | LCDR3 |
| 31 | 444 | VH/DNA |
| 31 | 445 | VH/amino acid |
| 31 | 179 | HCDR1 |
| 31 | 180 | HCDR2 |
| 31 | 181 | HCDR3 |
| 31 | 415 | VL/DNA |
| 31 | 416 | VL/amino acid |
| 31 | 184 | LCDR1 |
| 31 | 185 | LCDR2 |
| 31 | 186 | LCDR3 |
| 32 | 207 | VH/DNA |
| 32 | 208 | VH/amino acid |
| 32 | 209 | HCDR1 |
| 32 | 210 | HCDR2 |
| 32 | 211 | HCDR3 |
| 32 | 417 | VL/DNA |
| 32 | 418 | VL/amino acid |
| 32 | 214 | LCDR1 |
| 32 | 215 | LCDR2 |
| 32 | 216 | LCDR3 |
| 33 | 277 | VH/DNA |
| 33 | 278 | VH/amino acid |
| 33 | 279 | HCDR1 |
| 33 | 280 | HCDR2 |
| 33 | 281 | HCDR3 |
| 33 | 419 | VL/DNA |
| 33 | 420 | VL/amino acid |

(continued)

| Antibody | SEQ ID No. | Description |
|---|---|---|
| 33 | 284 | LCDR1 |
| 33 | 285 | LCDR2 |
| 33 | 286 | LCDR3 |
| 34 | 147 | VH/DNA |
| 34 | 148 | VH/amino acid |
| 34 | 149 | HCDR1 |
| 34 | 150 | HCDR2 |
| 34 | 151 | HCDR3 |
| 34 | 421 | VL/DNA |
| 34 | 422 | VL/amino acid |
| 34 | 154 | LCDR1 |
| 34 | 155 | LCDR2 |
| 34 | 156 | LCDR3 |
| 33GL | 425/277 | VH/DNA |
| 33GL | 426/278 | VH/amino acid |
| 33GL | 427/279 | HCDR1 |
| 33GL | 428/280 | HCDR2 |
| 33GL | 429/281 | HCDR3 |
| 33GL | 430 | VL/DNA |
| 33GL | 431 | VL/amino acid |
| 33GL | 350 | LCDR1 |
| 33GL | 351 | LCDR2 |
| 33GL | 352 | LCDR3 |
| 36 | 257 | VH/DNA |
| 36 | 258 | VH/amino acid |
| 36 | 259 | HCDR1 |
| 36 | 260 | HCDR2 |
| 36 | 261 | HCDR3 |
| 36 | 423 | VL/DNA |
| 36 | 424 | VL/amino acid |
| 36 | 264 | LCDR1 |
| 36 | 265 | LCDR2 |
| 36 | 266 | LCDR3 |
|  | 432 | Cynomolgus Ce3-4 FLAG His10 nucleotide |
|  | 433 | Cynomolgus Ce3-4 FLAG His 10 protein |
|  | 434 | D12_VHcyIgHE TQ nucleotide |
|  | 435 | D12_VHcyIgHE TQ protein |
|  | 436 | D12_VH cy IgHE ME nucleotide |

(continued)

| Antibody | SEQ ID No. | Description |
|---|---|---|
| | 437 | D12_VH cy IgHE ME protein |
| | 438 | D12 VL cyIgLC 4 nucleotide |
| | 439 | D12 VL cyIgLC 4 protein |
| | 440 | D12_VL cyIgLC 7 nucleotide |
| | 441 | D12_VL cyIgLC 7 protein |
| | 442 | FceRI_Fc (NSO) nucleotide |
| | 443 | FceRI_Fc (NSO) protein |

In the sequence listing filed with the provisional application the sequences of the 3' ggt codon, and corresponding Glycine residue, shown in the nucleotide and amino acid sequence for the VL DNA and corresponding VL aminio acid were included in the expressed scFv and IgG sequences of this antibody. The C terminal Glycine residue of the sequence corresponds to Kabat residue 108. This terminal glycine is not part of the VL sequence and has been removed from the sequences listed in Table 4a. The sequences for VL DNA and VL amino acid from the provisional application are included with the sequence listing and are listed in Table 4b below. The origin of this residue and its encoding triplet ggt is explained below.

To express the light chain of the IgG, a nucleotide sequence encoding the antibody light chain was provided, comprising a first exon encoding the VL domain, a second exon encoding the CL domain, and an intron separating the first exon and the second exon. Under normal circumstances, the intron is spliced out by cellular mRNA processing machinery, joining the 3' end of the first exon to the 5' end of the second exon. Thus, when DNA having the said nucleotide sequence was expressed as RNA, the first and second exons were spliced together. Translation of the spliced RNA produces a polypeptide comprising the VL domain and CL domain. After splicing, the Gly at Kabat residue 108 is encoded by the last base (g) of the VL domain framework 4 sequence and the first two bases (gt) of the CL domain.

Therefore, the Glycine residue at Kabat residue 108 was included in the sequence lisings of the VL sequences in the provisional application but as described above it should not be considered to be the C terminal residue of the VL domain of the antibody molecule and thus has been deleted from sequence listings in Table 4a.

**Table 4b**

| SEQUENCE ID NO. | ANTIBODY | DESCRIPTION |
|---|---|---|
| 6 | 1 | VL/DNA |
| 12 | 10 | VL/DNA |
| 13 | 10 | VL/amino acid |
| 22 | 23 | VL/DNA |
| 23 | 23 | VL/amino acid |
| 32 | 8 | VL/DNA |
| 33 | 8 | VL/amino acid |
| 42 | 16 | VL/DNA |
| 43 | 16 | VL/amino acid |
| 52 | 11 | VL/DNA |
| 53 | 11 | VL/amino acid |
| 62 | 25 | VL/DNA |
| 63 | 25 | VL/amino acid |
| 72 | 9 | VL/DNA |
| 73 | 9 | VL/amino acid |
| 82 | 18 | VL/DNA |

(continued)

| SEQUENCE ID NO. | ANTIBODY | DESCRIPTION |
|---|---|---|
| 83 | 18 | VL/amino acid |
| 92 | 24 | VL/DNA |
| 93 | 24 | VL/amino acid |
| 102 | 22 | VL/DNA |
| 103 | 22 | VL/amino acid |
| 112 | 26 | VL/DNA |
| 113 | 26 | VL/amino acid |
| 122 | 6 | VL/DNA |
| 123 | 6 | VL/amino acid |
| 132 | 17 | VL/DNA |
| 133 | 17 | VL/amino acid |
| 142 | 19 | VL/DNA |
| 143 | 19 | VL/amino acid |
| 152 | 34 | VL/DNA |
| 153 | 34 | VL/amino acid |
| 162 | 13 | VL/DNA |
| 163 | 13 | VL/amino acid |
| 172 | 15 | VL/DNA |
| 173 | 15 | VL/amino acid |
| 182 | 31 | VL/DNA |
| 183 | 31 | VL/amino acid |
| 192 | 20 | VL/DNA |
| 193 | 20 | VL/amino acid |
| 202 | 21 | VL/DNA |
| 203 | 21 | VL/amino acid |
| 212 | 32 | VL/DNA |
| 213 | 32 | VL/amino acid |
| 222 | 27 | VL/DNA |
| 223 | 27 | VL/amino acid |
| 232 | 29 | VL/DNA |
| 233 | 29 | VL/amino acid |
| 242 | 30 | VL/DNA |
| 243 | 30 | VL/amino acid |
| 252 | 28 | VL/DNA |
| 253 | 28 | VL/amino acid |
| 262 | 36 | VL/DNA |
| 263 | 36 | VL/amino acid |
| 272 | 14 | VL/DNA |

(continued)

| SEQUENCE ID NO. | ANTIBODY | DESCRIPTION |
|---|---|---|
| 273 | 14 | VL/amino acid |
| 282 | 33 | VL/DNA |
| 283 | 33 | VL/amino acid |
| 292 | 12 | VL/DNA |
| 293 | 12 | VL/amino acid |
| 302 | 2 | VL/DNA |
| 303 | 2 | VL/amino acid |
| 312 | 7 | VL/DNA |
| 313 | 7 | VL/amino acid |
| 322 | 4 | VL/DNA |
| 323 | 4 | VL/amino acid |
| 332 | 5 | VL/DNA |
| 333 | 5 | VL/amino acid |
| 342 | 3 | VL/DNA |
| 343 | 3 | VL/amino acid |
| 348 | 35 | VL/DNA |
| 349 | 35 | VL/amino acid |

In the sequence listing in the provisional application the sequences listed as Antibodies 35 is listed in Table 4a as Antibody 33GL. Antibody 33GL shares a common VH domain to Antibody 33 and therefore Sequence ID 347 was empty in the sequence lising for the provisional application. Consequently the VH domain sequences for 33GL are SEQ ID NO 277 (DNA) and SEQ ID NO 278 (Protein). This has been corrected in Table 4a.

**Example 1. Lead Isolation**

*1.1 Selections*

Naïve human single chain Fv (scFv) phage display libraries cloned in to a phagemid vector based on the filamentous phage M13 were used for selections (Vaughan et al., Nature Biotechnology 14: 309-314 (1996), Hutchings, Antibody Engineering, R.Kontermann and S. Dubel, Editors. 2001, Springer Laboratory Manuals, Berlin. P93). Anti-IgE specific scFv antibodies were isolated from the phage display libraries using a series of selection cycles on either plasma purified human IgEκ (Calbiochem) or plasma purified human IgEλ (Biodesign) essentially as previously described by Vaughan et al (Vaughan et al., Nature Biotechnology 14: 309-314 (1996). In brief, for panning selections, human IgE in PBS (Dulbecco's PBS, pH7.4) was adsorbed onto wells of a Maxisorp microtitre plate (Nunc) overnight at 4°C. Wells were washed with PBS then blocked for 1 h with PBS-Marvel (3% w/v). Purified phage in PBS-Marvel (3% w/v) were added to the wells and allowed to bind coated antigen for 1 h. Unbound phage were removed by a series of wash cycles using PBS-Tween (0.1% v/v) and PBS. Bound phage particles were eluted, infected into bacteria and rescued for the next round of selection (Vaughan et al., Nature Biotechnology 14: 309-314 (1996)). Alternate rounds of selection were performed using the kappa and lambda forms of IgE.

*1.2 Inhibition of IgE binding to FcεRI by unpurified scFv*

A representative number of individual scFv from the second round of selections were grown up in 96-well plates. ScFvs were expressed in the bacterial periplasm and screened for their inhibitory activity in a homogeneous FRET (Fluorescence resonance energy transfer) based human IgE/human FcεRI-binding assay. In this assay, samples competed for binding to human IgE (Calbiochem 401152) labelled with Europium Chelate (Perkin Elmer 1244-302), with human FcεRI-Fc (in house NS0 cell produced). The detailed assay method is provided in the Materials and Methods section.

*1.3 Inhibition of IgE binding to FcεRI by purified scFv*

ScFv which showed a significant inhibitory effect on the IgE:FcεRI interaction as unpurified periplasmic extracts, were subjected to DNA sequencing (Vaughan et al. 1996, Nature Biotechnology 14: 309-314), (Osbourn 1996;Immunotechnology. 2, 181-196). Unique scFvs were expressed again in bacteria and purified by affinity chromatography (as described by Bannister et al (2006) Biotechnology and bioengineering, 94. 931-937). The potencies of these samples were determined by competing a dilution series of the purified preparation against FcεRI (in house NS0 cell produced), for binding to human IgE (Calbiochem 401152) labelled with Europium Chelate (Perkin Elmer 1244-302). Purified scFv preparations e.g Antibody 1 were capable of inhibiting the IgE-FcεRI interaction. Detailed protocols are provided in Materials and Methods section.

*1.4 Reformatting of scFv to IgG*

Clones were converted from scFv to IgG format by sub-cloning the $V_H$ and $V_L$ domains into vectors expressing whole antibody heavy and light chains respectively. The $V_H$ domain was cloned into a vector (pEU15.1 1 or pEU9.2) containing the human heavy chain constant domains and regulatory elements to express whole IgG1 or IgG2 heavy chain in mammalian cells. Similarly, the $V_L$ domain was cloned into vector pEU4.4 for the expression of the human lambda light chain constant domain, with regulatory elements to express whole IgG light chain in mammalian cells. Vectors for the expression of heavy chains and light chains were originally described by Persic et al. (Persic, L., et al. (1997) Gene 187, 9-18). Cambridge Antibody Technology vectors have been engineered to include an EBV OriP element which, in combination with the EBNA1 protein, allows for episomal replication of the plasmid. To obtain IgGs, the heavy and light chain IgG expressing vectors were transfected into EBNA-HEK293 mammalian cells. IgGs were expressed and secreted into the medium. Harvests were pooled and filtered prior to purification. The IgG was purified using Protein A chromatography. Culture supernatants were loaded on a Ceramic Protein A column (BioSepra) and washed with 50 mM Tris-HCl pH 8.0, 250 mM NaCl. Bound IgG was eluted from the column using 0.1 M Sodium Citrate (pH 3.0) and neutralised by the addition of Tris-HCl (pH 9.0). The eluted material was buffer exchanged into PBS using Nap10 columns (Amersham, #17-0854-02) and the concentration of IgG was determined spectrophotometrically using an extinction coefficient based on the amino acid sequence of the IgG (Mach et al Anal. Biochem. 200(1): 20-26, 1992). The purified IgG were analysed for aggregation or degradation using SEC-HPLC and by SDS-PAGE.

*1.5 Inhibition of calcium signalling in RBL-ER51 cells by purified scFv and IgG*

The neutralisation potency of purified scFv and IgG preparations against human IgE bioactivity mediated through FcεRI was assessed using an RBL-ER51 calcium-signalling assay. RBL-2H3 cells (a rat basophilic cell line) were stably transfected with the human FcεRI (RBL-ER51 cells). Free IgE in the vicinity of the cells binds to the FcεRI on the cell surface and subsequent cross-linking of receptor-bound IgE leads to a calcium mobilisation that can be detected using a Fluorometric Imaging Plate Reader (FLIPR). A detailed description of the protocol is provided in the Materials and Methods section.

Purified scFv preparations of Antibody 1 were capable of inhibiting the IgE induced calcium signalling of the RBL-ER51 cells at the maximum concentration tested. When tested as a purified IgG, the $IC_{50}$ for Antibody 1 was calculated as being 267nM (n=3).

*1.6 Selectivity and species cross reactivity of antibodies in DELFIA® epitope competition assays*

The species cross reactivity and selectivity of antibodies to IgE and structurally related molecules; IgA, IgM, IgD and IgG, was established using DELFIA® epitope competition. assays. The assay determines relative cross reactivity by measuring inhibition of biotinylated IgE (plasma purified, BIODESIGN International), binding each immobilised anti-IgE antibody.

Titrations of purified IgA, IgM, IgD, and IgG (all Calbiochem) were tested in each assay to establish the specificity profile for each structurally related protein, as measured by IC50 values in the assay.

Titrations of IgE species including cynomolgus IgE Cε3-Cε4 domain (in house HEK-EBNA derived), human IgE Cε3-Cε4 domain (in house HEK-EBNA derived) and human IgE lambda (BIODESIGN International) were tested in each assay to establish the species cross-reactivity of the antibodies. Full-length human IgEλ produced an inhibition curve. No inhibition was observed with human or cynomolgus IgE Cε3-Cε4 domains or with any of the structurally related proteins. These data demonstrate that Antibody 1 binds to human IgEλ, although not to the Cε3-Cε4 domain. In addition, Antibody 1 does not bind to any of the most related human proteins to IgE. Details of the protocol are provided in the Materials and Methods section.

*1.7 Inhibition of IgE binding to CD23 by purified IgG*

IM9 cells (a human B cell line) were shown to express CD23 but not FcεRI under basal conditions. IgE binds to CD23 on the surface of IM9 cells. CD23-bound IgE can then be bound with anti-IgE- Phycoerythrin (Caltag) and detected by flow cytometry (FACSCalibur, BD Biosciences).

Antibodies were evaluated for inhibition of the IgE / CD23 interaction. A detailed protocol for this procedure is provided in Materials and Methods. In brief, titrations of the test IgG were mixed with IgE prior to incubation with IM9 cells. Following a 1 hour incubation, cells were washed and bound IgE was detected with anti-IgE-Phycoerythrin (Caltag). There was no detectable inhibition of IgE/CD23 interaction with antibody 1.

*1.8 Cross-linking of FcεRI-bound IgE*

Antibodies were evaluated for potential to cross-link FcεRI-bound IgE using an RBL-ER51 calcium-signalling assay. RBL-ER51 cells, described in materials and methods, were loaded with IgE. Antibodies were incubated with the IgE-loaded cells and assessed for their ability to stimulate a calcium response. Antibody 1 was not able to induce a detectable calcium response.

**Example 2. Antibody optimisation**

*2.1 Optimisation of parent clone*

Antibody 1 was optimised for improved affinity to IgE. This was achieved using either a targeted or random mutagenesis approach. For the targeted mutagenesis approach, large scFv-phage libraries were derived from Antibody 1 were created by oligonucleotide -directed mutagenesis of the variable heavy (VH) chain complementarity determining region 3 (CDR3) using standard molecular biology techniques as described by Clackson and Lowman (2204) Phage Display A Practical Approach, 2004. Oxford University Press.

The libraries were subjected to affinity-based phage display selections in order to select variants with higher affinity for human IgE. In consequence, these should show an improved inhibitory activity for IgE binding to its receptor. The selections were performed essentially as described previously (Thompson. Journal of Molecular Biology. 256:77-88, 1996). In brief, the scFv phage particles were incubated with recombinant biotinylated human -IgEλ(U266 derived [Ikeyama et. al. 1986. Molecular Immunology 23 (2); p159-167] and modified in house) in solution. ScFv-phage bound to antigen were then captured on streptavidin coated paramagnetic beads (Dynabeads® M280) following the manufacturer's recommendations. The selected scFv-phage particles were then rescued as described previously (Osboum, JK. Et al. Immunotechnology, 2(3):181-96, 1996), and the selection process was repeated in the presence of decreasing concentrations of biotinylated IgE (250 nM to 250 pM over 4 rounds). Further mutations were subsequently introduced into the variable heavy (VH) chain complementarity determining region 2 (CDR2) and variable light (VL) complementarity determining region 1 (CDR1) by site-directed mutagenesis using standard molecular biology techniques. For the random mutagenesis approach, large scFv ribosome display libraries derived from the lead clone were created by error-prone PCR of the variable heavy ($V_H$) and light ($V_L$) chain regions using standard molecular biology techniques.

The libraries were subjected to affinity-based ribosome display selections in order to select variants with higher affinity for IgE. In consequence, these should show an improved inhibitory activity for IgE binding its receptor. The selections were performed essentially as described previously (Hanes et al. 2000. Methods in Enzymology 328, 404). In brief, the mRNA-ribosome-scFv omplexes were incubated with recombinant biotinylated. human IgE λ( U266 derived [Ikeyama et. al. 1986. Molecular Immunology 23 (2); p159-167] and modified in house). mRNA-ribosome-scFv complexes bound to antigen were then captured on streptavidin-coated paramagnetic beads (Dynabeads® 280) following the manufacturer's recommendations. The selected mRNA-ribosome-scFv complexes were then dissociated and the mRNA was purified and used for reverse transcription and PCR amplification as previously described, (Hanes et al. 2000. Methods in Enzymology 328, 404). The selection process was repeated in the presence of decreasing concentrations of biotinylated-human IgE λ(100 nM to 100pM over 5 rounds).

*2.2 Identification of improved clones from the random mutagenesis using an antibody-ligand biochemical assay*

ScFv from the random mutagenesis selection outputs were sub-cloned into the pCantab6 vector (Cambridge Antibody Technology) and subsequently expressed in bacterial periplasm and screened in an epitope competition HTRF® (Homogeneous Time-Resolved Fluorescence) assay format for inhibition of human IgE (U266-derived [Ikeyama et. al. 1986. Molecular Immunology 23 (2); p159-167]) labelled with europium cryptate (CIS bio International 62EUSPEA), binding to anti human-IgE (Antibody 1, isolated in example 1). The detailed assay method is provided in the Materials and Methods section. ScFv that showed a significant inhibitory effect were subjected to DNA sequencing and unique scFv were prepared as purified preparations.

*2.3 Inhibition of IgE binding to FceR1 by purified scFv*

Purified scFv were tested in a receptor-ligand binding HTRF® (Homogeneous Time-Resolved Fluorescence) assay format for inhibition of either human IgE (U266-derived [Ikeyama et. al. 1986. Molecular Immunology 23 (2); p159-167]) or cyno IgE (recombinant, see materials and methods) labelled with europium cryptate (CIS bio International 62EUSPEA), binding to human FcεR1-Fc (in house NS0 cell produced). Example scFv potency data is included in Table 5a

***Table 5a:*** *Example potencies of clones identified from the random mutagenesis libraries when tested in the Receptor-ligand binding HTRF® Assays*

| Clone (non-germlined) | scFv Geomean (95% CI) $IC_{50}$ (nM) | |
|---|---|---|
| | Human IgE assay | Cynomolgus IgE assay |
| Antibody 1 | Weak/Incomplete | Weak/Incomplete |
| Antibody 2 | 7 (n=2) | 398 (n=2) |
| Antibody 3 | 5 (n=2) | 237 (n=2) |
| Antibody 4 | 5.4 (n=2) | 600 (n=2) |
| Antibody 5 | 6 (n=2) | 356 (n=2) |
| Antibody 6 | 8 (n=1) | Weak/Incomplete |
| Antibody 7 | 8 (n=1) | 569 (n=1) |
| Antibody 8 | 8 (n=1) | 438 (n=1) |
| Antibody 9 | 6 (n=2) | 138 (n=2) |
| Antibody 10 | 10 (n=1) | No Inhibition |
| Antibody 11 | 7 (n=1) | Weak/Incomplete |
| Antibody 12 | 8 (n=1) | 473 (n=1) |
| Antibody 13 | 9 (n=1) | Weak/Incomplete |
| Antibody 14 | 7 (n=1) | No Inhibition |
| Antibody 15 | 4 (n=2) | 135 (n=2) |
| Antibody 16 | 10 (n=2) | 280 (n=2) |
| Antibody 17 | 7 (n=2) | 264 (n=2) |
| Antibody 18 | 6 (n=1) | 129 (n=1) |
| Antibody 19 | 9 (n=1) | 197 (n=1) |
| Antibody 20 | 5 (n=1) | No Inhibition |
| Antibody 21 | 4 (n=1) | No Inhibition |
| Antibody 22 | 7 (n=1) | Weak/Incomplete |
| Antibody 23 | 7 (n=1) | 331 (n=1) |
| Antibody 24 | 4 (n=2) | 196 (n=2) |
| Antibody 25 | 6 (n=1) | 188 (n=1) |
| Antibody 26 | 8 (n=1) | 359 (n=1) |
| Antibody 27 | 2 (n=1) | 379 (n=1) |

(continued)

| Clone (non-germlined) | scFv Geomean (95% CI) $IC_{50}$ (nM) | |
|---|---|---|
| | **Human IgE assay** | **Cynomolgus IgE assay** |
| Antibody 28 | 4 (n=1) | No Inhibition |
| Antibody 29 | 6 (n=1) | No Inhibition |
| Antibody 30 | 4 (n=1) | No Inhibition |
| Antibody 31 | 9 (n=1) | No Inhibition |
| Antibody 33 | 14 (n=1) | 533 (n=1) |
| Antibody 34 | 3 (n=2) | 500 (n=2) |
| Antibody 36 | 8 (n=1) | 407 (n=1) |

*2.4 Inhibition of calcium signalling in RBL-ER51 cells by purified IgG*

After re-formatting as IgG, potencies of optimised clones were determined using a modified RBL-ER51 calcium signalling assay. This assay was adapted from the method used during lead isolation to improve sensitivity for detection of more potent antibodies. A detailed description of the protocol is provided in the Materials and Methods section. $IC_{50}$ potency data against human and cynomolgus IgE are given in Table 5b.

**Table 5b:** Binding affinity Calculation using BIAcore and Potency measurement using RBL-ER51 calcium signalling assay for optimised antibodies.

| Antibody | Biacore KD (nM) (Geomean) | | RBL-ER51 calcium signalling $IC_{50}$ (nM) Geomean (95% CI) | |
|---|---|---|---|---|
| | Human IgE | Cynomolgus IgE | Human IgE | Cynomolgus IgE |
| Antibody 9 | 79 | | 0.286 (0.28-0.29) n=3 | 4.5 (0.486-42.23) n=2 |
| Antibody 10 | 1.3 | | 1.5 (0.064-35.61) n=2 | na |
| Antibody 15 | 0.9 | 7 | 0.341 (0.206-0.56) n=5 | 5.4 (0.211-139.19) n=2 |
| Antibody 26 | | | 1.2 (0.378-3.84) n=2 | 17 (0.057-5174.93) n=2 |
| Antibody 33 | 0.8 | 2.3 | 0.112 (0.090-0.14) n=10 | 5.1 (2.86-9.12) n=5 |
| Antibody 34 | 26 | | 0.223 ((0.014-3.45) n=2 | 22 (0.090-5408) n=2 |

*2.5. Germlining*

The amino acid sequences of the $V_H$ and $V_L$ domains of the optimised anti-IgE antibodies were aligned to the known human germline sequences in the VBASE database (Tomlinson 1997; Journal of Molecular biology. 224. 487-499), and the closest germline was identified by sequence similarity. For the $V_H$ domains of the optimised antibody lineage this was Vh3 DP-47 (3-23). For the VL domains it was Vλ3 DPL23 (3r).

Without considering the Vernier residues(Foote & Winter 1992), which were left unchanged, there were no differences from germline in the frameworks of the VH domain and 6 in the VL domain of Antibody 1. All of the changes in the VL domain were reverted to the closest human germline sequence to identically match human antibodies. Germlining of these amino acid residues was carried out using standard site directed mutagenesis techniques with the appropriate mutagenic primers. Germlined IgG were then re-evaluated to confirm there had not been a reduction in potency or affinity.

Example affinities and potencies for germlined (GL) antibodies are provided in Table 6.

***Table 6:*** *Example binding affinity Calculation using BIAcore and Potency measurement using RBL-ER51 calcium signalling assay for germlined antibodies.*

| Antibody (germlined) | Biacore KD (nM) (Geomean) Human IgE | RBL-ER51 calcium signalling $IC_{50}$ (nM) Human IgE Geomean (95% CI) |
|---|---|---|
| *Antibody 33 GL* | 0.142 | 0.197 (0.154-0.25) |

*2.6 Inhibition of IgE binding to CD23 by purified IgG*

Some of these optimised antibodies were evaluated for inhibition of the IgE / CD23 interaction using the IM9 binding assay as previously described. Antibody 33 tested in this system was found to inhibit the IgE/CD23 interaction with an $IC_{50}$ of 83nM (n=2).

*2.7 Cross-linking of FεRI-bound IgE*

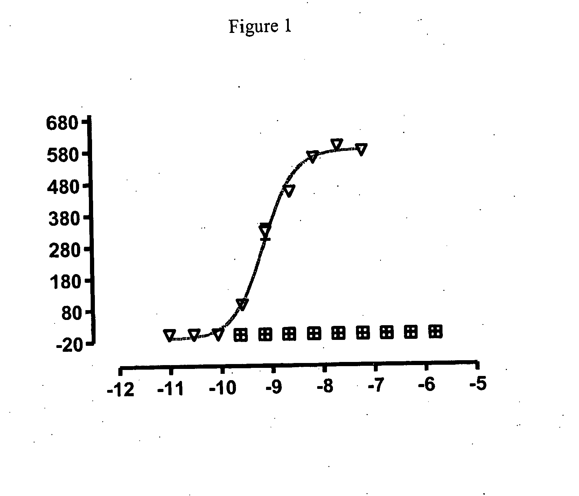

Optimised antibodies were evaluated for potential to cross-link FεERI-bound IgE using an RBL-ER51 calcium-signalling assay. RBL-ER51 cells, described in materials and methods, were maximally loaded with IgE. Optimised antibodies were incubated with the IgE-loaded cells and assessed for their ability to stimulate a calcium response. No signalling could be detected (figure 1).

*2.8. Selectivity and species cross reactivity of optimised antibodies in DELFIA® epitope competition assays*

The selectivity and species cross reactivity of the lead antibodies was re-evaluated using the DELFIA® epitope competition assay as previously described (see section 1.6 and Materials and Methods).

Titrations of purified IgA, IgM, IgD, and IgG (all Calbiochem) were tested in each assay to establish the specificity profile for each structurally related protein, as measured by IC50 values in the assay.

Titrations of IgE species including human IgEλ (U266 derived)and IgEκ (Calbiochem), cynomolgus IgE Cε3-Cε4 domain (in house HEK-EBNA derived), human IgE Cε3-Cε4 domain (in house HEK-EBNA derived) and cynomolgus IgE (in house HEK-EBNA derived) were tested in each assay to establish the species cross-reactivity of the antibodies. Full-length human IgEλ and IgEκ and cyno IgE produced an inhibition curve. No inhibition was observed with human or cynomolgus IgE Cε3-Cε4 domains or with any of the structurally related proteins. These data demonstrate that Antibody 33GL binds to full length human and cyno IgE, although not to the Cε3-Cε4 domain. In addition, Antibody 33GL does not bind to any of the most related human proteins to IgE. Details of the protocol are provided in the Materials and Methods section.

*2.9 Binding affinity Calculation of affinity data for optimised clones using BIAcore*

The binding affinity of purified IgG samples of a representative number of clones to human and cynomolgus IgE was determined by surface plasmon resonance using BIAcore 2000 biosensor (BIAcore AB) essentially as described by Karlsson et al 1991; Journal of Immunological Methods 145 (1-2) 229-240. In brief, purified human or cynomolgus IgE was covalently coupled to the surface of a CM5 sensor chip (BIAcore) using standard amine coupling reagents according to the manufacturer's instructions to provide a surface density of 100 RU. IgG samples prepared in HBS-EP buffer (BIAcore (AB), at a range of concentrations, between 250 nM and 15.6 nM were passed over the sensor chip surface. The surface was regenerated using 10mM Glycine, pH 1.75 between each injection of antibody. The resulting sensorgrams were evaluated using BIA evaluation 3.1 software and fitted to a bivalent analyte model, to provide relative binding data.
Example affinities for the IgG tested are shown in Table 5b and Table 6.

**Materials and Methods for Examples 1 and 2**

*Inhibition of IgE binding to FcεRI by unpurified scFv*

Selection outputs were screened in a receptor-ligand binding homogeneous FRET (Fluorescence resonance energy transfer) based assay format for inhibition of human IgE (Calbiochem 401152) labelled with Europium Chelate (Perkin Elmer 1244-302) binding to human FcεRI-Fc (in house NS0 cell produced).

Outputs during lead isolation were screened as undiluted, periplasmic extracts containing unpurified scFv, prepared in: 50mM MOPS buffer pH7.4, 0.5 mM EDTA and 0.5 M sorbitol.

15 μl of unpurified scFv sample was added to a 384 well assay plate (Perkin Elmer 6006280). This was followed by the addition of 15 μl of 11nM human FcεRI-Fc (based on a MW of 260kDa), 15 μl of 40 nM anti human Fc IgG labelled with XL665 (CIS Bio International 61HFCXLA), and then 15 μl of 0.75 nM europium labelled human IgE. Non-specific control binding was defined using 300nM human IgE (Calbiochem). All dilutions were performed in 50 mM Tris-HCl (pH 7.8) containing 250 mM sodium chloride and 0.05% Tween20 (assay buffer).

Assay plates were then incubated for 1.5 h at room temperature, prior to reading time resolved fluorescence at 615 nm and 665 nm emission wavelengths sequentially using a VICTOR2 plate reader (Perkin Elmer).

Data was normalised by VICTOR2 software to calculate counts per second (CPS). CPS values were subsequently used to calculate % specific binding as described in equation 1.

Equation 1:

$$\% \text{ specific binding} = \frac{(\text{CPS of sample} - \text{CPS of non-specific binding control})}{(\text{CPS of total binding control} - \text{non-specific binding control})} \times 100$$

*Inhibition of IgE binding to FcεRI by purified scFv*

Purified scFv from positive clones identified from screening were tested in receptor-ligand binding homogeneous FRET (Fluorescence resonance energy transfer) based assay format for inhibition of human IgE (Calbiochem 401152) labelled with Europium Chelate (Perkin Elmer 1244-302), binding to human FcεRI-Fc (in house NS0 cell produced).

A titration of scFv concentrations was used in order to establish the scFv potency as measured by $IC_{50}$ values in the assay. 15 μl of titration of purified scFv sample was added to a 384 well assay plate (Perkin Elmer 6006280). This was followed by the addition of 15 μl of 11nM human FcεRI-Fc (based on a MW of 260kDa); 15 μl of 40 nM anti human Fc IgG labelled with XL665 (CIS Bio International 61 HFCXLA), and then 15 μl of 0.75 nM europium labelled human IgE. Non-specific control binding was defined using 300nM human IgE (Calbiochem). All dilutions were performed in 50 mM Tris-HCl (pH 7.8) containing 250 mM sodium chloride and 0.05 % Tween20 (assay buffer).

Assay plates were then incubated for 1.5 h at room temperature, prior to reading time resolved fluorescence at 615 nm and 665 nm emission wavelengths sequentially using a VICTOR2 plate reader (Perkin Elmer).

Data was normalised by VICTOR2 software to calculate counts per second (CPS). CPS values were subsequently used to calculate % specific binding as described in equation 1.

Equation 1:

% specific binding =

$$\frac{\text{(CPS of sample} - \text{CPS of non-specific binding control)}}{\text{(CPS of total binding control - non-specific binding control)}} \times 100$$

$IC_{50}$ values were determined using GraphPad Prism software by curve fitting using a four-parameter logistic equation (Equation 2).

Equation 2:

$$Y=Bottom + (Top-Bottom)/(1+10^\wedge((LogEC50-X)*HillSlope))$$

X is the logarithm of concentration. Y is specific binding
Y starts at Bottom and goes to Top with a sigmoid shape.

*Inhibition of calcium signalling by purified scFv and IgG in RBL-2H3 cells stably transfected with the human FcεR1 (RBL-ER51 cells)*

The neutralisation potency of purified scFv and IgG preparations against human IgE bioactivity mediated through FcεRI was assessed using an RBL-ER51 calcium-signalling assay. Human FcεRI was cloned from human peripheral blood lymphocytes into the pcDNA3.1 vector and transfected, using a standard electroporation method, into RBL-2H3 cells (a rat basophilic cell line). Transfected cells were cloned by limited dilution and analysed for surface FcεRI expression. The resulting RBL-ER51 cells were maintained in media containing G418 (Invitrogen 10131-027) to maintain stable receptor expression.

Free IgE in the vicinity of the cells binds to the FcεRI and subsequent cross-linking of receptor-bound IgE leads to a calcium mobilisation that can be defected using a Fluorometric Imaging Plate Reader (FLIPR).

RBL-ER51 cells were seeded at $5x10^4$/100μl/well in culture media [DMEM (Invitrogen 41966)with 9% v/v FBS Non-Heat Inactivated (Invitrogen 10100-147)and 400μg/mL G418 (Invitrogen 10131-027)] into 96 well black-walled, flat-bottomed, tissue culture-treated plates (Costar) and incubated at 37°C, 5% $CO_2$ for 18-24 hours. After this time, media was aspirated, leaving cell monolayer intact, and replaced with 100μL/well of FLUO-4AM loading buffer [DMEM with 0.1% FBS, 20mM HEPES, 2.5mM probenicid and 2μg/mL FLUO-4AM (Teff Labs)] for 1-2 hours at 37°C, 5% $CO_2$. Loading buffer was aspirated and cells washed 3 times with 200μL/well of PBS. The final wash was aspirated and replaced with 70μL/well of FL1PR buffer [125mM $NaCl_2$, 5nM KCl, 1mM $MgCl_2$, 1.5mM $CaCl_2$, 30mM Hepes, 2.5mM Probenicid, 5mM glucose, 0.01% v/v FCS]. Plates were incubated at 37°C, 5% $CO_2$ for 5-45 minutes.

Test solutions of purified ScFv or IgG (in duplicate) were diluted to the desired concentration in FLIPR buffer in V-bottom plates (Greiner). An irrelevant antibody not directed at IgE was used as negative control. IgE (Calbiochem or U266-derived [Ikeyama et. al. 1986. Molecular Immunology 23 (2); p159-167]) was prepared in FLIPR buffer and mixed with appropriate test antibody to give a final IgE concentration of 3.33μg/mL in a total volume of 40μll/welL The concentration of IgE used in the assay was selected as the dose that at final assay concentration gave approximately 80% of maximal calcium response. All samples were incubated for 30 mins at room temperature, prior to transfer of 30μl of IgE / antibody mixture to the dye-loaded cells prepared above. Assay plates were incubated at 37°C for 10 minutes to allow free IgE to bind to the RBL-ER51 cells.

To measure calcium mobilisation following addition of cross-linking anti-IgE, the FLIPR (Molecular Devices) was calibrated for suitable exposure according to manufacturers instructions. Anti-IgE (Biosource AHI0501), diluted in FLIPR buffer, was added to the assay plates to a final concentration of 10μg/mL. Fluorescence of the FLUO-4AM dye was recorded at 1-second intervals for 80 measurements followed by 8-second intervals for 40 measurements. The peak response from each well was exported and data was then analysed using Graphpad Prism software.

*Measurement of anti-IgE cross-linking in RBL-ER51 cells*

To measure ability of purified IgGs to cross-link FcεRI-bound IgE, RBL-ER51 cells were prepared and dye-loaded as described in the inhibition assay. Cells were incubated for 10 minutes in 100μL of 1 μg/mL human IgE (Calbiochem or U266-derived [Ikeyama et. al. 1986. Molecular Immunology 23 (2); p159-167]), diluted in FLIPR buffer, to allow IgE to bind to FcεRI on the cell surface. The concentration of IgE used in the assay was selected as the dose that gave approximately 80% of maximal calcium response. To measure calcium mobilisation following addition of cross-linking anti-IgE, the FLIPR (Molecular Devices) was calibrated for suitable exposure according to manufacturers instructions. 30μL of test antibodies, diluted to appropriate concentrations in FLIPR buffer were added to the IgE loaded assay plates. Anti-IgE (Biosource AHI0501) was used as a positive control. Fluorescence of the FLUO-4AM dye (Teff Labs) was recorded at 1-second intervals for 80 measurements followed by 8-second intervals for 40 measurements. The peak response from each well was exported and data was then analysed using Graphpad Prism software

*Selectivity and species cross reactivity of antibodies in DELFIA® epitope competition assays*

Purified IgG were adsorbed onto 96-well Maxisorp microtitre plates (Nunc) in PBS at a concentration which gave a significant signal when biotinylated human IgE was added at approximately its estimated KD for that particular IgG. Excess IgG was washed away with PBS-Tween (0.1% v/v) and the wells were blocked with PBS-Marvel (3% w/v) for 1 h. A dilution series of each of the following competitors was prepared in PBS, starting at a concentration of approximately 1000-fold the KD value of the interaction between biotinylated human IgE and the respective IgG; human IgE lambda (BIODESIGN International or U266 derived [Ikeyama et. al. 1986. Molecular Immunology 23 (2); p159-167]), human IgE kappa (Calbiochem), cynomolgus IgE (in house HEK-EBNA derived), human IgE Cε3-Cε4 domain (in house HEK-EBNA derived), cynomolgus IgE Cε3-Cε4 domain (in house HEK-EBNA derived), human IgA, IgM, IgD, and IgD (all Calbiochem). To this series, an equal volume of biotinylated human IgE at a concentration of approximately the KD was added (resulting in a series starting at a ratio of competitor antigen:biotinylated human IgE of approximately 1000: 1). These mixtures were then transferred onto the blocked IgG and allowed to equilibrate for 1 h. Unbound antigen was removed by washing with PBS-Tween (0.1% v/v), while the remaining biotinylated human IgE was detected by streptavidin-Europium3+ conjugate (DELFIA® detection, PerkinElmer). Time-resolved fluorescence was measured at 620nm on an EnVision plate reader (PerkinElmer). Fluorescence data were analysed using either Graphpad Prism or Microsoft Excel software.

*Identification of improved clones using an antibody-ligand biochemical assay*

Selection outputs were screened in epitope competition HTRF® (Homogeneous Time-Resolved Fluorescence) assay format for inhibition of cryptate labelled human IgE (U266-derived [Ikeyama et. al. 1986. Molecular Immunology 23 (2); p159-167]) labelled with europium cryptate (CIS bio International 62EUSPEA), binding to anti human IgE antibody (Antibody 1).

During lead optimisation, selection outputs were screened as undiluted or diluted, periplasmic extracts, containing unpurified scFv, prepared in; 50mM MOPS buffer pH7.4, 0.5 mM EDTA and 0.5 M sorbitol.

4 nM anti human IgE antibody was pre-mixed with 20 nM anti human Fc IgG labelled with XL665 (CIS Bio International 61HFCXLA). 10 μl of unpurified scFv sample was added to a 384 well low volume assay plate (Costar 3676). This was followed by the addition of 5 μl of the anti human IgE antibody anti Fc-XL665 mix, and then 5 μl of a 1/245 dilution of cryptate labelled human IgE (approximately 2.3nM cryptate labelled human IgE). Non-specific control binding was defined using 300nM human IgE (U266-derived [Ikeyama et. al. 1986. Molecular Immunology 23 (2); p159-167]). All dilutions were performed in phosphate buffered saline (PBS) containing 0.4 M potassium fluoride and 0.1% BSA (assay buffer).

Assay plates were then centrifuged at 1000rpm at room temperature for 1 min, and incubated for 3 h at room temperature, prior to reading time resolved fluorescence at 620 nm and 665 nm emission wavelengths using an En Vision plate reader (Perkin Elmer).

Data was analysed by calculating % Delta F values for each sample. Delta F was determined according to equation 1.

Equation 1:

$$\% \text{ Delta F} = \frac{(\text{sample 665nm/620nm ratio value}) - (\text{non-specific control 665nm/620nm ratio value})}{(\text{non-specific control 665nm/620nm ratio value})} \times 100$$

% Delta F values were subsequently used to calculate % specific binding as described in equation 2.

Equation 2:

$$\% \text{ specific binding} = \frac{\% \text{ Delta F of sample}}{\% \text{ Delta F of total binding control}} \times 100$$

*Inhibition of IgE binding to FcεRI by improved scFv (purified)*

Purified scFv were tested in a receptor-ligand binding HTRF® (Homogeneous Time-Resolved Fluorescence) assay format for inhibition of either human IgE (U266-derived [Ikeyama et. al. 1986. Molecular Immunology 23 (2); p159-167])or cyno IgE (recombinant, see materials and methods) labelled with europium cryptate (CIS bio International 62EUSPEA), binding to human FcεR1-Fc (in house NS0 cell produced).

A titration of scFv concentrations was used in order to establish the scFv potency as measured by $IC_{50}$ values in the assay. 1.9nM human FcεRI-Fc (based on MW of 260kDa) was pre-mixed with 20 nM anti human Fc IgG labelled with XL665 (CIS Bio International 61HFCXLA). 10 μl of titration of purified scFv sample was added to a 384 well low volume assay plate (Costar 3676). This was followed by the addition of 5 μl of the FcεR1-Fc anti Fc-XL665 mix, and then 5 μl of a 1/197 dilution of cryptate labelled human or cyno IgE (approximately 2.9nM cryptate labelled human or cyno IgE). Non-specific control binding was defined using 300nM of human or cynomolgus IgE (in house derived). All dilutions were performed in phosphate buffered saline (PBS) containing 0.4 M potassium fluoride and 0.1% BSA (assay buffer).

Assay plates were then centrifuged at 1000rpm at room temperature for 1 min, and incubated for 3 h at room temperature, prior to reading time resolved fluorescence at 620 nm and 665 nm emission wavelengths using an EnVision plate reader (Perkin Elmer).

Data was analysed by calculating % Delta F values for each sample. Delta F was determined according to equation 1.

Equation 1:

$$\% \text{ Delta F} = \frac{(\text{sample 665nm/620nm ratio value}) - (\text{non-specific control 665nm/620nm ratio value})}{(\text{non-specific control 665nm/620nm ratio value})} \times 100$$

% Delta F values were subsequently used to calculate % specific binding as described in equation 2.

Equation 2:

$$\%\ \text{specific binding} = \frac{\%\ \text{Delta F of sample}}{\%\ \text{Delta F of total binding control}} \times 100$$

$IC_{50}$ values were determined using GraphPad Prism software by curve fitting using a four-parameter logistic equation (Equation 3).

Equation 3:

Y=Bottom + (Top-Bottom)/(1+10^((LogEC50-X)*HillSlope))

X is the logarithm of concentration. Y is specific binding
Y starts at Bottom and goes to Top with a sigmoid shape.

*Identification of improved clones in the RBL-ER51 calcium signalling assay*

The neutralisation potency of purified IgG preparations from improved antibodies was assessed in a modified version of the RBL-ER51 calcium-signalling assay described for lead isolation.

RBL-ER51 cells were seeded at $5x10^4$/100μl/well in culture media [DMEM (Invitrogen 41966)with 9% v/v FBS Non-Heat Inactivated (Invitrogen 10100-147)and 400μg/mL G418 (Invitrogen 10131-027)] into 96 well black-walled, flat-bottomed, tissue culture-treated plates (Costar) and incubated at 37°C, 5% $CO_2$ for 18-24 hours. After this time, media was aspirated and replaced with 50μL/well dilutions of test antibodies (66.7nM to 13.3pM) in assay media [DMEM (Invitrogen 41966)with 9% v/v FBS Non-Heat Inactivated (Invitrogen 10100-147), 400μg/mL G418 (Invitrogen 10131-027) and 1.6% Penicillin / Streptomycin (Invitrogen 15140-122)]followed by addition of IgE [human (U266-derived [Ikeyama et. al. 1986. Molecular Immunology 23 (2); p159-167]) or cynomolgus (recombinant, see materials and methods)] diluted in assay media to give a final IgE concentration of 25ng/ml and 100ng/ml respectively. Assay plates were incubated for 4 hours at 37°C, 5% $CO_2$.

After this time, antibody/IgE mixture was aspirated, leaving cell monolayer intact, and replaced with 100μL/well of FLUO-4AM loading buffer [DMEM with 0.1% FBS, 20mM HEPES, 2.5mM probenicid and 2μg/mL FLUO-4AM (Invitrogen)]for 1-2 hours at 37°C, 5% $CO_2$. Loading buffer was aspirated and cells washed 3 times with 200uL/well of PBS. The final wash was aspirated and replaced with 100μL/well of FLIPR buffer [125mM $NaCl_2$, 5nM KCl, 1mM $MgCl_2$, 1.5mM $CaCl_2$, 30mM Hepes, 2.5mM Probenicid, 5mM glucose, 0.01% v/v FCS]. Plates were incubated at 37°C, 5% $CO_2$ for 5-45 minutes.

To measure calcium mobilisation following addition of cross-linking anti-IgE, the FLIPR (Molecular Devices) was calibrated for suitable exposure according to manufacturers instructions. Anti-IgE (Biosource AHI0501), diluted in FLIPR buffer, was added to the assay plates to a final concentration of 2.3μg/mL (to cross-link human IgE) or 20μg/mL (to cross-link cynomolgus IgE). Fluorescence of the FLUO-4AM dye was recorded at 1-second intervals for 80 measurements followed by 3-second intervals for 40 measurements. The peak response from each well was exported and data was then analysed using Graphpad Prism software.

*Measurement of cross-linking of FcεRI-bound IgE by optimised antibodies*

To measure ability of purified IgGs to cross-link FcεRI-bound IgE, RBL-ER51 cells were prepared as described in the inhibition assay for assessment of improved antibodies. RBL-ER51 cells were seeded at $5x10^4$/100μl/well in culture media [DMEM (Invitrogen 41966) with 9% v/v FBS Non-Heat Inactivated (Invitrogen 10100-147)and 400μg/mL G418 (Invitrogen 10131-027)] into 96 well black-walled, flat-bottomed, tissue culture-treated plates (Costar) and incubated at 37°C, 5% $CO_2$ for 18-24 hours. After this time, media was aspirated and replaced with 100 μL/well of human IgE (U266-derived [Ikeyama et. al. 1986. Molecular Immunology 23 (2); p159-167]) diluted to 1μg/mL in assay media [DMEM (Invitrogen 41966)with 9% v/v FBS Non-Heat Inactivated (Invitrogen 10100-147), 400μg/mL G418 (Invitrogen 10131-027) and 1.6% Penicillin / Streptomycin (Invitrogen 15140-122)]. The IgE concentration was chosen to give maximal loading of the RBL-ER51 cells. Assay plates were incubated for 4 hours at 37°C, 5% $CO_2$.

After this time, the IgE solution was aspirated, leaving cell monolayer intact, and replaced with 100μL/well of FLUO-4AM loading buffer [DMEM with 0.1% FBS, 20mM HEPES, 2.5mM probenicid and 2μg/mL FLUO-4AM (Invitrogen)] for 1-2 hours at 37°C, 5% $CO_2$. Loading buffer was aspirated and cells washed 3 times with 200μL/well of PBS. The final wash was aspirated and replaced with 100μL/well of FLIPR buffer [125mM $NaCl_2$, 5nM KCl, 1mM $MgCl_2$, 1.5mM $CaCl_2$, 30mM Hepes, 2.5mM Probenicid, 5mM glucose, 0.01% v/v FCS]. Plates were incubated at 37°C, 5% $CO_2$ for 5-45 minutes.

To measure calcium mobilisation following addition of cross-linking anti-IgE (1.53μM to 2.33nM), the FLIPR (Molecular Devices) was calibrated for suitable exposure according to manufacturers instructions. 30μL of test antibodies, diluted to appropriate concentrations in FLIPR buffer, were added to the assay plates. Anti-IgE (Biosource AHI0501) was used as a positive control. Fluorescence of the FLUO-4AM dye (Invitrogen) was recorded at 1-second intervals for 80 measurements followed by 3-second intervals for 40 measurements. The peak response from each well was exported and data was then analysed using Graphpad Prism software.

*Inhibition of IgE binding to CD23 on IM9 cells by purified IgG*

Antibodies were evaluated for inhibition of the IgE / CD23 interaction using the IM9 cell binding assay. IM9 cells (a human B cell line) were maintained in culture media [RPMI 1640 glutamax (Invitrogen 61870-010); 9% v/v heat-inactivated FBS (Invitrogen 10100-147)] using standard tissue culture procedures.
To test the optimised IgG the IM9 cells were pre-treated with 25ng/ml human IL-4 (recombinant, Peprotech 200-04) for 3 days at 37°C/5%$CO_2$ in order to up-regulate CD23 expression.

IM9 cells were harvested and resuspended in Flow buffer [PBS with 1% Goat serum (Sigma) and 0.1% BSA fraction V (Sigma) at $1x10^6$ cells/mL. Fc receptor blocking was performed by addition of Fc fragments (TEBU-bio) to a final concentration of 5μg/mL. This cell suspension was plated at 100μL/well in U-bottomed polypropylene plates (Greiner) and incubated on ice for 30 minutes.

Antibody dilutions (667nM to 1nM) were prepared in U-bottomed polypropylene plates (Greiner) and mixed with IgE (U266-derived [Ikeyama et. al. 1986. Molecular Immunology 23 (2); p159-167]) to a final IgE concentration of 10μg/mL for 30 minutes at room temperature. Cell plates were spun at 2000rpm for 2 minutes and supernatant was aspirated, leaving the cell pellet intact. Cells were resuspended in 100μL/well antibody/IgE mix and incubated on ice for 1 hour. Cell plates were centrifuged at 2000rpm for 2 minutes and antibody/IgE supernatants were aspirated. Cells were washed by resuspending in 200uL/well of Flow buffer and centrifuging as above.

IgE bound to the cell surface was detected with anti-IgE-phycoerythin (Caltag) diluted 1/30, v/v, 100μL/well. Assay plates were incubated on ice for 20 minutes in the dark before centrifuging at 2000rpm for 2 minutes and washing with 2 x 200uL of Flow buffer as described above. Cells were resuspended in 100uL Cell Fix (BD biosciences) and analysed using a FACSCalibur (BD Biosciences) to detect FL2 staining.

Data was analysed using CellQuest Software (BD biosciences). FL2 Geomean fluorescence was exported and data was then analysed using Microsoft Excel and Graphpad Prism software.

*Generation Human IgE Ce3-4-C-terminally tagged with FLAG HislO*

The fragment of human IgE Ce3-4 was as described previously in Wurzburg et. al. (2000) Structure of the Human IgE-Fc C3-C4 Reveals Conformational Flexibility in the Antibody Effector Domains. A cDNA fragment that encompassed nucleotides 2135 - 2868 (GenBank accession number J00222) was amplified using RT-PCR from total RNA of IL13 stimulated human PBMC. This PCR product was cloned into pCR2.1 TA (Invitrogen).

To allow secretion of the expressed protein and generate a sequence that incorporated an inframe C-terminal FLAG epitope and polyhistidine tag (His 10), the IgE Ce3-4 fragment was PCR amplified with primers that incorporated a 5' BssHII site, and 3' FLAG epitope, polyhistidine tag (His10) and Xbal site and subsequent insertion into pEU8.2 vector. The modified pEU8.2 vector contains an EF-1 promoter, the genomic sequence for murine IgGI leader peptide, oriP origin of replication to allow episomal plasmid replication upon transfection into cell lines expressing the EBNA-1 gene product (such as HEK293-EBNA cells).

Protein was purified from conditioned media using IMAC chromatography followed by Size Exclusion chromatography.

*Generation Cynomolgus IgE Ce3-4 C-terminally tagged with FLAG His10*

The cynomolgus IgE constant region was determined by direct sequencing of PCR products amplified from genomic DNA using primers that encompass nucleotides 1174 - 2989 of the human IgHE locus (GenBank Accession J00222).
The exons were identified by homology with the human sequence, and thus it was possible to predict the cDNA sequence for the cynomolgus IgE heavy chain constant region.

A cDNA encoding the sequence for murine IgG1 leader peptide, cynomolgus, Ce3-4, and C-terminal FLAG epitope and polyhistidine tag was synthesised (DNA2.0) and cloned into pDONR221 (Invitrogen). Then using LR Gateway® reaction (Invitrogen) the gene of interest was transferred to the expression vector pDEST12.2 (Invitrogen) modified by the insertion of the oriP origin of replication from the pCEP4 vector (Invitrogen) to allow episomal plasmid replication upon transfection into cell lines expressing the EBNA-1 gene product (such as HEK293-EBNA cells).

Protein was purified from conditioned media using IMAC chromatography followed by Size Exclusion chromatography.

*Generation chimaeric D12 variable region and cynomolgus IgE constant region*

A cDNA encoding the variable heavy chain region that encoded the Human anti-eostradiol scFv (D12_VH) and one of either two different haplotypes cynomolgus IgHE gene (cyIGHE TQ and cyIGHE ME) were synthesised (DNA2.0) and cloned into pDONR221 (Invitrogen). Also a cDNA representing the variable light chain Human anti-eostradiol scFv (D12_VL) and one of either two cynomolgus lambda constant region genes (cyIGLC4 and cyIGLC7) were synthesised (DNA2.0) and cloned into pDONR221 (Invitrogen).

Then using LR Gateway® reaction (Invitrogen) the gene of interest was transferred to the expression vector pDEST12.2 (Invitrogen) modified by the insertion of the oriP origin of replication from the pCEP4 vector (Invitrogen) to allow episomal plasmid replication upon transfection into cell lines expressing the EBNA-1 gene product (such as HEK293-EBNA cells).

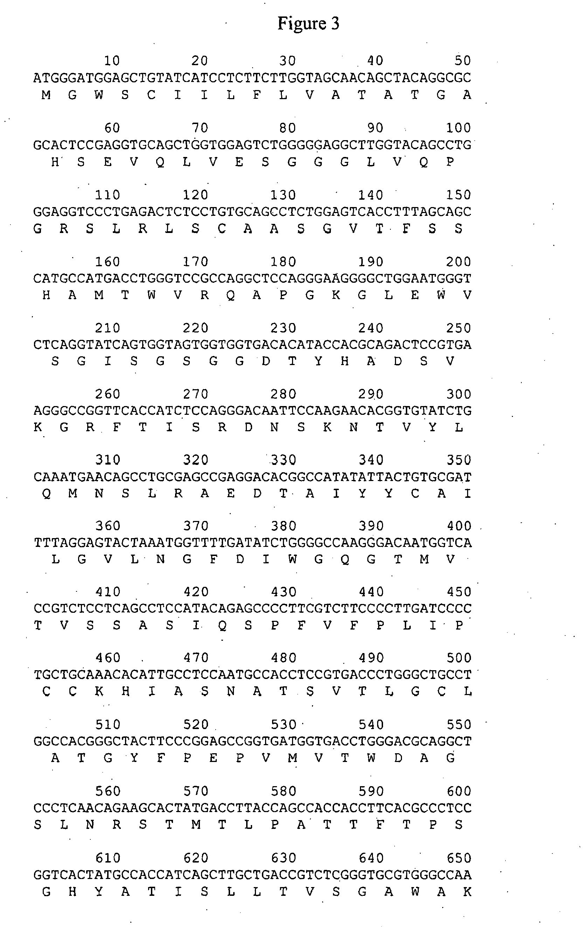

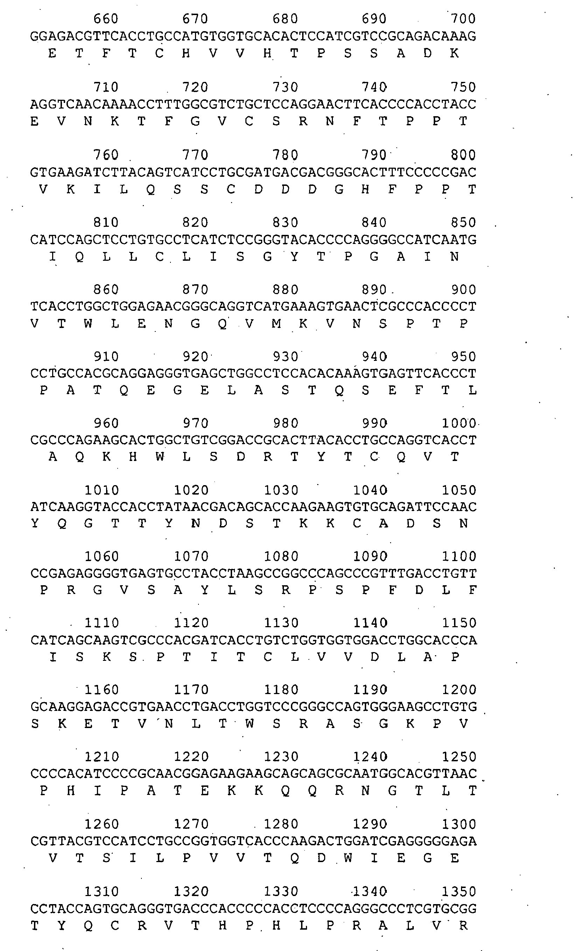

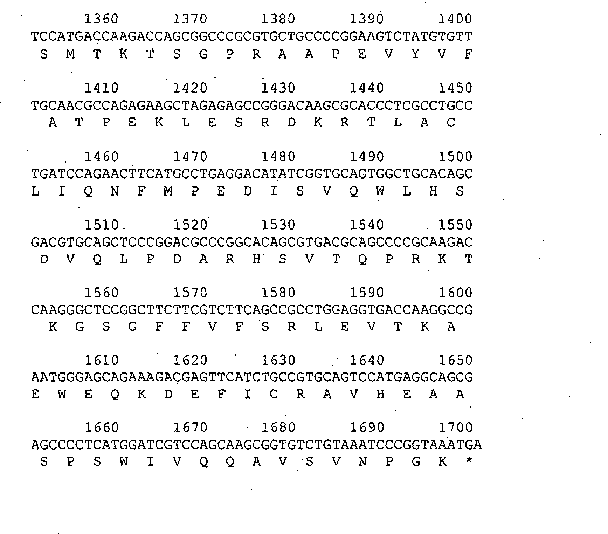

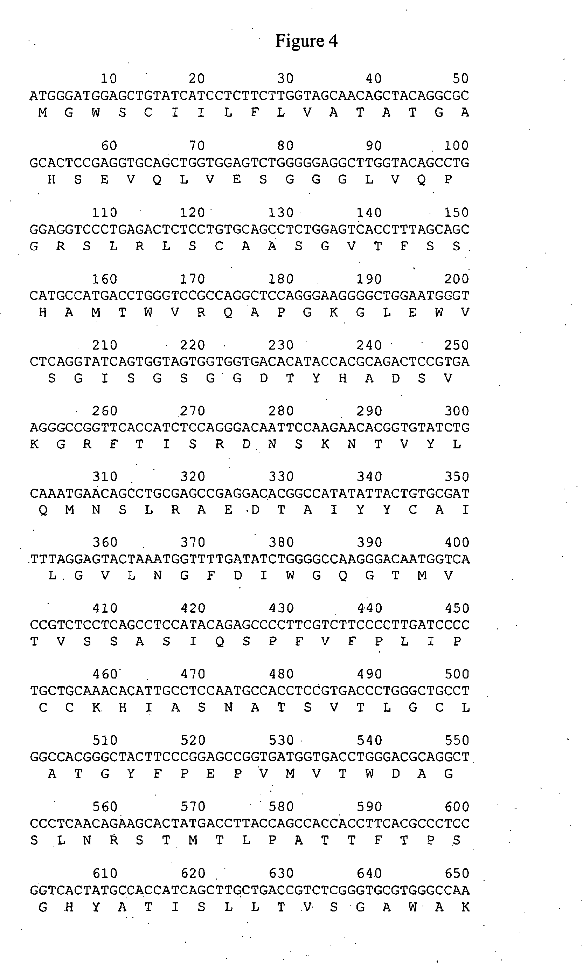

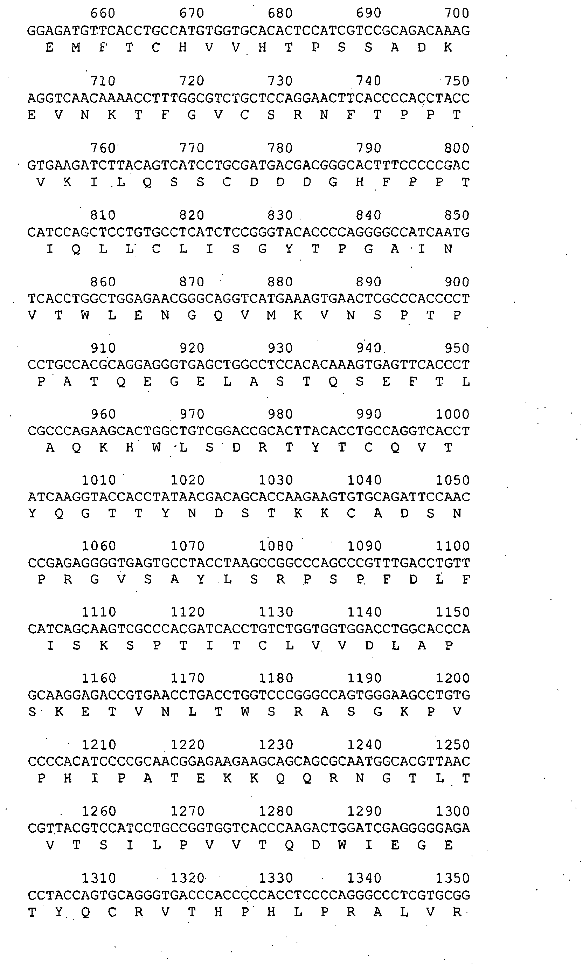

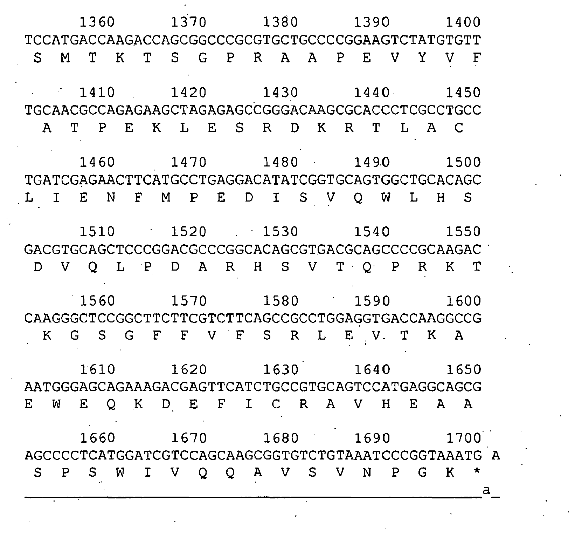

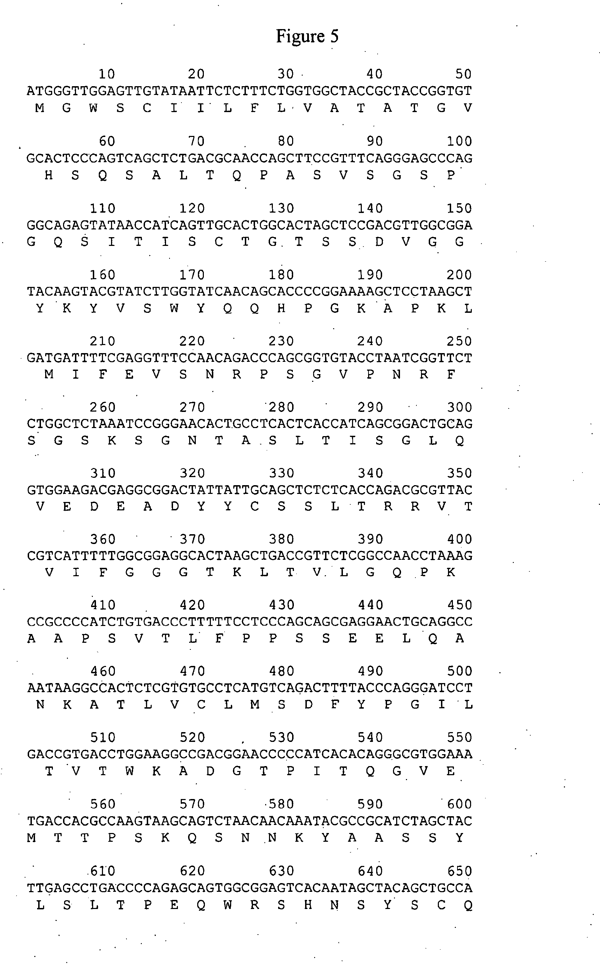

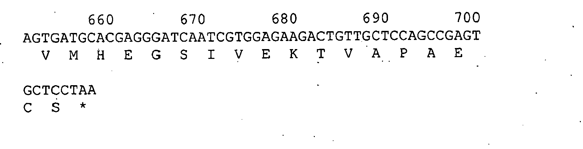

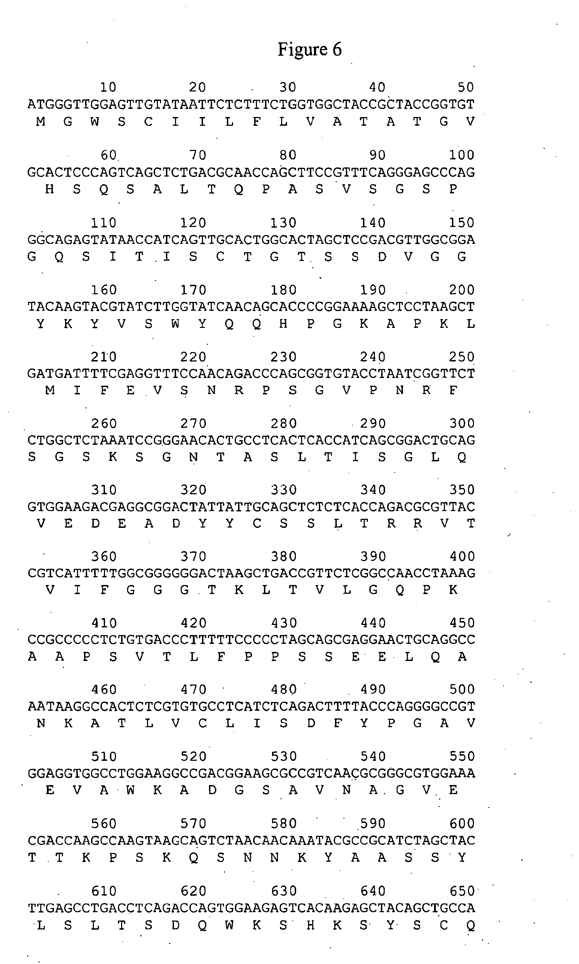

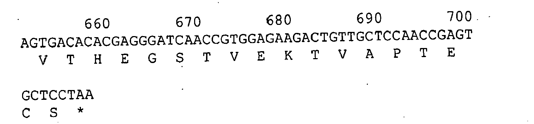

Recombinant chimaeric IgE protein representing the variable heavy chain region of the Human anti-eostradiol scFv fused to cynomolgus IgE Ce1-4 (Figures 3 and 4), and variable light chain region of the Human anti-eostradiol scFv fused to cynomolgus Lambda constant region (Figures 5 and 6), expressed from HEK293 EBNA cells was purified using the method as described in Ikeyam et. al. (1986) Mol Immunol 23 p159-67.

*human Fc□RI-Fc (in house NS0 cell produced)*

The FceRI encompassing the nucleotides 67- 711 (GenBank Accession number NM_002001) was cloned up stream of the genomic region of the human IgG 1 Fc as from pEU 1.2 first described in Persic et al. (1997) Gene 187; 9-18. This was cloned into pcDNA3.1 EcoRI - XbaI. Expression of the recombinant fusion protein FceRI_Fc was achieved by stable transfection of NS0 cells with the pcDNA3. FceRI_Fc construct. Stable expression was established by selection with G418, isolation of clones via limiting dilution and identification of the those clones with the high expression level. The FceRI_Fc fusion protein was then purified from the conditioned medium using Protein A affinity chromatography, followed by preparative Size Exclusion Chromatography.

Example 3 Measurement of complex formation between IgE and purified IgG.

Characterisation of the immune complexes formed between purified human IgE and purified anti-IgE IgG (antibody 33) was performed by high-performance size exclusion liquid chromatography. In addition, on line multi-angle light scattering (MALS) was used to estimate complex size. Complexes were formed by incubating IgE and IgG together in a 1:1 molar ratio in Dulbecco's PBS at 18 deg C for one hour. The concentration of each protein in the sample was 2.5 μM. The sample was analysed on two Bio-Sep-SEC-S 4000 columns (300 x 7.8mm) arranged in tandem. The columns were equilibrated and sample analysed in Dulbecco's PBS at a flow rate of 1 ml/min on an Agilent HP1100 HPLC system. Peaks were detected at 220 and 280nm using a diode array detector and the eluate was also directed through Wyatt Technologies DAWN EOS (MALS) and Optilab rEX(refractive index) detectors.

Chromatography of the 1:1 molar ratio sample gave three peaks (detected by UV absorbance) which were not completely resolved and corresponded to retention times of 13.38min, 14.24min and 15.64 min. These retention times indicate the formation of non-covalent complexes of IgE with IgG. MALS analysis gave molecular masses of 1239kDa (13.88min peak), 914kDa(14.24 min peak) and 576 kDa (15.64min peak) which are all higher than the masses of the IgG and IgE proteins alone. No evidence of protein eluting in the void volume was observed indicating that higher molecular mass aggregates (>2 million Da) were not formed.

**Example 4: Human B cells - inhibition of intracellular IgE**

Peripheral Blood Mononuclear Cells (PBMC) were isolated from human heparinised whole blood by centrifugation on a Ficoll-Paque gradient (Pharmacia). B cells were subsequently isolated from the PBMC population using positive anti-CD19 selection with magnetic beads (Miltenyi). Both the positive and the negative B cell fractions were collected as the cells were passed over the magnetic column. The cells from the negative B cell fraction containing all PBMC cells except B cells, were treated with Mitomycin C to prevent proliferation. The cells were incubated with 50 μg/mL Mitomycin C for 30 min, then washed with tissue culture media (RPMI 1640 with Glutamax (Gibco) / 10% FCS (Gibco) / 50 U/mL Penicillin /50μg/mL Streptomycin (Gibco)) and further incubated with PBS for 70 min before a final wash to ensure all Mitomycin C was removed. To induce the differentiation of the B cells, 4 x $10^4$ B cells and 9.2x $10^5$cells from the B cell negative fraction were plated in a 96-well plate in tissue culture media supplemented with 3.5μM beta-mercaptoethanol (Sigma) and 20 μg/mL transferrin (Chemicon), and pre-incubated with arlti-IgE Mab's or a control antibody at 0.001-100nM for 30 min before addition of human interleukin-4 (IL-4) at 0-100ng/mL. The cells were subsequently incubated in a humidified $CO_2$ incubator for 12 days. At day 12, the plates were given a brief spin, supernatants collected and the cells stained for intracellular IgE using the following protocol.

The cells were first incubated with PBS with 1% human serum for 10 minutes to block Fc Receptor binding. Cells were then fixed and permeabilised on ice using Cytofix/Cytoperm kit from Becton Dickinson. The cells were then washed before addition of a polyclonal rabbit anti-human IgE-FITC antibody (DAKO) at 1:6 final dilution and a monoclonal mouse anti-human CD 19 - RPE/Cy5 antibody (DAKO) at 1:10 final dilution. It is important that the cells are thoroughly washed to avoid interference from residual anti-IgE MAb's (monoclonal antibodies). After 30 min incubation the cells were washed and samples were analysed on a FACS Calibur using a HTS 96-well plate loader device. The percentage of cells in the CD19+ population that co-express IgE were then recorded, and the expression of intracellular IgE is presented as % inhibition of maximum IgE expression in cells not treated with blocking anti-IgE.

To validate no interference of the anti-IgE MAb with the intracellular staining, supernatants were analysed in parallel for IgE production using an assay that has been validated to show no significant interference by the anti-IgE clones used, i.e. the Pharmacia diagnostics (currently Phadia) ImmunoCap assay.

**Example 5. Assessment of the General Safety and Capacity of Anti-IgE mAbs to Induce Decreases in Platelet Numbers in Juvenile Cynomolgus Monkeys**

*Introduction*

An investigative (non-GLP compliant) study was performed in juvenile cynomolgus monkeys to assess the general safety and relative abilities of anti-IgE mAbs Antibody 33 and two other anti-IgE antibodies E85-50 $IgG_1$ and E85-50 $IgG_2$ to cause decreases in numbers of blood platelets.

The objectives of the study were 1) to determine the general safety and relative abilities of the three candidate anti-IgE mAbs to induce a reduction in platelet counts/ thrombocyopaenia (TCP) and associated effects in juvenile cynomolgus monkeys 2) to determine preliminary pharmacokinetic parameters for the mAbs in monkeys 3) to assess the capacity of the three candidate mAbs to cause a reduction in free IgE and determine the (PK/PD) relationship between mAb concentraton and free IgE levels.

*Materials And Methods for Example 5*

Eighteen purpose-bred cynomolgus monkeys (*Macaca fascicularis*) were obtained from Bioculture, Mauritius. The animals were between 63 to 67 weeks old at the start of dosing. Monkeys were pre-selected (from a larger pool of 100 animals) to have high IgE levels (U/ml) which were normalised across 3 groups each containing 3 male and 3 female monkeys and receiving either E85-50 $IgG_1$ (Group 1), E85-50 $IgG_2$ (Group 2) or Antibody 33 (Group 3). Each of the 3 mAbs were formulated in 50mM sodium acetate, 100mM NaCl, pH5.5 and adminstered to animals in a dose volume of 2 mL/kg by slow intravenous injection (using a motorised syringe/infusion pump) at a rate of 1 mL/min. The animals were dosed once weekly (for 5 weeks/5 doses) with rising dose levels of 1mg/kg, 30mg/kg and 100mg/kg (3 times) on Days 1, 8, 16, 22 and 29 (Table 7). Additional doses of E85-50 $IgG_1$ and E85-50 $IgG_2$ were administered to Groups 1 and 2 respectively on Day 37. The 2 highest dose levels were predicted to achieve serum concentrations that have previously shown to result in TCP with Xolair in juvenile cynomolgus monkeys. The low dose was expected to allow a determination of the ability of the mAbs to effect a reduction in free IgE levels.

**Table 7 Summary Study Design**

| Group | Description | Dose level (mg/kg/day) on Day: | | | | | | Number of Animals | |
|---|---|---|---|---|---|---|---|---|---|
| | | 1 | 8 | 16 | 22 | 29 | 37 | Male | Female |
| **1** | E85-50 $IgG_1$ | 1 | 30 | 100 | 100 | 100 | 100 | 3 | 3 |
| 2 | E85-50 $IgG_2$ | 1 | 30 | 100 | 100 | 100 | 100 | 3 | 3 |

(continued)

| Group | Description | Dose level (mg/kg/day) on Day: | | | | | | Number of Animals | |
|---|---|---|---|---|---|---|---|---|---|
| | | 1 | 8 | 16 | 22 | 29 | 37 | Male | Female |
| 3 | Antibody 33 | 1 | 30 | 100 | 100 | 100 | - | 3 | 3 |

Animals were observed for 8 weeks post the Day 28 dose and examined for recovery from any toxicological effects.

All animals were observed daily for signs of ill health or overt toxicity and body weights and food consumption recorded. In addition, each animal was given a detailed physical examination daily during dosing periods and at least once weekly during non-dosing periods. All animals were also observed prior to each dose and at 0.5, 2, 6, 24, 48 and 168 hours post dose.

Blood samples for analysis of standard haematology parameters (including platelet counts; collected in EDTA) and coagulation parameters (collected in trisodium citrate) were taken from the femoral vein/artery twice pre-treatment (Weeks -2 and-1). Further samples for platelet counts and standard haematology were collected at 24 hours and 144 hours after each dosing occasion (Days 2, 7, 9, 14, 17, 22, 23, 26, 30 and 35; samples from Groups 1 and 2 only on Days 38 and 43) and every 2 weeks during the 8-week recovery period (Days 43, 57, 71 and 82). Samples for coagulation were collected at 144 hours after each dosing occasion (Days 7, 14, 22, 26 and 35) and at the end of the recovery period (Day 82). Samples for coagulation were also collected on Day 57. Blood samples for complement activation (C3a, C3b and BB fragments) were taken once pre-treatment (Week-1) and approximately 24 hours following completion of the treatment period (Day 30)

Serum samples for TK analysis were collected from all groups on day Day 1 at pre-dose, 0.5, 6, 12, 24, 48, 144 hours post-dose, on Days 8, 16, 22 and 29 at 0.5, 24 and 144 hours post-dose, on Day 29 (Groups 3 & 4 only) at 336, 672, 1008, 1272 hours post-dose, on day 37(Groups 1 & 2 only) at 0.5, 24, 144, 480, 816, 1080 hours post-dose. Samples were analysed for mAb using a generic sandwich immunoassay (using biotinylated human IgE for mAb and Alexa-647 labelled murine anti-human IgG detection reagent) and the Gyrolab Bioaffy platform (incorporating streptavidin bead columns). Further serum samples for IgE analysis were collected on Day 1 at pre-dose, 0.5, 6, 12, 24, 48, 144 hours post-dose, on Days 8, 16, 22 and 29 at 0.5, 144 hours post-dose and at the end of the study (Day 82) at 1272 hours (Groups 3 & 4) or 1080 hours (Groups 1 & 2) post-final dose. Samples were analysed for free IgE by immunoassay using the ImmunoCap system (Phadia AB, Uppsala,Sweden) with human IgG-FcεRIa for free IgE capture and Rabbit anti-human IgE (PCS-conjugate) for detection.

On termination of the animals on Day 85, a full macroscopic examination was performed under the general supervision of a pathologist and all lesions were recorded. Absolute organs weights and organ:body weight raions were determined. Tissues from a range of organs were collected and stored frozen but no microscopic examination was performed (except for macroscopic abnormalities or an unscheduled death, see below).

*Results for Example 5*

*General Safety Observations*

All 3 mAbs were generally well-tolerated with no clinical signs of ill-health throughout the study with the exception of a single animal receiving E85-Ig$G_1$ that was sent to necropsy ahead of schedule due to deteriorating clinical condition and reduced bodyweight. Since this animal deteriorated well into the recovery period and there were no findings noted during the pathology or haematology review of this animal (and any others), the observed effects are not believed to be mAb-related. Incidences of soft or liquid faeces were noted across all groups, however since these findings were not dose-related, were not seen in all animals or at all timepoints within the same animal and were seen as frequently during the dosing and recovery periods, they are unlikely to be mAb-related. Mean body weights and mean body weight gains showed some individual variation in animals within each group throughout the study. However all animals gained weight as expected over the treatment period. (with the exception of the 1 animal discussed above) and there was no clear differences between the groups. No clear treatment-related effects on absolute or relative organ weights were noted in any group. In gross pathology and microscopic pathology examinations, no findings were noted in the limited range of tissues examined that would suggest an effect of mAb treatment.

*Toxicokinetics and IgE levels*

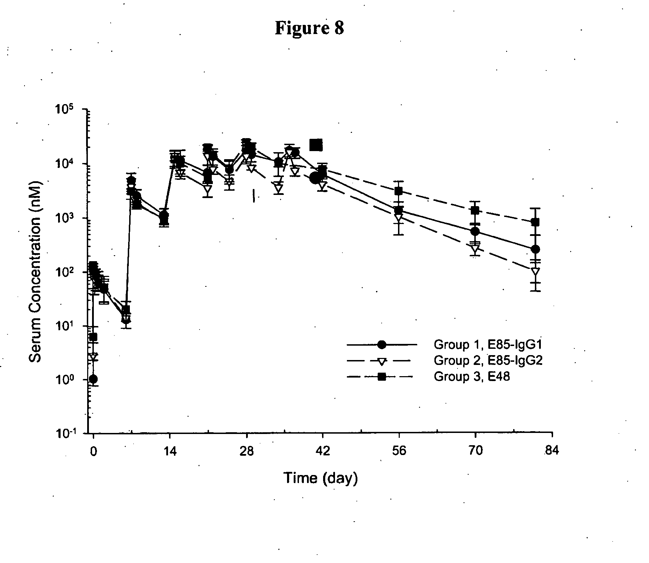

No gender difference in TK was observed in this study: In general the exposure was similar for these 3 mAbs, and appeared linear with dose in the 1-100 mg/kg dose range. The mean TK profiles of E85-50 Ig$G_1$, E85-50 Ig$G_2$ and Antibody 33 are shown in Figure 8. No apparent IgE-sink effect on TK was observed, even at the lowest dose level. The TK of these 3 antibodies appeared typical for an human IgG in cynomolgus monkeys.

The mean maximum observed concentration (Cmax) following the last 100 mg/kg dose was 18700, 15900 and 24000 nM for E85-50 $IgG_1$, E85-50 $IgG_2$ and Antibody 33, respectively. The mean terminal TK half-life following the last 100 mg/kg dose was approximately 10-13 days. There was no evidence of reduced TK exposure due to the potential development of primate anti-human antibodies in these animals.

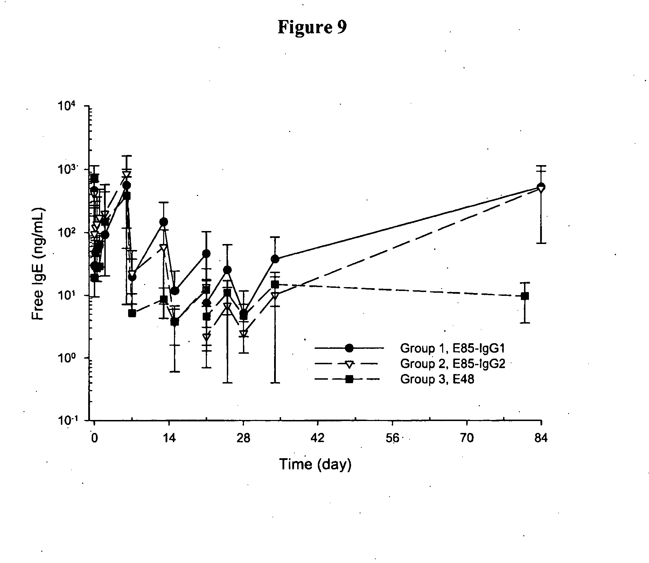

The mean free IgE profiles following weekly dosing of E85-50 $IgG_1$, E85-50 $IgG_2$ and Antibody 33 at various dose levels in cynomolgus monkeys are shown in Figure 9. The average baseline IgE before the animals received the first dose was 514, 414 and 690 ng/mL for E85-50 $IgG_1$, E85-50 $IgG_2$ and Antibody 33 groups, respectively. On Day 1, 1 mg/kg dose induced a 75-80% reduction in free IgE at 1 hour after the dose. Due to the low exposure after the 1 mg/kg dose, free IgE returned to baseline level within 1 week. Higher doses resulted in consistent suppression of free IgE during the treatment period. Free IgE returned to baseline for the 2 E85-50 groups at the end of the study, while the free IgE in the Antibody 33 group remained suppressed.

*Effects on platelets*

None of the 3 candidate mAbs (E85-50 $IgG_1$, E85-50 $IgG_2$ nor Antibody 33) induced a significant decrease in platelet counts from mean baseline levels at any timepoint in any animal with the exception of a single animal receiving E85-$IgG_1$ that had a reduction in platelets (34.9% decrease from baseline) at a single timepoint on day 29 (24 hours following the third 100mg/kg dose on day 28). A $4^{th}$ dose of E85-50 $IgG_1$ and E85-50 $IgG_2$ on day 37 did not induce any further platelet reduction in this or any animal within these 2 groups.

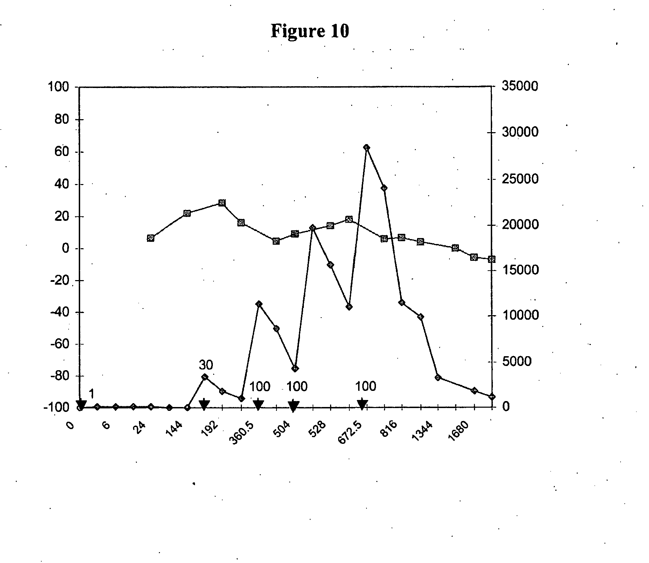

Figure 10 shows a plot of platelet numbers ($x10^9$/L) expressed as a percentage change from the mean of the 2 pre-dose values vesus plasma concentration from an animal in Group 3 (Antibody 33-treated). This plot is is representative of the other 16 animals across the 3 groups that showed no significant effect on platelets.

Interestingly, the E85-50 $IgG_1$-treated monkey that showed a transient drop in platelet numbers after dosing on day 29 had the highest Cmax value (29400 nmol/L)(but not exposure) at this time. The plasma levels subsequently dropped sharply and the platelet counts returned to pre-dose values. This hints to the possibility that a higher threshold of plasma concentration might be required to evoke decreases in platelet numbers. However a single Antibody 33-treated animal reached similar levels (28500nm/L) with no corresponding platelet effects (Figure 8).

*Other Haematological Effects*

With the exception of platelet counts (see below), no consistent effects of mAb treatment were noted on the majority of of haematological parameters (haemoglobin concentration, packed cell volume, mean cell volume, mean cell haemoglobin concentration, red cell distribution width, platelet crit, platelet distribution width, red blood cell count, mean cell haemoglobin, haemoglobin distribution width, mean platelet volume, reticulocyte count, total and differential white cell count) and blood coagulation parameters (prothrombin time, activated partial thromboplastin time). An increase in the numbers of reticulocytes was observed in all groups however the changes were not dose/exposure-related, were not consistent within a group (animals within a group had higher, lower or unchanged levels from pre-dose values) or within an animal (values within animals rose and fell between time-points independent of exposure) and, in the absence of a parallel control group, the relationship to mAb treatment cannot be fully determined at this time. Any such changes had generally reversed at the end of the recovery period. No significant treatment-related effects on complement activation (C5a, C3a or BB fragments) were noted.

*Discussion and Conclusions*

This study has shown that anti-IgE mAbs E85-50 $IgG_1$, E85-50 $IgG_2$ and Antibody 33 were well-tolerated when administered at high repeated dose levels (up to 100mg/kg) to juvenile cynomolgus monkeys with no significant adverse toxicological effects. Only 1 animal out of 18 monkeys showed a significant drop in platelet numbers from mean baseline levels at a single timepoint after dosing with 100mg/kg E85-50 $IgG_1$ when plasma concentrations of mAb reached almost 30000 nmol/L. The plasma Cmax concentrations reached with all 3 mAbs in this study are expected to be far in excess of those that will be achieved in the clinic (e.g. 200nmol/L.

**References**

All references cited anywhere in this specification, including those cited anywhere above, are incorporated herein by reference in their entirety and for all purposes.

1 Haan & Maggos (2004) BioCentury, 12(5): A 1-A6

2 Koide et al. (1998) Journal of Molecular Biology, 284: 1141-1151.
3 Nygren et al. (1997) Current Opinion in Structural Biology, 7: 463-469
4 Wess, L. In: BioCentury, The Bernstein Report on BioBusiness, 12(42), A1-A7, 2004
5 Kabat, E.A. et al, Sequences of Proteins of Immunological Interest. 4th Edition. US Department of Health and Human Services. 1987
6 Kabat, E.A. et al. (1991) Sequences of Proteins of Immunological Interest, 5th Edition. US Department of Health and Human Services, Public Service, NIH, Washington
7 Segal et al., PNAS, 71:4298-4302, 1974
8 Amit et al., Science, 233:747-753, 1986
9 Chothia et al., J. Mol. Biol., 196:901-917, 1987
10 Chothia et al., Nature; 342:877- 883, 1989
11 Caton et al., J. Immunol., 144:1965-1968, 1990
12 Sharon et al., PNAS, 87:4814-4817, 1990
13 Sharon et al., J. Immunol., 144:4863-4869, 1990
14 Kabat et al., J. Immunol., 147:1709-1719, 1991
15 Holliger & Hudson, Nature Biotechnology 23(9):1126-1136 2005
16 Kontermann, R & Dubel, S, Antibody Engineering, Springer-Verlag New York, LLC; 2001, ISBN: 3540413545
17 Mendez, M. et al. (1997) Nature Genet, 15(2): 146-156
18 Knappik et al. J. Mol. Biol. (2000) 296, 57-86
19 Krebs et al. Journal of Immunological Methods 254 2001 67-84
20 Ward, E.S. et al., Nature 341, 544-546 (1989)
21 McCafferty et al (1990) Nature, 348, 552-554
22 Holt et al (2003) Trends in Biotechnology 21, 484-490
23 Bird et al, Science, 242, 423-426, 1988
24 Huston et al, PNAS USA, 85, 5879-5883, 1988
25 Holliger, P. et al, Proc. Natl. Acad. Sci. USA 90 6444-6448; 1993
26 Reiter, Y. et al, Nature Biotech, 14, 1239-1245, 1996
27 Hu, S. et al, Cancer Res., 56, 3055-3061, 1996
28 Holliger and Bohlen 1999 Cancer and metastasis rev. 18: 411-419
29 Holliger, P. and Winter G. Current Opinion Biotechnol 4, 446-449 1993
30 Glennie M J et al., 1987 J. Immunol. 139, 2367-2375
31 Repp R. et al., 1995 J. Hemat. 377-382
32 Staerz U. D. and Bevan M. J. 1986 PNAS 83
33 Suresh M. R. et al., 1986 Method Enzymol. 121: 210-228
34 Merchand et al., 1998 Nature Biotech. 16:677-681
35 Ridgeway, J. B. B. et al, Protein Eng., 9, 616-621, 1996
36 Harlow and Lane, Antibodies: A Laboratory Manual, Cold Spring Harbor Laboratory, Cold Spring Harbor N.Y., pp. 726, 1988
37 Köhler and Milstein, Nature, 256:495-497, 1975
38 Wold, et al. Multivariate data analysis in chemistry. Chemometrics -Mathematics and Statistics in Chemistry (Ed.: B. Kowalski), D. Reidel Publishing Company, Dordrecht, Holland, 1984 (ISBN 90-277-1846-6)
39 Norman et al. Applied Regression Analysis. Wiley-Interscience; 3rd edition (April 1998) ISBN: 0471170828
40 Kandel, Abraham & Backer, Eric. Computer-Assisted Reasoning in Cluster Analysis. Prentice Hall PTR, (May 11, 1995), ISBN: 0133418847
41 Krzanowski, Wojtek. Principles of Multivariate Analysis: A User's Perspective (Oxford Statistical Science Series, No 22 (Paper)). Oxford University Press; (December 2000), ISBN: 0198507089
42 Witten, Ian H. & Frank, Eibe. Data Mining: Practical Machine Learning Tools and Techniques with Java Implementations. Morgan Kaufmann; (October 11, 1999), ISBN: 1558605525
43 Denison David G. T. (Editor), Christopher C. Holmes, Bani K. Mallick, Adrian F. M. Smith. Bayesian Methods for Nonlinear Classification and Regression (Wiley Series in Probability and Statistics). John Wiley & Sons; (July 2002), ISBN: 0471490369
44 Ghose, Arup K. & Viswanadhan, Vellarkad N.. Combinatorial Library Design and Evaluation Principles, Software, Tools, and Applications in Drug Discovery. ISBN: 0-8247-0487-8
45 Chothia C. et al. Journal Molecular Biology (1992) 227, 799-817
46 Al-Lazikani, et al. Journal Molecular Biology (1997) 273(4), 927-948
47 Chothia, et al. Science, 223,755-758 (1986)
48 Whitelegg, N.R.u. and Rees, A.R (2000). Prot. Eng., 12, 815-824
49 Guex, N. and Peitsch, M.C. Electrophoresis (1997) 18, 2714-2723

50 Altschul et al. (1990) J. Mol. Biol. 215: 405-410
51 Pearson and Lipman (1988) PNAS USA 85: 2444-2448
52 Smith and Waterman (1981) J. Mol Biol. 147: 195-197
53 Voet & Voet, Biochemistry, 2nd Edition, (Wiley) 1995.
54 Gram et al., 1992, Proc. Natl. Acad. Sci., USA, 89:3576-3580
55 Barbas et al., 1994, Proc. Natl. Acad. Sci., USA, 91:3809-3813
56 Schier et al., 1996, J. Mol. Biol. 263:551-567
57 Marks et al Bio/Technology, 1992, 10:779-783
58 Kay, B.K., Winter, J., and McCafferty, J. (1996) Phage Display of Peptides and Proteins: A Laboratory Manual, San Diego: Academic Press
59 Hunter W. M. and Greenwood F. C. (1962) Nature 194:495
60 Plückthun, A. Bio/Tcchnology 9: 545-551 (1991)
61 Chadd HE and Chamow SM (2001) Current Opinion in Biotechnology 12: 188-194
62 Andersen DC and Krummen L (2002) Current Opinion in Biotechnology 13: 117
63 Larrick JW and Thomas DW (2001) Current Opinion in Biotechnology 12:411-418
64 Sambrook and Russell, Molecular Cloning: a Laboratory Manual: 3rd edition, 2001, Cold Spring Harbor Laboratory Press
65 Ausubel et al. eds., Short Protocols in Molecular Biology: A Compendium of Methods from Current Protocols in Molecular Biology, John Wiley & Sons, 4th edition 1999
66 Robinson, J. R. ed., Sustained and Controlled Release Drug Delivery Systems, Marcel Dekker, Inc., New York, 1978
67 Ledermann J.A. et al. (1991) Int. J. Cancer 47: 659-664
68 Bagshawe K.D. et al. (1991) Antibody, Immunoconjugates and Radiopharmaceuticals 4: 915-922
69 Vaughan, T. J., et al. (1996). Nature Biotechnology 14, 309-314.
70 Hutchings, C. Generation of Naive Human Antibody Libraries, in Antibody Engineering, R. Kontermann and S. Dubel, Editors. 2001, Springer Laboratory Manuals, Berlin. p. 93

## Table 1a

| Kabat numbering | HCDR1 | | | | | HCDR2 | | | | | | | | | | | | | | | | | | HCDR3 | | | | | | | | | | | |
|---|---|---|---|---|---|---|---|---|---|---|---|---|---|---|---|---|---|---|---|---|---|---|---|---|---|---|---|---|---|---|---|---|---|---|---|
| | 31 | 32 | 33 | 34 | 35 | 50 | 51 | 52 | 52A | 52B | 53 | 54 | 55 | 56 | 57 | 58 | 59 | 60 | 61 | 62 | 63 | 64 | 65 | 95 | 96 | 97 | 98 | 99 | 100 | 100A | 100B | 100C | 100D | 101 | 102 |
| Antibody 33 | S | Y | A | M | S | A | I | S | G | G | S | G | G | R | T | Y | Y | A | D | S | V | K | G | H | I | A | V | A | G | T | R | G | F | D | Y |
| Antibody 8 | | | | | | | | | | | | | | | | | | | | | | | | | | | | | | | | | | | |
| Antibody 15 | | | | | | | | | | | | | | | | | | | | | | | | | | | | | | | | | | | |
| Antibody 16 | | | | | | | | | | | | | | | | | | | | | | | | | | | | | | | | | | | |
| Antibody 17 | | | | | | | | | | | | | | | | | | | | | | | | | | | | | | | | | | | |
| Antibody 33GL | | | | | | | | | | | | | | | | | | | | | | | | | | | | | | | | | | | |
| Antibody 6 | | | | | | | | | | - | | | | | | | | | | | | | | | | | | | | | | | | | |
| Antibody 24 | | | | | | | | | | | | | | | | | | | | | | | | | | | | | | | | | | | |
| Antibody 25 | | | | | | | | | | | | | | | | | | | | | | | | | | | | | | | | | | | |
| Antibody 9 | | | | | | | | | | | | | | | | | | | | | | | | | | | | | | | | | | | |
| Antibody 2 | | | | | | | | | | - | | | | | | | | | | | | | | | | | | | | | | | | | |
| Antibody 3 | | | | | | | | | | - | | | | | | | | | | | | | | | | | | | | | | | | | |
| Antibody 18 | | | | V | | | | | | | | | | | | | | | | | | | | | | | | | | | | | | | |
| Antibody 19 | | | | | | | V | | | | | | | | | | | | | | | | | | | | | | | | | | | | |
| Antibody 1 | | | | | | | | | | - | | | | S | | | | | | | | | | | | | | | | | | | | | |
| Antibody 4 | | | | | | | | | | - | | | | | | | | | | | | | | | | | | | | | | | | | |
| Antibody 5 | | | | | | | | | | - | | | | | | | | | | | | | | | | | | | | | | | | | |

## Table 1b

| Kabat numbering | HCDR1 | | | | | HCDR2 | | | | | | | | | | | | | | | | | | HCDR3 | | | | | | | | | | | |
|---|---|---|---|---|---|---|---|---|---|---|---|---|---|---|---|---|---|---|---|---|---|---|---|---|---|---|---|---|---|---|---|---|---|---|---|
| | 31 | 32 | 33 | 34 | 35 | 50 | 51 | 52 | 52A | 52B | 53 | 54 | 55 | 56 | 57 | 58 | 59 | 60 | 61 | 62 | 63 | 64 | 65 | 95 | 96 | 97 | 98 | 99 | 100 | 100A | 100B | 100C | 100D | 101 | 102 |
| | S | Y | A | M | S | A | I | S | G | G | S | G | G | R | T | Y | Y | A | D | S | V | K | G | H | I | A | V | A | G | T | R | G | F | D | Y |
| Antibody 7 | | | | | | | | | | - | G | | | | | | | | | | | | | | | | | | | | | | | | |
| Antibody 23 | | | | | | | | | | | | | | | | | | | | | | | | | | | | | | | | | | | |
| Antibody 11 | | | | | | | | | | - | | | | | | | | | | | | | | | | | | | | | | | | | |
| Antibody 12 | | | | | | | | | | - | | | | | | | | | | | | | | | | | | | | | | | | | |
| Antibody 36 | | | | | | | | | | - | | | | | | | | | | | | | | | | | | | | | | | | | |
| Antibody 13 | | | | | | | | | | - | | | | | | | | | | | | | | | | | | | | | | | | | |
| Antibody 34 | | | | | | | | | | | | | | | | | | | | | | | | | | | | | | | | | | | |
| Antibody 14 | | | P | | | | | | | - | | | | S | | | | | | | | | | | | | | | | | | | | | |
| Antibody 22 | | | | | | | | | | - | | | | | | | | | | | A | | | | | | | | | | | | | | |
| Antibody 26 | G | | | | | | | | | - | | | | | | | | | | | | | | | | | | | | | | | | | |
| Antibody 27 | | | | | | | | | | - | | | | | | | | | | | | | | S | T | Y | F | | S | | | | | | |
| Antibody 28 | | | | | | | | | | - | | | | | | | | | | | | | | L | W | F | P | | I | | | | | | |
| Antibody 29 | | | | | | | | | | - | | | | | | | | | | | | | | L | T | F | P | | I | | | | | | |
| Antibody 30 | | | | | | | | | | - | | | | | | | | | | | | | | L | S | Y | P | | I | | | | | | |
| Antibody 32 | | | | | | | | | | - | | | | | | | | | | | | | | L | F | Y | P | Y | I | | | | | | |

## Table 1c

| Kabat numbering | HCDR1 | | | | | HCDR2 | | | | | | | | | | | | | | | | | | HCDR3 | | | | | | | | | | | |
|---|---|---|---|---|---|---|---|---|---|---|---|---|---|---|---|---|---|---|---|---|---|---|---|---|---|---|---|---|---|---|---|---|---|---|---|
| | 31 | 32 | 33 | 34 | 35 | 50 | 51 | 52 | 52A | 52B | 53 | 54 | 55 | 56 | 57 | 58 | 59 | 60 | 61 | 62 | 63 | 64 | 65 | 95 | 96 | 97 | 98 | 99 | 100 | 100A | 100B | 100C | 100D | 101 | 102 |
| | S | Y | A | M | S | A | I | S | G | G | S | G | G | R | T | Y | Y | A | D | S | V | K | G | H | I | A | V | A | G | T | R | G | F | D | Y |
| Antibody 20 | | | | | | | | | | - | | | | | | | | | | | | | | S | T | Y | F | | S | | | | | | |
| Antibody 21 | | | | | | | | | | - | | | | | | | | | | | | | | L | T | F | P | | I | | | | | | |
| Antibody 31 | | | | | | | | | | - | | | | | | | | | | | | | | L | S | Y | P | | I | | | | | | |
| Antibody 10 | | | | | | | | | | - | | | | S | | | | | | | | | | L | S | Y | P | | I | | | | | | |

**Table 1d**

| Kabat numbering | LCDR1 | | | | | | | | | | | LCDR2 | | | | | | | LCDR3 | | | | | | | | | | |
|---|---|---|---|---|---|---|---|---|---|---|---|---|---|---|---|---|---|---|---|---|---|---|---|---|---|---|---|---|---|
| | 24 | 25 | 26 | 27 | 28 | 29 | 30 | 31 | 32 | 33 | 34 | 50 | 51 | 52 | 53 | 54 | 55 | 56 | 89 | 90 | 91 | 92 | 93 | 94 | 95 | 95A | 95B | 96 | 97 |
| Antibody 33 | S | G | D | K | L | G | D | T | Y | A | S | Q | D | D | K | R | P | S | L | A | W | D | T | R | T | S | G | A | V |
| Antibody 8 | | | | | | | | | | | | | | | | | | | | | | | | | | | | | |
| Antibody 15 | | | | | | | | | | | | | | | R | | | | | | | | | | | | | | |
| Antibody 16 | | | | | | | | | | | | | | | | | | | | | | | | | | | | | |
| Antibody 17 | | | | | | | | | | | | | | | | | | | | | | | | | | | | | |
| Antibody 33GL | | | | | | | | | | | | | | | | | | | | | | | | | | | | | |
| Antibody 6 | | | | | | | | | | | | | | | | | | | | | | | | | | | | | |
| Antibody 24 | | | | | | | | | | | | | | | | | | | | | | | | | | | | | |
| Antibody 25 | | | | | | | | | | | | | | | | | | | | | | | | | | | | | |
| Antibody 9 | | | | | | | | | | | | | | | | | | | | | | | | | | | | | |
| Antibody 2 | | | | | | | | | | | | | | | R | | | | | | | | | | | | | | |
| Antibody 3 | | | | | | | | | | | | | | | | | | | | | | | | | | | | | |
| Antibody 18 | | | | | | | | | | | | | | | | | | | | | | | | | | | | | |
| Antibody 19 | | | | | | | | | | | | | | | | | | | | | | | | | | | | | |
| Antibody 1 | | | | | | | | K | | | | | | | | | | | | | | | | | | | | | |
| Antibody 4 | | | | | | | | | | | | | | | | | | | | | | | | | | | | | |
| Antibody 5 | | | | | | | | | | | | | | | R | | | | | | | | | | | | | | |

## Table 1e

| Kabat numbering | LCDR1 | | | | | | | | | | | LCDR2 | | | | | | | LCDR3 | | | | | | | | | | | |
|---|---|---|---|---|---|---|---|---|---|---|---|---|---|---|---|---|---|---|---|---|---|---|---|---|---|---|---|---|---|---|
| | 24 | 25 | 26 | 27 | 28 | 29 | 30 | 31 | 32 | 33 | 34 | 50 | 51 | 52 | 53 | 54 | 55 | 56 | 89 | 90 | 91 | 92 | 93 | 94 | 95 | 95A | 95B | 96 | 97 |
| | S | G | D | K | L | G | D | T | Y | A | S | Q | D | D | K | R | P | S | L | A | W | D | T | R | T | S | G | A | V |
| Antibody 7 | | | | | | | | | | | | | | | | | | | | | | | | | | | | | |
| Antibody 23 | | | | | | | | | | | | | | | | | | | | | | | | | | | | | |
| Antibody 11 | | | | | | | | | | | | | | | | | | | | | | | | | | | | | |
| Antibody12 | | | | | | | | | | | | | | | | | | | | | | | | | | | | | |
| Antibody 36 | | | | | | | | | | | | | | | | | | | | | | | | | | | | | |
| Antibody 13 | | | | | | | | | | | | | | | R | | | | | | | | | | | | | | |
| Antibody 34 | | | | | | | | | | | | | | | | | | P | | | | | | | | | | | |
| Antibody 14 | | | | | | | | | | | | | | | | | | | | | | | | | | | | | |
| Antibody 22 | | | | | | | | | | T | | | | | | | | | | | | | | | | | | | |
| Antibody 26 | | | | | | | | | | | | | | | | | | | | | | | | | | | | | |
| Antibody 27 | | | | | | | | | | | | | | | | | | | | | | | | | | | | | |
| Antibody 28 | | | | | | | | | | | | | | | | | | | | | | | | | | | | | |
| Antibody 29 | | | | | | | | | | | | | | | | | | | | | | | | | | | | | |
| Antibody 30 | | | | | | | | | | | | | | | | | | | | | | | | | | | | | |
| Antibody 32 | | | | | | | | | | | | | | | | | | | | | | | | | | | | | |

**Table 1f**

| Kabat numbering | LCDR1 | | | | | | | | | | | LCDR2 | | | | | | | LCDR3 | | | | | | | | | | |
|---|---|---|---|---|---|---|---|---|---|---|---|---|---|---|---|---|---|---|---|---|---|---|---|---|---|---|---|---|---|
| | 24 | 25 | 26 | 27 | 28 | 29 | 30 | 31 | 32 | 33 | 34 | 50 | 51 | 52 | 53 | 54 | 55 | 56 | 89 | 90 | 91 | 92 | 93 | 94 | 95 | 95A | 95B | 96 | 97 |
| | S | G | D | K | L | G | D | T | Y | A | S | Q | D | D | K | R | P | S | L | A | W | D | T | R | T | S | G | A | V |
| Antibody 20 | | | | | | | | K | | | | | | | | | | | | | | | | | | | | | |
| Antibody 21 | | | | | | | | K | | | | | | | | | | | | | | | | | | | | | |
| Antibody 31 | | | | | | | | K | | | | | | | | | | | | | | | | | | | | | |
| Antibody 10 | | | | | | | | K | | | | | | | | | | | | | | | | | | | | | |

# Table 2a

| Kabat numbering | FW1 | | | | | | | | | | | | | | | | | | | | | | | LCDR1 | | | | | | | | | | |
|---|---|---|---|---|---|---|---|---|---|---|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 |
| Antibody 33 | S | Y | V | L | T | Q | P | P | S | - | V | S | V | S | P | G | Q | T | A | S | I | T | C | S | G | D | K | L | G | D | T | Y | A | S |
| Antibody 8 | | | | | | | | | | | | | | | | | | | | | | | | | | | | | | | | | | |
| Antibody 15 | | | | | | | | | | | | | | | | | | | | | | | | | | | | | | | | | | |
| Antibody 16 | | | | | | | | | | | | | | | | | | | | | | | | | | | | | | | | | | |
| Antibody 17 | | | | | | | | | | | | | | | | | | | | | | | | | | | | | | | | | | |
| Antibody 33GL | | | E | | | | | | | | | | | | | | | | | | | | | | | | | | | | | | | |
| Antibody 6 | | | | | | | | | | | | | | | | | | | | | | | | | | | | | | | | | | |
| Antibody 24 | | F | | | | | | | | | | | | | | | | | | | | | | | | | | | | | | | | |
| Antibody 25 | | F | | | | | | | | | | | | | | | | | | | | | | | | | | | | | | | | |
| Antibody 9 | | F | | | S | | | | | | | | | | | | | | | | | | | | | | | | | | | | | |
| Antibody 2 | | | | | | | | | | | | | | | | | | | | | | | | | | | | | | | | | | |
| Antibody 3 | | | | | | | | | | | | | | | | | | | | | | | | | | | | | | | | | | |
| Antibody 18 | | | | | | | | | | | | | | | | | | | | | | | | | | | | | | | | | | |
| Antibody 19 | | F | | | | | | | | | | | | | | | | | | | | | | | | | | | | | | | | |
| Antibody 1 | | | | | | | | | | | | | | | | | | | | | | | | | | | | | | | K | | | |
| Antibody 4 | | F | | | | | | | | | | | | | | | | | | | | | | | | | | | | | | | | |
| Antibody 5 | | | | | | | | | | | | | | | | | | | | | | | | | | | | | | | | | | |
| Antibody 7 | | | | | | | | | | | | | A | | | | | | | | | | | | | | | | | | | | | |
| Antibody 23 | | | | | | | | | | | | | | | | | | | | | | | | | | | | | | | | | | |

## Table 2b

| Kabat numbering | FW1 | | | | | | | | | | | | | | | | | | | | | | | LCDR1 | | | | | | | | | | |
|---|---|---|---|---|---|---|---|---|---|---|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 |
| | S | Y | V | L | T | Q | P | P | S | - | V | S | V | S | P | G | Q | T | A | S | I | T | C | S | G | D | K | L | G | D | T | Y | A | S |
| Antibody 11 | | | | | | | | | | | | | | | | | | | | | | A | | | | | | | | | | | | |
| Antibody 12 | | F | M | | S | | | | | | | | | | | | | | | | | | | | | | | | | | | | | |
| Antibody 36 | | F | M | | S | | | | | | | | | | | | | | | | | | | | | | | | | | | | | |
| Antibody 13 | | | | | | | | | | | | | | | | | | | | | | | | | | | | | | | | | | |
| Antibody 34 | | | | | | | | | | | | | A | | | | | | | | | | | | | | | | | | | | | |
| Antibody 14 | | F | | | S | | | | | | | | | | | | | | | | | | | | | | | | | | | | | |
| Antibody 22 | | | | | | | | | | | | | | | | | | | | | | | | | | | | | | | | | T | |
| Antibody 26 | | F | | | | | | | | | | | | | | | | | | | | | | | | | | | | | | | | |
| Antibody 27 | | | | | | | | | | | | | | | | | | | | | | | | | | | | | | | | | | |
| Antibody 28 | | | | | | | | | | | | | | | | | | | | | | | | | | | | | | | | | | |
| Antibody 29 | | | | | | | | | | | | | | | | | | | | | | | | | | | | | | | | | | |
| Antibody 30 | | | | | | | | | | | | | | | | | | | | | | | | | | | | | | | | | | |
| Antibody 32 | | | | | | | | | | | | | | | | | | | | | | | | | | | | | | | | | | |
| Antibody 20 | | | | | | | | | | | | | | | | | | | | | | | | | | | | | | | K | | | |
| Antibody 21 | | | | | | | | | | | | | | | | | | | | | | | | | | | | | | | K | | | |
| Antibody 31 | | | | | | | | | | | | | | | | | | | | | | | | | | | | | | | K | | | |
| Antibody 10 | | | | | | | | | | | | | | | | | | | | | | | | | | | | | | | K | | | |

**Table 2c**

| Kabat numbering | FW2 | | | | | | | | | | | | | | LCDR2 | | | | | | | | FW3 | | | | | | | | | | | | | | |
|---|---|---|---|---|---|---|---|---|---|---|---|---|---|---|---|---|---|---|---|---|---|---|---|---|---|---|---|---|---|---|---|---|---|---|---|---|---|
| | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 | 66 | 67 | 68 | 69 | 70 | 71 |
| **Antibody 33** | W | Y | R | Q | R | S | G | L | S | P | A | L | V | I | **Y** | **Q** | **D** | **D** | **K** | **R** | **P** | **S** | G | I | P | E | R | F | S | G | S | N | S | G | N | T | A |
| **Antibody 8** | | | | | | | | **Q** | | | **V** | | | | | | | | | | | | | | | | | | | | | | | | | | |
| **Antibody 15** | | | | | | | | **Q** | | | **V** | | | | | | | | **R** | | | | | | | | | | | | | | | | | | |
| **Antibody 16** | | | | | | | | **Q** | | | **V** | | | | | | | | | | | | | | | | | | | | | | | | | | |
| **Antibody 17** | | | | | | | | **Q** | | | **V** | | | | | | | | | | | | | | | | | | | | | | | | **D** | | |
| **Antibody 33GL** | | | **Q** | | **K** | **P** | | | | | | | | | | | | | | | | | | | | | | | | | | | | | | | |
| **Antibody 6** | | | | | | | | **Q** | | | **V** | | | | | | | | | | | | | | | | | | | | | | | | | | |
| **Antibody 24** | | | | | | | | **Q** | | | **V** | | | | | | | | | | | | | | | | | | | | | | | | | | |
| **Antibody 25** | | | | | | | | **Q** | | | **V** | | | | | | | | | | | | | | | | | | | | | | | | | | |
| **Antibody 9** | | | | | | | | **Q** | | | **V** | | | | | | | | | | | | | | | | | | | | | | | | | | |
| **Antibody 2** | | | | | | | | **Q** | | | **V** | | | | | | | | **R** | | | | | | | | | | | | | | | | | | |
| **Antibody 3** | | | | | | | | **Q** | | | **V** | | | | | | | | | | | | | | | | | | | | | | | | **D** | | |
| **Antibody 18** | | | | | | | | **Q** | | | **V** | | | | | | | | | | | | | | | | | | | | | | | | **D** | | |
| **Antibody 19** | | | | | | | | **Q** | | | **V** | | | | | | | | | | | | | | | | | | | | | | | | | | |
| **Antibody 1** | | | | | | | | **Q** | | | **V** | | | | | | | | | | | | | | | | | | | | | | | | | | |
| **Antibody 4** | | | | | | | | **Q** | | | **V** | | | | | | | | | | | | | | | | | | | | | | | | | | |
| **Antibody 5** | | | | | | | | **Q** | | | **V** | | | | | | | | **R** | | | | | | | | | | | | | | | | | | |
| **Antibody 7** | | | | | | | | **Q** | | | **V** | | | | | | | | | | | | | | | | | | | | | | | | | | |
| **Antibody 23** | | | | | | | | **Q** | | | **V** | | | | | | | | | | | | | | | | | | | | | | | | | **A** | |

**Table 2d**

| Kabat numbering | FW2 | | | | | | | | | | | | | | LCDR2 | | | | | | | | FW3 | | | | | | | | | | | | | | |
|---|---|---|---|---|---|---|---|---|---|---|---|---|---|---|---|---|---|---|---|---|---|---|---|---|---|---|---|---|---|---|---|---|---|---|---|---|---|
| | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 | 66 | 67 | 68 | 69 | 70 | 71 |
| | W | Y | R | Q | R | S | G | L | S | P | A | L | V | I | Y | Q | D | D | K | R | P | S | G | I | P | E | R | F | S | G | S | N | S | G | N | T | A |
| Antibody 11 | | | | | | | | Q | | | V | | | | | | | | | | | | | | | | | | | | | | | | | | |
| Antibody 12 | | | | | | | | Q | | | V | | | | | | | | | | | | | | | | | | | | | | | | | | |
| Antibody 36 | | | | | | | | Q | | | V | | | | | | | | | | | | | | | | | | | | | | | | | | |
| Antibody 13 | | | | | | | | Q | | | V | | | | | | | | R | | | | | | | | | | | | | | | | | | |
| Antibody 34 | | | | | | | | Q | | | V | | | | | | | | | | | P | | | | | | | | | | | | | | | |
| Antibody 14 | | | | | | | | Q | | | V | | | | | | | | | | | | | | | | | | | | | | | | | | |
| Antibody 22 | | | | | | | | Q | | | V | | | | | | | | | | | | | V | | | | | | | | | | | | | |
| Antibody 26 | | | | | | | | Q | | | V | | | | | | | | | | | | | | | | | | | | | | | | | | |
| Antibody 27 | | | | | | | | Q | | | V | | | | | | | | | | | | | | | | | | | | | | | | | | |
| Antibody 28 | | | | | | | | Q | | | V | | | | | | | | | | | | | | | | | | | | | | | | | | |
| Antibody 29 | | | | | | | | Q | | | V | | | | | | | | | | | | | | | | | | | | | | | | | | |
| Antibody 30 | | | | | | | | Q | | | V | | | | | | | | | | | | | | | | | | | | | | | | | | |
| Antibody 32 | | | | | | | | Q | | | V | | | | | | | | | | | | | | | | | | | | | | | | | | |
| Antibody 20 | | | | | | | | Q | | | V | | | | | | | | | | | | | | | | | | | | | | | | | | |
| Antibody 21 | | | | | | | | Q | | | V | | | | | | | | | | | | | | | | | | | | | | | | | | |
| Antibody 31 | | | | | | | | Q | | | V | | | | | | | | | | | | | | | | | | | | | | | | | | |
| Antibody 10 | | | | | | | | Q | | | V | | | | | | | | | | | | | | | | | | | | | | | | | | |

**Table 2e**

| Kabat numbering | FW3 | | | | | | | | | | | | | | | | | | LCDR3 | | | | | | | | | | FW4 | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|
| | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | 95A | 95B | 96 | 97 | 98 | 99 | 100 | 101 | 102 | 103 | 104 | 105 | 106 | 107 |
| Antibody 33 | T | L | T | I | S | G | T | H | T | M | D | E | A | D | Y | Y | C | L | A | W | D | T | R | T | S | G | A | V | F | G | G | G | T | K | L | T | V | L |
| Antibody 8 | | | | | | | | | | | | | | | | | | | | | | | | | | | | | | | | | | | | | | |
| Antibody 15 | | | | | | | | | | | | | | | | | | | | | | | | | | | | | | | | | | | | | | |
| Antibody 16 | | | | | | | | | | | | | | | | | | | | | | | | | | | | | | | | | | E | | | | |
| Antibody 17 | | | | | | | | | | | | | | | | | | | | | | | | | | | | | | | | | | | | | | |
| Antibody 33GL | | | | | | | | Q | A | | | | | | | | | | | | | | | | | | | | | | | | | | | | | |
| Antibody 6 | | | | | | | | | | | | | | | | | | | | | | | | | | | | | | | | | | | | | | |
| Antibody 24 | | | | | | | | | | | | | | | | | | | | | | | | | | | | | | | | | | | | | | |
| Antibody 25 | | | | | | | | | | | | | | | | | | | | | | | | | | | | | | | | | | | | | | |
| Antibody 9 | | | | | | | | | | | | | | | | | | | | | | | | | | | | | | | | | | | | | | |
| Antibody 2 | | | | | | | | | | | | | | | | | | | | | | | | | | | | | | | | | | | | | | |
| Antibody 3 | | | | | | | | | | | | | | | | | | | | | | | | | | | | | | | | | | | | | | |
| Antibody 18 | | | | | | | | R | | | | | | | | | | | | | | | | | | | | | | | | | | | | | | |
| Antibody 19 | | | | | | R | | | | | | | | | | | | | | | | | | | | | | | | | | | | | | | | |
| Antibody 1 | | | | | | | | | | | | | | | | | | | | | | | | | | | | | | | | | | | | | | |
| Antibody 4 | | | | | | | | | | | | | | | | | | | | | | | | | | | | | | | | | | | | | | |
| Antibody 5 | | | | | | | | | | | | | | | | | | | | | | | | | | | | | | | | | | | | | A | |
| Antibody 7 | | | | | | | | | | | | | | | | | | | | | | | | | | | | | | | | | | | | | | |
| Antibody 23 | | | | | | | | | | | | | | | | | | | | | | | | | | | | | | | | | | | | | | |

## Table 2f

| Kabat numbering | FW3 | | | | | | | | | | | | | | | | | LCDR3 | | | | | | | | | FW4 | | | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|
| | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | 95A | 95B | 96 | 97 | 98 | 99 | 100 | 101 | 102 | 103 | 104 | 105 | 106 | 107 |
| Antibody 11 | T | L | T | I | S | G | T | H | T | M | D | E | A | D | Y | Y | C | L | A | W | D | T | R | T | S | G | A | V | F | G | G | G | T | K | L | T | V | L |
| Antibody 12 | | | | | | | | | | | | | | | | | | | | | | | | | | | | | | | | | | | | | | |
| Antibody 36 | | | | | | | | | | | | | | | | | | | | | | | | | | | | | | | | | | | | | | |
| Antibody 13 | | | | | | | | | | | | | | | | | | | | | | | | | | | | | | | | | | | | | | |
| Antibody 34 | | | | | R | | | | | | | | | | | | | | | | | | | | | | | | | | | | | | | S | | |
| Antibody 14 | | | | | | | | | | | | | | | | | | | | | | | | | | | | | | | | | | | | | | |
| Antibody 22 | | | | | | | | | | | | | | | | | | | | | | | | | | | | | | | | | | | | | | |
| Antibody 26 | | | | | G | | | | | | | | | | | | | | | | | | | | | | | | | | | | | | | | | |
| Antibody 27 | | | | | | | | | | | | | | | | | | | | | | | | | | | | | | | | | | | | | | |
| Antibody 28 | | | | | | | | | | | | | | | | | | | | | | | | | | | | | | | | | | | | | | |
| Antibody 29 | | | | | | | | | | | | | | | | | | | | | | | | | | | | | | | | | | | | | | |
| Antibody 30 | | | | | | | | | | | | | | | | | | | | | | | | | | | | | | | | | | | | | | |
| Antibody 32 | | | | | | | | | | | | | | | | | | | | | | | | | | | | | | | | | | | | | | |
| Antibody 20 | | | | | | | | | | | | | | | | | | | | | | | | | | | | | | | | | | | | | | |
| Antibody 21 | | | | | | | | | | | | | | | | | | | | | | | | | | | | | | | | | | | | | | |
| Antibody 31 | | | | | | | | | | | | | | | | | | | | | | | | | | | | | | | | | | | | | | |
| Antibody 10 | | | | | | | | | | | | | | | | | | | | | | | | | | | | | | | | | | | | | | |

**Table 3a**

| Kabat numbering | FW1 | | | | | | | | | | | | | | | | | | | | | | | | | | | | | HCDR1 | | | | |
|---|---|---|---|---|---|---|---|---|---|---|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 |
| Antibody 33 | E | V | Q | L | L | E | S | G | G | G | L | V | Q | P | G | G | S | L | S | L | S | C | A | A | P | G | F | T | F | S | S | Y | A | M | S |
| Antibody 8 | | | | | | | | | | | | | | | | | | | R | | | | | | | | | | | | | | | | |
| Antibody 15 | | | | | | | | | | | | | | | | | | | R | | | | | | | | | | | | | | | | |
| Antibody 16 | | | | | | | | | | | | | | | | | | | R | | | | | | | | | | | | | | | | |
| Antibody 17 | | | | | | | | | | | | | | | | | | | R | | | | | | | | | | | | | | | | |
| Antibody 33GL | | | | | | | | | | | | | | | | | | | | | | | | | | | | | | | | | | | |
| Antibody 6 | | | | | | | | | | | | | | | | | | | R | | | | | | S | | | | | | | | | | |
| Antibody 24 | | | | | | | | | | | | | | | | | | | R | | | | | | | | | | | | | | | | |
| Antibody 25 | | | | | | | | | | | | | | | | | | | R | | | | | | | | | | | | | | | | |
| Antibody 9 | | | | | | | | | | | | | | | | | | | R | | | | | | S | | | | | | | | | | |
| Antibody 2 | | | | | | | | | | | | | | | | | | | R | | | | | | S | | | | | | | | | | |
| Antibody 3 | | | | | | | | | | | | | | | | | | | R | | | | | | S | | | | | | | | | | |
| Antibody 18 | | | | | | | | | | | | | | | | | | | R | | | | | | S | | | | | | | | | V | |
| Antibody 19 | | | | | | | | | | | | | | | | | | | R | | | | | | S | | | | | | | | | | |
| Antibody 1 | | | | | | | | | | | | | | | | | | | R | | | | | | S | | | | | | | | | | |
| Antibody 4 | | | | | | | | | | | | | | | | | | | R | | | | | | S | | | | | | | | | | |
| Antibody 5 | | | | | | | | | | | | | | | | | | | R | | | | | | S | | | | | | | | | | |
| Antibody 7 | | | | | | | | | | | | | | | | | | | R | | | | | | S | | | | | | | | | | |
| Antibody 23 | | | | | | | | | | | | | | | | | | | R | | | | | | S | E | | | | G | | | | | |

**Table 3b**

| Kabat numbering | FW1 | | | | | | | | | | | | | | | | | | | | | | | | | | | | | | HCDR1 | | | | |
|---|---|---|---|---|---|---|---|---|---|---|---|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 |
| | E | V | Q | L | L | E | S | G | G | G | L | V | Q | P | G | G | S | L | S | L | S | C | A | A | P | G | F | T | F | S | S | Y | A | M | S |
| Antibody 11 | | | | | | | | | | | | | | | | | | | R | | | | | | S | | | | | | | | | | |
| Antibody 12 | | | | | | | | | | | | | | | | | | | R | | | | | | S | | | | | | | | | | |
| Antibody 36 | | | | | | | | | | | | | | | | | | | R | | | | | | S | | | | | | | | | | |
| Antibody 13 | | | | | | | | | | | | | | | | | | | R | | | | | | S | | | | | | | | | | |
| Antibody 34 | | | | | | | | | | | | | | | | | | | R | | | | | | | | | | L | | | | | | |
| Antibody 14 | | | | | | | | | | | | | | | | | | | R | | | | | | | | | | | | | | P | | |
| Antibody 22 | | | | | | | | | | | | | | | | | | | R | | | | | | S | | | | | | | | | | |
| Antibody 26 | | | | | | | | | | | | | | | | | | | R | | | | | | S | | L | | | | G | | | | |
| Antibody 27 | | | | | | | | | | | | | | | | | | | R | | | | | | S | | | | | | | | | | |
| Antibody 28 | | | | | | | | | | | | | | | | | | | R | | | | | | S | | | | | | | | | | |
| Antibody 29 | | | | | | | | | | | | | | | | | | | R | | | | | | S | | | | | | | | | | |
| Antibody 30 | | | | | | | | | | | | | | | | | | | R | | | | | | S | | | | | | | | | | |
| Antibody 32 | | | | | | | | | | | | | | | | | | | R | | | | | | S | | | | | | | | | | |
| Antibody 20 | | | | | | | | | | | | | | | | | | | R | | | | | | S | | | | | | | | | | |
| Antibody 21 | | | | | | | | | | | | | | | | | | | R | | | | | | S | | | | | | | | | | |
| Antibody 31 | | | | | | | | | | | | | | | | | | | R | | | | | | S | | | | | | | | | | |
| Antibody 10 | | | | | | | | | | | | | | | | | | | R | | | | | | S | | | | | | | | | | |

# Table 3c

| Kabat numbering | FW2 | | | | | | | | | | | | | | HCDR2 | | | | | | | | | | | | | | | | | |
|---|---|---|---|---|---|---|---|---|---|---|---|---|---|---|---|---|---|---|---|---|---|---|---|---|---|---|---|---|---|---|---|---|
| | 36 | 37 | 38 | 39 | 40 | 41 | 42 | 43 | 44 | 45 | 46 | 47 | 48 | 49 | 50 | 51 | 52 | 52A | 52B | 53 | 54 | 55 | 56 | 57 | 58 | 59 | 60 | 61 | 62 | 63 | 64 | 65 |
| **Antibody 33** | W | V | R | Q | A | P | G | K | G | L | E | W | V | S | A | I | S | G | G | S | G | G | R | T | Y | Y | A | D | S | V | K | G |
| **Antibody 8** | | | | | | | | | | | | | | | | | | | | | | | | | | | | | | | | |
| **Antibody 15** | | | | | | | | | | | | | | | | | | | | | | | | | | | | | | | | |
| **Antibody 16** | | | | | | | | | | | | | | | | | | | | | | | | | | | | | | | | |
| **Antibody 17** | | | | | | | | | | | | | | | | | | | | | | | | | | | | | | | | |
| **Antibody 33GL** | | | | | | | | | | | | | | | | | | | | | | | | | | | | | | | | |
| **Antibody 6** | | | | | | | | | | | | | | | | | | | - | | | | | | | | | | | | | |
| **Antibody 24** | | | | | | | | | | | | | | | | | | | | | | | | | | | | | | | | |
| **Antibody 25** | | | | | | | | | | | | | | | | | | | | | | | | | | | | | | | | |
| **Antibody 9** | | | | | | | | | | | | | | | | | | | | | | | | | | | | | | | | |
| **Antibody 2** | | | | | | | | | | | | | | | | | | | - | | | | | | | | | | | | | |
| **Antibody 3** | | | | | | | | | | | | | | | | | | | - | | | | | | | | | | | | | |
| **Antibody 18** | | | | | | | | | | | | | | | | | | | | | | | | | | | | | | | | |
| **Antibody 19** | | | | | | | | | | | | | | | | V | | | | | | | | | | | | | | | | |
| **Antibody 1** | | | | | | | | | | | | | | | | | | | - | | | | S | | | | | | | | | |
| **Antibody 4** | | | | | | | | | | | | | | | | | | | - | | | | | | | | | | | | | |
| **Antibody 5** | | | | | | | | | | | | | | | | | | | - | | | | | | | | | | | | | |
| **Antibody 7** | | | | | | | | | | | | | | | | | | | - | G | | | | | | | | | | | | |
| **Antibody 23** | | | | | | | | | | | | | | | | | | | | | | | | | | | | | | | | |

**Table 3d**

| | FW2 | | | | | | | | | | | | | | HCDR2 | | | | | | | | | | | | | | | | | |
|---|---|---|---|---|---|---|---|---|---|---|---|---|---|---|---|---|---|---|---|---|---|---|---|---|---|---|---|---|---|---|---|---|
| Kabat numbering | 36 | 37 | 38 | 39 | 40 | 41 | 42 | 43 | 44 | 45 | 46 | 47 | 48 | 49 | 50 | 51 | 52 | 52A | 52B | 53 | 54 | 55 | 56 | 57 | 58 | 59 | 60 | 61 | 62 | 63 | 64 | 65 |
| | W | V | R | Q | A | P | G | K | G | L | E | W | V | S | A | I | S | G | G | S | G | G | R | T | Y | Y | A | D | S | V | K | G |
| Antibody 11 | | | | | | | | | | | | | | | | | | | - | | | | | | | | | | | | | |
| Antibody 12 | | | | | | | | | | | | | | | | | | | - | | | | | | | | | | | | | |
| Antibody 36 | | | | | | | | | | | | | | | | | | | - | | | | | | | | | | | | | |
| Antibody 13 | | | | | | | | | | | | | | | | | | | - | | | | | | | | | | | | | |
| Antibody 34 | | | | | | | | | | | | | | | | | | | | | | | | | | | | | | | | |
| Antibody 14 | | | | | | | | | | | | | | | | | | | - | | | | S | | | | | | | | | |
| Antibody 22 | | | | | | | | | | | | | | | | | | | - | | | | | | | | | | | A | | |
| Antibody 26 | | | | | | | | | | | | | | | | | | | - | | | | | | | | | | | | | |
| Antibody 27 | | | | | | | | | | | | | | | | | | | - | | | | | | | | | | | | | |
| Antibody 28 | | | | | | | | | | | | | | | | | | | - | | | | | | | | | | | | | |
| Antibody 29 | | | | | | | | | | | | | | | | | | | - | | | | | | | | | | | | | |
| Antibody 30 | | | | | | | | | | | | | | | | | | | - | | | | | | | | | | | | | |
| Antibody 32 | | | | | | | | | | | | | | | | | | | - | | | | | | | | | | | | | |
| Antibody 20 | | | | | | | | | | | | | | | | | | | - | | | | | | | | | | | | | |
| Antibody 21 | | | | | | | | | | | | | | | | | | | - | | | | | | | | | | | | | |
| Antibody 31 | | | | | | | | | | | | | | | | | | | - | | | | | | | | | | | | | |
| Antibody 10 | | | | | | | | | | | | | | | | | | | - | | | | S | | | | | | | | | |

## Table 3e

| Kabat numbering | FW3 | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|
| | 66 | 67 | 68 | 69 | 70 | 71 | 72 | 73 | 74 | 75 | 76 | 77 | 78 | 79 | 80 | 81 | 82 | 82A | 82B | 82C | 83 | 84 | 85 | 86 | 87 | 88 | 89 | 90 | 91 | 92 | 93 | 94 |
| **Antibody 33** | R | F | T | I | S | K | D | N | S | K | N | T | L | Y | L | Q | M | N | S | L | R | A | E | D | T | A | V | Y | Y | C | A | R |
| **Antibody 8** | | | | | | R | | | | | | | | | | | | | | | | | | | | | | | | | | |
| **Antibody 15** | | | | | | R | | | | | | | | | | | | | | | | | | | | | | | | | | |
| **Antibody 16** | | | | | | R | | | | | | | | | | | | | | | | | | | | | | | | | | |
| **Antibody 17** | | | | | | R | | | | | | | | | | | | | | | | | | | | | | | | | | |
| **Antibody 33GL** | | | | | | | | | | | | | | | | | | | | | | | | | | | | | | | | |
| **Antibody 6** | | | | | | | | | | | | | | | | | | | | | | | | | | | | | | | | |
| **Antibody 24** | | | | | | R | | | | | | | | | | | | | | | | | | | | | | | | | | |
| **Antibody 25** | | | | | | R | | | | | | | | | | | | | | | | | | | | | | | | | | |
| **Antibody 9** | | | | | | R | | | | | | | | | | | | | | | | | | | | | | | | | | |
| **Antibody 2** | | | | | | R | | | | | | | | | | | | | | | | | | | | | | | | | | |
| **Antibody 3** | | | | | | R | | | | | | | | | | | | | | | | | | | | | | | | | | |
| **Antibody 18** | | | | | | R | | | | | | | | | | | | | | | | | | | | | | | | | | |
| **Antibody 19** | | | | | | R | | | | | | | | | | | | | | | | | | | | | | | | | | |
| **Antibody 1** | | | | | | R | | | | | | | | | | | | | | | | | | | | | | | | | | |
| **Antibody 4** | | | | | | R | | | | | | | | | | | | | | | | | | | | | | | | | | |
| **Antibody 5** | | | | | | R | | | | | | | | | | | | | | | | | | | | | | | | | | |
| **Antibody 7** | | | | | | R | | | | | | | | | | | | | | | | | | | | | | | | | | |
| **Antibody 23** | | | | | | R | | | | | | | | | | | | | | | | | | | | | | | | | | |

**Table 3f**

| Kabat numbering | FW3 | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|
| | 66 | 67 | 68 | 69 | 70 | 71 | 72 | 73 | 74 | 75 | 76 | 77 | 78 | 79 | 80 | 81 | 82 | 82A | 82B | 82C | 83 | 84 | 85 | 86 | 87 | 88 | 89 | 90 | 91 | 92 | 93 | 94 |
| | R | F | T | I | S | K | D | N | S | K | N | T | L | Y | L | Q | M | N | S | L | R | A | E | D | T | A | V | Y | Y | C | A | R |
| Antibody 11 | | | | | | R | | | | | | | | | | | | | | P | | | | | | | | | | | | |
| Antibody 12 | | | | | | R | | | | | | | | | | | | | | | | | | | | | | | | | | |
| Antibody 36 | | | | | | R | | | | | | | | | | | | | | | | | | | | | | | | | | |
| Antibody 13 | | | A | V | | R | | | | | | | | | | | | | | | | | | | | | | | | | | |
| Antibody 34 | | | | | | R | | | | | | | | | | | | | | | | | | | | | | | | | | |
| Antibody 14 | | | | | | R | | | | | | | | | | | | | | | | | | | | | | | | | | |
| Antibody 22 | | | | | | R | | | | | | | | | | | | | G | | | | | | | | | | | | | |
| Antibody 26 | | | | | | R | | | | | | M | | | | | | | | | | | | | | | | | | | | |
| Antibody 27 | | | | | | R | | | | | | | | | | | | | | | | | | | | | | | | | | |
| Antibody 28 | | | | | | R | | | | | | | | | | | | | | | | | | | | | | | | | | |
| Antibody 29 | | | | | | R | | | | | | | | | | | | | | | | | | | | | | | | | | |
| Antibody 30 | | | | | | R | | | | | | | | | | | | | | | | | | | | | | | | | | |
| Antibody 32 | | | | | | R | | | | | | | | | | | | | | | | | | | | | | | | | | |
| Antibody 20 | | | | | | R | | | | | | | | | | | | | | | | | | | | | | | | | | |
| Antibody 21 | | | | | | R | | | | | | | | | | | | | | | | | | | | | | | | | | |
| Antibody 31 | | | | | | R | | | | | | | | | | | | | | | | | | | | | | | | | | |
| Antibody 10 | | | | | | R | | | | | | | | | | | | | | | | | | | | | | | | | | |

## Table 3g

| Kabat numbering | HCDR3 | | | | | | | | | | | | FW4 | | | | | | | | | | |
|---|---|---|---|---|---|---|---|---|---|---|---|---|---|---|---|---|---|---|---|---|---|---|---|
| | 95 | 96 | 97 | 98 | 99 | 100 | 100A | 100B | 100C | 100D | 101 | 102 | 103 | 104 | 105 | 106 | 107 | 108 | 109 | 110 | 111 | 112 | 113 |
| Antibody 33 | H | I | A | V | A | G | T | R | G | F | D | Y | W | G | R | G | T | L | V | T | V | S | S |
| Antibody 8 | | | | | | | | | | | | | | | | | | | | | | | |
| Antibody 15 | | | | | | | | | | | | | | | | | | | | | | | |
| Antibody 16 | | | | | | | | | | | | | | | | | | | | | | | |
| Antibody 17 | | | | | | | | | | | | | | | | | | | | | | | |
| Antibody 33GL | | | | | | | | | | | | | | | | | | | | | | | |
| Antibody 6 | | | | | | | | | | | | | | | | | A | | | | | | |
| Antibody 24 | | | | | | | | | | | | | | | | | | | | | | P | |
| Antibody 25 | | | | | | | | | | | | | | | | | | | | | I | | |
| Antibody 9 | | | | | | | | | | | | | | | | | | | | | | | |
| Antibody 2 | | | | | | | | | | | | | | | | | | | | | | | |
| Antibody 3 | | | | | | | | | | | | | | | | | | | | | | | |
| Antibody 18 | | | | | | | | | | | | | | | | | | | | | | | |
| Antibody 19 | | | | | | | | | | | | | | | | | | | | | | | |
| Antibody 1 | | | | | | | | | | | | | | | | | | | | | | | |
| Antibody 4 | | | | | | | | | | | | | | | | | | P | | | | | |
| Antibody 5 | | | | | | | | | | | | | | | | | | | | | | | |
| Antibody 7 | | | | | | | | | | | | | | | | | | | | | | | |
| Antibody 23 | | | | | | | | | | | | | | | | | | | | | | | |

## Table 3h

| Kabat numbering | HCDR3 | | | | | | | | | | | | FW4 | | | | | | | | | | |
|---|---|---|---|---|---|---|---|---|---|---|---|---|---|---|---|---|---|---|---|---|---|---|---|
| | 95 | 96 | 97 | 98 | 99 | 100 | 100A | 100B | 100C | 100D | 101 | 102 | 103 | 104 | 105 | 106 | 107 | 108 | 109 | 110 | 111 | 112 | 113 |
| | H | I | A | V | A | G | T | R | G | F | D | Y | W | G | R | G | T | L | V | T | V | S | S |
| Antibody 11 | | | | | | | | | | | | | | | | | | | | A | | | |
| Antibody 12 | | | | | | | | | | | | | | | | | | | | | | | |
| Antibody 36 | | | | | | | | | | | | | | | | | | | | | | | |
| Antibody 13 | | | | | | | | | | | | | | | | | | | | | | | |
| Antibody 34 | | | | | | | | | | | | | | | | | | | | | | | |
| Antibody 14 | | | | | | | | | | | | | | | | | | | | | | | |
| Antibody 22 | | | | | | | | | | | | | | | | | | | | | | | |
| Antibody 26 | | | | | | | | | | | | | | | | | | | | | | | |
| Antibody 27 | S | T | Y | F | | S | | | | | | | | | | | | | | | | | |
| Antibody 28 | L | W | F | P | | I | | | | | | | | | | | | | | | | | |
| Antibody 29 | L | T | F | P | | I | | | | | | | | | | | | | | | | | |
| Antibody 30 | L | S | Y | P | | I | | | | | | | | | | | | | | | | | |
| Antibody 32 | L | F | Y | P | Y | I | | | | | | | | | | | | | | | | | |
| Antibody 20 | S | T | Y | F | | S | | | | | | | | | | | | | | | | | |
| Antibody 21 | L | T | F | P | | I | | | | | | | | | | | | | | | | | |
| Antibody 31 | L | S | Y | P | | I | | | | | | | | | | | | | | | | | |
| Antibody 10 | L | S | Y | P | | I | | | | | | | | | | | | | | | | | |

**Claims**

1. An isolated antibody or antigen-binding fragment thereof specific for human immunoglobulin E wherein the antibody or antigen-binding fragment thereof comprises a set of CDRs: HCDR1, HCDR2, HCDR3, LCDR1, LCDR2, and LCDR3 in which:

   HCDR1 has the amino acid sequence of SEQ ID NO: 279;
   HCDR2 has the amino acid sequence of SEQ ID NO: 280;
   HCDR3 has the amino acid sequence of SEQ ID NO: 281;
   LCDR1 has the amino acid sequence of SEQ ID NO: 284;
   LCDR2 has the amino acid sequence of SEQ ID NO: 285; and
   LCDR3 has the amino acid sequence of SEQ ID NO: 286.

2. An isolated nucleic acid molecule encoding an isolated antibody or antigen-binding fragment thereof according to claim 1.

3. A host cell transformed with a nucleic acid molecule according to claim 2.

4. A method of producing an isolated antibody or antigen-binding fragment thereof according to claim 1 comprising culturing the host cells according to claim 3 under conditions for production of the antibody or antigen-binding fragment thereof.

5. A pharmaceutical composition comprising an antibody or antigen-binding fragment thereof according to claim 1 and a pharmaceutically acceptable excipient.

6. The composition according to claim 5 for use as a medicament.

7. A composition according to claim 6 for use in treating one or more disorders associated with IgE selected from: an allergy, asthma and bronchitis.

8. A composition according to claim 6 for use in treating one or more disorders associated with IgE selected from: allergic rhinitis, allergic contact dermatitis, atopic dermatitis, anaphylactic reaction, food allergy, urticaria, inflammatory bowel disease, eosinophilic gastroenteritis, drug-induced rash, allergic opthalmopathy, allergic conjunctivitis, asthma bronchiale, airway hyperresponsiveness, cosmetic allergy, drug-induced allergy, drug-induced hypersensitivity syndrome, metal allergy, occupational hypersensitivity pneumonitis, chronic hypersensitivity pneumonitis, cold hypersensitivity, helminthic infection induced hypersensitivity, latex allergy and hay fever.

Figure 1

680
580
480
380
280
180
80
-20
-12
-11
-10
-9
-8
-7
-6
-5

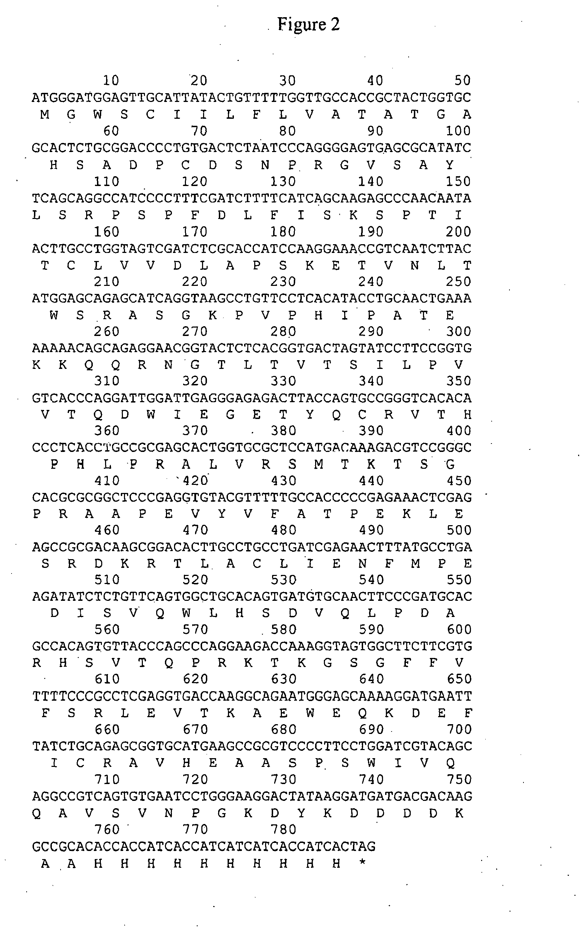

# Figure 2

```
        10         20         30         40         50
ATGGGATGGAGTTGCATTATACTGTTTTTGGTTGCCACCGCTACTGGTGC
 M  G  W  S  C  I  I  L  F  L  V  A  T  A  T  G  A
        60         70         80         90        100
GCACTCTGCGGACCCCTGTGACTCTAATCCCAGGGGAGTGAGCGCATATC
  H  S  A  D  P  C  D  S  N  P  R  G  V  S  A  Y
       110        120        130        140        150
TCAGCAGGCCATCCCCTTTCGATCTTTTCATCAGCAAGAGCCCAACAATA
L  S  R  P  S  P  F  D  L  F  I  S  K  S  P  T  I
       160        170        180        190        200
ACTTGCCTGGTAGTCGATCTCGCACCATCCAAGGAAACCGTCAATCTTAC
 T  C  L  V  V  D  L  A  P  S  K  E  T  V  N  L  T
       210        220        230        240        250
ATGGAGCAGAGCATCAGGTAAGCCTGTTCCTCACATACCTGCAACTGAAA
  W  S  R  A  S  G  K  P  V  P  H  I  P  A  T  E
       260        270        280        290        300
AAAAACAGCAGAGGAACGGTACTCTCACGGTGACTAGTATCCTTCCGGTG
K  K  Q  Q  R  N  G  T  L  T  V  T  S  I  L  P  V
       310        320        330        340        350
GTCACCCAGGATTGGATTGAGGGAGAGACTTACCAGTGCCGGGTCACACA
 V  T  Q  D  W  I  E  G  E  T  Y  Q  C  R  V  T  H
       360        370        380        390        400
CCCTCACCTGCCGCGAGCACTGGTGCGCTCCATGACAAAGACGTCCGGGC
  P  H  L  P  R  A  L  V  R  S  M  T  K  T  S  G
       410        420        430        440        450
CACGCGCGGCTCCCGAGGTGTACGTTTTTGCCACCCCCGAGAAACTCGAG
P  R  A  A  P  E  V  Y  V  F  A  T  P  E  K  L  E
       460        470        480        490        500
AGCCGCGACAAGCGGACACTTGCCTGCCTGATCGAGAACTTTATGCCTGA
 S  R  D  K  R  T  L  A  C  L  I  E  N  F  M  P  E
       510        520        530        540        550
AGATATCTCTGTTCAGTGGCTGCACAGTGATGTGCAACTTCCCGATGCAC
  D  I  S  V  Q  W  L  H  S  D  V  Q  L  P  D  A
       560        570        580        590        600
GCCACAGTGTTACCCAGCCCAGGAAGACCAAAGGTAGTGGCTTCTTCGTG
R  H  S  V  T  Q  P  R  K  T  K  G  S  G  F  F  V
       610        620        630        640        650
TTTTCCCGCCTCGAGGTGACCAAGGCAGAATGGGAGCAAAAGGATGAATT
 F  S  R  L  E  V  T  K  A  E  W  E  Q  K  D  E  F
       660        670        680        690        700
TATCTGCAGAGCGGTGCATGAAGCCGCGTCCCCTTCCTGGATCGTACAGC
  I  C  R  A  V  H  E  A  A  S  P  S  W  I  V  Q
       710        720        730        740        750
AGGCCGTCAGTGTGAATCCTGGGAAGGACTATAAGGATGATGACGACAAG
Q  A  V  S  V  N  P  G  K  D  Y  K  D  D  D  D  K
       760        770        780
GCCGCACACCACCATCACCATCATCATCACCATCACTAG
 A  A  H  H  H  H  H  H  H  H  H  H  *
```

# Figure 3

```
        10        20        30        40        50
ATGGGATGGAGCTGTATCATCCTCTTCTTGGTAGCAACAGCTACAGGCGC
 M  G  W  S  C  I  I  L  F  L  V  A  T  A  T  G  A

        60        70        80        90       100
GCACTCCGAGGTGCAGCTGGTGGAGTCTGGGGGAGGCTTGGTACAGCCTG
  H  S  E  V  Q  L  V  E  S  G  G  G  L  V  Q  P

       110       120       130       140       150
GGAGGTCCCTGAGACTCTCCTGTGCAGCCTCTGGAGTCACCTTTAGCAGC
G  R  S  L  R  L  S  C  A  A  S  G  V  T  F  S  S

       160       170       180       190       200
CATGCCATGACCTGGGTCCGCCAGGCTCCAGGGAAGGGGCTGGAATGGGT
 H  A  M  T  W  V  R  Q  A  P  G  K  G  L  E  W  V

       210       220       230       240       250
CTCAGGTATCAGTGGTAGTGGTGGTGACACATACCACGCAGACTCCGTGA
  S  G  I  S  G  S  G  G  D  T  Y  H  A  D  S  V

       260       270       280       290       300
AGGGCCGGTTCACCATCTCCAGGGACAATTCCAAGAACACGGTGTATCTG
K  G  R  F  T  I  S  R  D  N  S  K  N  T  V  Y  L

       310       320       330       340       350
CAAATGAACAGCCTGCGAGCCGAGGACACGGCCATATATTACTGTGCGAT
 Q  M  N  S  L  R  A  E  D  T  A  I  Y  Y  C  A  I

       360       370       380       390       400
TTTAGGAGTACTAAATGGTTTTGATATCTGGGGCCAAGGGACAATGGTCA
  L  G  V  L  N  G  F  D  I  W  G  Q  G  T  M  V

       410       420       430       440       450
CCGTCTCCTCAGCCTCCATACAGAGCCCCTTCGTCTTCCCCTTGATCCCC
T  V  S  S  A  S  I  Q  S  P  F  V  F  P  L  I  P

       460       470       480       490       500
TGCTGCAAACACATTGCCTCCAATGCCACCTCCGTGACCCTGGGCTGCCT
 C  C  K  H  I  A  S  N  A  T  S  V  T  L  G  C  L

       510       520       530       540       550
GGCCACGGGCTACTTCCCGGAGCCGGTGATGGTGACCTGGGACGCAGGCT
  A  T  G  Y  F  P  E  P  V  M  V  T  W  D  A  G

       560       570       580       590       600
CCCTCAACAGAAGCACTATGACCTTACCAGCCACCACCTTCACGCCCTCC
S  L  N  R  S  T  M  T  L  P  A  T  T  F  T  P  S

       610       620       630       640       650
GGTCACTATGCCACCATCAGCTTGCTGACCGTCTCGGGTGCGTGGGCCAA
 G  H  Y  A  T  I  S  L  L  T  V  S  G  A  W  A  K
```

```
        660       670       680       690       700
GGAGACGTTCACCTGCCATGTGGTGCACACTCCATCGTCCGCAGACAAAG
 E  T  F  T  C  H  V  V  H  T  P  S  S  A  D  K

        710       720       730       740       750
AGGTCAACAAAACCTTTGGCGTCTGCTCCAGGAACTTCACCCCACCTACC
E  V  N  K  T  F  G  V  C  S  R  N  F  T  P  P  T

        760       770       780       790       800
GTGAAGATCTTACAGTCATCCTGCGATGACGACGGGCACTTTCCCCCGAC
 V  K  I  L  Q  S  S  C  D  D  D  G  H  F  P  P  T

        810       820       830       840       850
CATCCAGCTCCTGTGCCTCATCTCCGGGTACACCCCAGGGGCCATCAATG
 I  Q  L  L  C  L  I  S  G  Y  T  P  G  A  I  N

        860       870       880       890       900
TCACCTGGCTGGAGAACGGGCAGGTCATGAAAGTGAACTCGCCCACCCCT
V  T  W  L  E  N  G  Q  V  M  K  V  N  S  P  T  P

        910       920       930       940       950
CCTGCCACGCAGGAGGGTGAGCTGGCCTCCACACAAAGTGAGTTCACCCT
 P  A  T  Q  E  G  E  L  A  S  T  Q  S  E  F  T  L

        960       970       980       990      1000
CGCCCAGAAGCACTGGCTGTCGGACCGCACTTACACCTGCCAGGTCACCT
 A  Q  K  H  W  L  S  D  R  T  Y  T  C  Q  V  T

       1010      1020      1030      1040      1050
ATCAAGGTACCACCTATAACGACAGCACCAAGAAGTGTGCAGATTCCAAC
Y  Q  G  T  T  Y  N  D  S  T  K  K  C  A  D  S  N

       1060      1070      1080      1090      1100
CCGAGAGGGGTGAGTGCCTACCTAAGCCGGCCCAGCCCGTTTGACCTGTT
 P  R  G  V  S  A  Y  L  S  R  P  S  P  F  D  L  F

       1110      1120      1130      1140      1150
CATCAGCAAGTCGCCCACGATCACCTGTCTGGTGGTGGACCTGGCACCCA
 I  S  K  S  P  T  I  T  C  L  V  V  D  L  A  P

       1160      1170      1180      1190      1200
GCAAGGAGACCGTGAACCTGACCTGGTCCCGGGCCAGTGGGAAGCCTGTG
S  K  E  T  V  N  L  T  W  S  R  A  S  G  K  P  V

       1210      1220      1230      1240      1250
CCCCACATCCCCGCAACGGAGAAGAAGCAGCAGCGCAATGGCACGTTAAC
 P  H  I  P  A  T  E  K  K  Q  Q  R  N  G  T  L  T

       1260      1270      1280      1290      1300
CGTTACGTCCATCCTGCCGGTGGTCACCCAAGACTGGATCGAGGGGGAGA
 V  T  S  I  L  P  V  V  T  Q  D  W  I  E  G  E

       1310      1320      1330      1340      1350
CCTACCAGTGCAGGGTGACCCACCCCCACCTCCCCAGGGCCCTCGTGCGG
T  Y  Q  C  R  V  T  H  P  H  L  P  R  A  L  V  R
```

```
      1360       1370       1380       1390       1400
TCCATGACCAAGACCAGCGGCCCGCGTGCTGCCCCGGAAGTCTATGTGTT
 S  M  T  K  T  S  G  P  R  A  A  P  E  V  Y  V  F

      1410       1420       1430       1440       1450
TGCAACGCCAGAGAAGCTAGAGAGCCGGGACAAGCGCACCCTCGCCTGCC
  A  T  P  E  K  L  E  S  R  D  K  R  T  L  A  C

      1460       1470       1480       1490       1500
TGATCCAGAACTTCATGCCTGAGGACATATCGGTGCAGTGGCTGCACAGC
L  I  Q  N  F  M  P  E  D  I  S  V  Q  W  L  H  S

      1510       1520       1530       1540       1550
GACGTGCAGCTCCCGGACGCCCGGCACAGCGTGACGCAGCCCCGCAAGAC
 D  V  Q  L  P  D  A  R  H  S  V  T  Q  P  R  K  T

      1560       1570       1580       1590       1600
CAAGGGCTCCGGCTTCTTCGTCTTCAGCCGCCTGGAGGTGACCAAGGCCG
  K  G  S  G  F  F  V  F  S  R  L  E  V  T  K  A

      1610       1620       1630       1640       1650
AATGGGAGCAGAAAGACGAGTTCATCTGCCGTGCAGTCCATGAGGCAGCG
E  W  E  Q  K  D  E  F  I  C  R  A  V  H  E  A  A

      1660       1670       1680       1690       1700
AGCCCCTCATGGATCGTCCAGCAAGCGGTGTCTGTAAATCCCGGTAAATGA
 S  P  S  W  I  V  Q  Q  A  V  S  V  N  P  G  K  *
```

Figure 4

```
        10         20         30         40         50
ATGGGATGGAGCTGTATCATCCTCTTCTTGGTAGCAACAGCTACAGGCGC
 M  G  W  S  C  I  I  L  F  L  V  A  T  A  T  G  A

        60         70         80         90        100
GCACTCCGAGGTGCAGCTGGTGGAGTCTGGGGGAGGCTTGGTACAGCCTG
  H  S  E  V  Q  L  V  E  S  G  G  G  L  V  Q  P

       110        120        130        140        150
GGAGGTCCCTGAGACTCTCCTGTGCAGCCTCTGGAGTCACCTTTAGCAGC
G  R  S  L  R  L  S  C  A  A  S  G  V  T  F  S  S

       160        170        180        190        200
CATGCCATGACCTGGGTCCGCCAGGCTCCAGGGAAGGGGCTGGAATGGGT
 H  A  M  T  W  V  R  Q  A  P  G  K  G  L  E  W  V

       210        220        230        240        250
CTCAGGTATCAGTGGTAGTGGTGGTGACACATACCACGCAGACTCCGTGA
  S  G  I  S  G  S  G  G  D  T  Y  H  A  D  S  V

       260        270        280        290        300
AGGGCCGGTTCACCATCTCCAGGGACAATTCCAAGAACACGGTGTATCTG
K  G  R  F  T  I  S  R  D  N  S  K  N  T  V  Y  L

       310        320        330        340        350
CAAATGAACAGCCTGCGAGCCGAGGACACGGCCATATATTACTGTGCGAT
 Q  M  N  S  L  R  A  E  D  T  A  I  Y  Y  C  A  I

       360        370        380        390        400
TTTAGGAGTACTAAATGGTTTTGATATCTGGGGCCAAGGGACAATGGTCA
  L  G  V  L  N  G  F  D  I  W  G  Q  G  T  M  V

       410        420        430        440        450
CCGTCTCCTCAGCCTCCATACAGAGCCCCTTCGTCTTCCCCTTGATCCCC
T  V  S  S  A  S  I  Q  S  P  F  V  F  P  L  I  P

       460        470        480        490        500
TGCTGCAAACACATTGCCTCCAATGCCACCTCCGTGACCCTGGGCTGCCT
 C  C  K  H  I  A  S  N  A  T  S  V  T  L  G  C  L

       510        520        530        540        550
GGCCACGGGCTACTTCCCGGAGCCGGTGATGGTGACCTGGGACGCAGGCT
  A  T  G  Y  F  P  E  P  V  M  V  T  W  D  A  G

       560        570        580        590        600
CCCTCAACAGAAGCACTATGACCTTACCAGCCACCACCTTCACGCCCTCC
S  L  N  R  S  T  M  T  L  P  A  T  T  F  T  P  S

       610        620        630        640        650
GGTCACTATGCCACCATCAGCTTGCTGACCGTCTCGGGTGCGTGGGCCAA
 G  H  Y  A  T  I  S  L  L  T  V  S  G  A  W  A  K
```

```
        660       670       680       690       700
GGAGATGTTCACCTGCCATGTGGTGCACACTCCATCGTCCGCAGACAAAG
  E  M  F  T  C  H  V  V  H  T  P  S  S  A  D  K

        710       720       730       740       750
AGGTCAACAAAACCTTTGGCGTCTGCTCCAGGAACTTCACCCCACCTACC
E  V  N  K  T  F  G  V  C  S  R  N  F  T  P  P  T

        760       770       780       790       800
GTGAAGATCTTACAGTCATCCTGCGATGACGACGGGCACTTTCCCCCGAC
 V  K  I  L  Q  S  S  C  D  D  D  G  H  F  P  P  T

        810       820       830       840       850
CATCCAGCTCCTGTGCCTCATCTCCGGGTACACCCCAGGGGCCATCAATG
  I  Q  L  L  C  L  I  S  G  Y  T  P  G  A  I  N

        860       870       880       890       900
TCACCTGGCTGGAGAACGGGCAGGTCATGAAAGTGAACTCGCCCACCCCT
V  T  W  L  E  N  G  Q  V  M  K  V  N  S  P  T  P

        910       920       930       940       950
CCTGCCACGCAGGAGGGTGAGCTGGCCTCCACACAAAGTGAGTTCACCCT
 P  A  T  Q  E  G  E  L  A  S  T  Q  S  E  F  T  L

        960       970       980       990      1000
CGCCCAGAAGCACTGGCTGTCGGACCGCACTTACACCTGCCAGGTCACCT
  A  Q  K  H  W  L  S  D  R  T  Y  T  C  Q  V  T

       1010      1020      1030      1040      1050
ATCAAGGTACCACCTATAACGACAGCACCAAGAAGTGTGCAGATTCCAAC
Y  Q  G  T  T  Y  N  D  S  T  K  K  C  A  D  S  N

       1060      1070      1080      1090      1100
CCGAGAGGGGTGAGTGCCTACCTAAGCCGGCCCAGCCCGTTTGACCTGTT
 P  R  G  V  S  A  Y  L  S  R  P  S  P  F  D  L  F

       1110      1120      1130      1140      1150
CATCAGCAAGTCGCCCACGATCACCTGTCTGGTGGTGGACCTGGCACCCA
  I  S  K  S  P  T  I  T  C  L  V  V  D  L  A  P

       1160      1170      1180      1190      1200
GCAAGGAGACCGTGAACCTGACCTGGTCCCGGGCCAGTGGGAAGCCTGTG
S  K  E  T  V  N  L  T  W  S  R  A  S  G  K  P  V

       1210      1220      1230      1240      1250
CCCCACATCCCCGCAACGGAGAAGAAGCAGCAGCGCAATGGCACGTTAAC
 P  H  I  P  A  T  E  K  K  Q  Q  R  N  G  T  L  T

       1260      1270      1280      1290      1300
CGTTACGTCCATCCTGCCGGTGGTCACCCAAGACTGGATCGAGGGGGAGA
  V  T  S  I  L  P  V  V  T  Q  D  W  I  E  G  E

       1310      1320      1330      1340      1350
CCTACCAGTGCAGGGTGACCCACCCCCACCTCCCCAGGGCCCTCGTGCGG
T  Y  Q  C  R  V  T  H  P  H  L  P  R  A  L  V  R
```

```
       1360        1370        1380        1390        1400
TCCATGACCAAGACCAGCGGCCCGCGTGCTGCCCCGGAAGTCTATGTGTT
 S  M  T  K  T  S  G  P  R  A  A  P  E  V  Y  V  F

       1410        1420        1430        1440        1450
TGCAACGCCAGAGAAGCTAGAGAGCCGGGACAAGCGCACCCTCGCCTGCC
  A  T  P  E  K  L  E  S  R  D  K  R  T  L  A  C

       1460        1470        1480        1490        1500
TGATCGAGAACTTCATGCCTGAGGACATATCGGTGCAGTGGCTGCACAGC
L  I  E  N  F  M  P  E  D  I  S  V  Q  W  L  H  S

       1510        1520        1530        1540        1550
GACGTGCAGCTCCCGGACGCCCGGCACAGCGTGACGCAGCCCCGCAAGAC
 D  V  Q  L  P  D  A  R  H  S  V  T  Q  P  R  K  T

       1560        1570        1580        1590        1600
CAAGGGCTCCGGCTTCTTCGTCTTCAGCCGCCTGGAGGTGACCAAGGCCG
  K  G  S  G  F  F  V  F  S  R  L  E  V  T  K  A

       1610        1620        1630        1640        1650
AATGGGAGCAGAAAGACGAGTTCATCTGCCGTGCAGTCCATGAGGCAGCG
E  W  E  Q  K  D  E  F  I  C  R  A  V  H  E  A  A

       1660        1670        1680        1690        1700
AGCCCCTCATGGATCGTCCAGCAAGCGGTGTCTGTAAATCCCGGTAAATG A
 S  P  S  W  I  V  Q  Q  A  V  S  V  N  P  G  K  *
__________________________________________________a_
```

# Figure 5

```
        10        20        30        40        50
ATGGGTTGGAGTTGTATAATTCTCTTTCTGGTGGCTACCGCTACCGGTGT
 M  G  W  S  C  I  I  L  F  L  V  A  T  A  T  G  V

        60        70        80        90       100
GCACTCCCAGTCAGCTCTGACGCAACCAGCTTCCGTTTCAGGGAGCCCAG
  H  S  Q  S  A  L  T  Q  P  A  S  V  S  G  S  P

       110       120       130       140       150
GGCAGAGTATAACCATCAGTTGCACTGGCACTAGCTCCGACGTTGGCGGA
G  Q  S  I  T  I  S  C  T  G  T  S  S  D  V  G  G

       160       170       180       190       200
TACAAGTACGTATCTTGGTATCAACAGCACCCCGGAAAAGCTCCTAAGCT
 Y  K  Y  V  S  W  Y  Q  Q  H  P  G  K  A  P  K  L

       210       220       230       240       250
GATGATTTTCGAGGTTTCCAACAGACCCAGCGGTGTACCTAATCGGTTCT
  M  I  F  E  V  S  N  R  P  S  G  V  P  N  R  F

       260       270       280       290       300
CTGGCTCTAAATCCGGGAACACTGCCTCACTCACCATCAGCGGACTGCAG
S  G  S  K  S  G  N  T  A  S  L  T  I  S  G  L  Q

       310       320       330       340       350
GTGGAAGACGAGGCGGACTATTATTGCAGCTCTCTCACCAGACGCGTTAC
 V  E  D  E  A  D  Y  Y  C  S  S  L  T  R  R  V  T

       360       370       380       390       400
CGTCATTTTTGGCGGAGGCACTAAGCTGACCGTTCTCGGCCAACCTAAAG
  V  I  F  G  G  G  T  K  L  T  V  L  G  Q  P  K

       410       420       430       440       450
CCGCCCCATCTGTGACCCTTTTTCCTCCCAGCAGCGAGGAACTGCAGGCC
A  A  P  S  V  T  L  F  P  P  S  S  E  E  L  Q  A

       460       470       480       490       500
AATAAGGCCACTCTCGTGTGCCTCATGTCAGACTTTTACCCAGGGATCCT
 N  K  A  T  L  V  C  L  M  S  D  F  Y  P  G  I  L

       510       520       530       540       550
GACCGTGACCTGGAAGGCCGACGGAACCCCCATCACACAGGGCGTGGAAA
  T  V  T  W  K  A  D  G  T  P  I  T  Q  G  V  E

       560       570       580       590       600
TGACCACGCCAAGTAAGCAGTCTAACAACAAATACGCCGCATCTAGCTAC
M  T  T  P  S  K  Q  S  N  N  K  Y  A  A  S  S  Y

       610       620       630       640       650
TTGAGCCTGACCCCAGAGCAGTGGCGGAGTCACAATAGCTACAGCTGCCA
 L  S  L  T  P  E  Q  W  R  S  H  N  S  Y  S  C  Q
```

```
          660       670       680       690       700
AGTGATGCACGAGGGATCAATCGTGGAGAAGACTGTTGCTCCAGCCGAGT
  V  M  H  E  G  S  I  V  E  K  T  V  A  P  A  E

GCTCCTAA
C  S  *
```

# Figure 6

```
        10        20        30        40        50
ATGGGTTGGAGTTGTATAATTCTCTTTCTGGTGGCTACCGCTACCGGTGT
 M  G  W  S  C  I  I  L  F  L  V  A  T  A  T  G  V

        60        70        80        90       100
GCACTCCCAGTCAGCTCTGACGCAACCAGCTTCCGTTTCAGGGAGCCCAG
  H  S  Q  S  A  L  T  Q  P  A  S  V  S  G  S  P

       110       120       130       140       150
GGCAGAGTATAACCATCAGTTGCACTGGCACTAGCTCCGACGTTGGCGGA
G  Q  S  I  T  I  S  C  T  G  T  S  S  D  V  G  G

       160       170       180       190       200
TACAAGTACGTATCTTGGTATCAACAGCACCCCGGAAAAGCTCCTAAGCT
 Y  K  Y  V  S  W  Y  Q  Q  H  P  G  K  A  P  K  L

       210       220       230       240       250
GATGATTTTCGAGGTTTCCAACAGACCCAGCGGTGTACCTAATCGGTTCT
  M  I  F  E  V  S  N  R  P  S  G  V  P  N  R  F

       260       270       280       290       300
CTGGCTCTAAATCCGGGAACACTGCCTCACTCACCATCAGCGGACTGCAG
S  G  S  K  S  G  N  T  A  S  L  T  I  S  G  L  Q

       310       320       330       340       350
GTGGAAGACGAGGCGGACTATTATTGCAGCTCTCTCACCAGACGCGTTAC
 V  E  D  E  A  D  Y  Y  C  S  S  L  T  R  R  V  T

       360       370       380       390       400
CGTCATTTTTGGCGGGGGGACTAAGCTGACCGTTCTCGGCCAACCTAAAG
  V  I  F  G  G  G  T  K  L  T  V  L  G  Q  P  K

       410       420       430       440       450
CCGCCCCCTCTGTGACCCTTTTTCCCCCTAGCAGCGAGGAACTGCAGGCC
A  A  P  S  V  T  L  F  P  P  S  S  E  E  L  Q  A

       460       470       480       490       500
AATAAGGCCACTCTCGTGTGCCTCATCTCAGACTTTTACCCAGGGGCCGT
 N  K  A  T  L  V  C  L  I  S  D  F  Y  P  G  A  V

       510       520       530       540       550
GGAGGTGGCCTGGAAGGCCGACGGAAGCGCCGTCAACGCGGGCGTGGAAA
  E  V  A  W  K  A  D  G  S  A  V  N  A  G  V  E

       560       570       580       590       600
CGACCAAGCCAAGTAAGCAGTCTAACAACAAATACGCCGCATCTAGCTAC
T  T  K  P  S  K  Q  S  N  N  K  Y  A  A  S  S  Y

       610       620       630       640       650
TTGAGCCTGACCTCAGACCAGTGGAAGAGTCACAAGAGCTACAGCTGCCA
 L  S  L  T  S  D  Q  W  K  S  H  K  S  Y  S  C  Q
```

```
        660       670       680       690       700
AGTGACACACGAGGGATCAACCGTGGAGAAGACTGTTGCTCCAACCGAGT
 V  T  H  E  G  S  T  V  E  K  T  V  A  P  T  E

GCTCCTAA
C  S  *
```

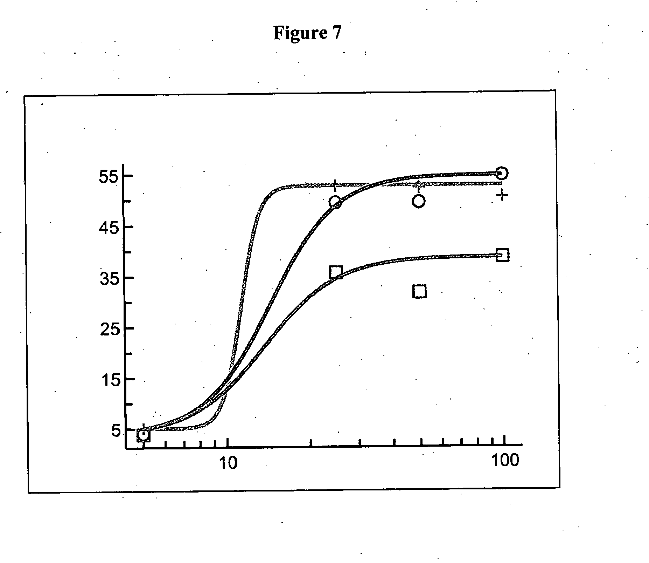

**Figure 7**

55
45
35
25
15
5
10
100

Figure 8

Serum Concentration (nM)
Time (day)
Group 1, E85-IgG1
Group 2, E85-IgG2
Group 3, E48
0
14
28
42
56
70
84

# Figure 9

$10^4$
$10^3$
$10^2$
$10^1$
$10^0$
$10^{-1}$
Free IgE (ng/mL)
0
14
28
42
56
70
84
Time (day)
Group 1, E85-IgG1
Group 2, E85-IgG2
Group 3, E48

# Figure 10

100
80
60
40
20
0
-20
-40
-60
-80
-100
35000
30000
25000
20000
15000
10000
5000
0
1
30
100
100
100
0
6
24
144
192
360.5
504
528
672.5
816
1344
1680

Europäisches Patentamt
European Patent Office
Office européen des brevets

# EUROPEAN SEARCH REPORT

Application Number
EP 13 15 1217

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 00/50460 A (SMITHKLINE BEECHAM BIOLOG [BE]; PEPTIDE THERAPEUTICS LTD [GB]; DYSON M) 31 August 2000 (2000-08-31)<br>* examples 1-11 *<br>* figures 17,18 *<br>----- | 1-8 | INV.<br>C07K16/42<br>A61K39/395<br>A61P37/08 |
| A | WO 2005/075504 A (TANOX INC [US]; SINGH SANJAYA [US]; HUANG DANYANG [US]; FUNG SEK CHUNG) 18 August 2005 (2005-08-18)<br>* examples 1-11 *<br>----- | 1-8 | |
| A | MCDONNELL J M ET AL: "The structure of the IgE Cepsilon2 domain and its role in stabilizing the complex with its high-affinity receptor FcepsilonRIalpha.", NATURE STRUCTURAL BIOLOGY MAY 2001, vol. 8, no. 5, May 2001 (2001-05), pages 437-441, XP002485409, ISSN: 1072-8368<br>* the whole document *<br>----- | 1-8 | |
| | | | TECHNICAL FIELDS SEARCHED (IPC) |
| | | | C07K<br>A61K<br>A61P |

The present search report has been drawn up for all claims

| Place of search | Date of completion of the search | Examiner |
|---|---|---|
| The Hague | 3 April 2013 | Bumb, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

2

# ANNEX TO THE EUROPEAN SEARCH REPORT ON EUROPEAN PATENT APPLICATION NO. EP 13 15 1217

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2013

| Patent document cited in search report | Publication date | Patent family member(s) | Publication date |
|---|---|---|---|
| WO 0050460 A | 31-08-2000 | AR 029336 A1 | 25-06-2003 |
| | | AU 2672700 A | 14-09-2000 |
| | | BR 0008963 A | 27-11-2001 |
| | | CA 2363637 A1 | 31-08-2000 |
| | | CN 1348466 A | 08-05-2002 |
| | | CO 5210906 A1 | 30-10-2002 |
| | | CZ 20013082 A3 | 13-02-2002 |
| | | EP 1155037 A1 | 21-11-2001 |
| | | HU 0200049 A2 | 29-05-2002 |
| | | JP 2004538238 A | 24-12-2004 |
| | | MX PA01008613 A | 24-06-2003 |
| | | NO 20014130 A | 13-09-2001 |
| | | NZ 513679 A | 28-09-2001 |
| | | PL 350993 A1 | 24-02-2003 |
| | | TR 200102506 T2 | 21-06-2002 |
| | | WO 0050460 A1 | 31-08-2000 |
| WO 2005075504 A | 18-08-2005 | AU 2004315197 A1 | 18-08-2005 |
| | | AU 2009202408 A1 | 09-07-2009 |
| | | CA 2552999 A1 | 18-08-2005 |
| | | CN 102702359 A | 03-10-2012 |
| | | EP 1718669 A1 | 08-11-2006 |
| | | JP 2008507474 A | 13-03-2008 |
| | | KR 20070008578 A | 17-01-2007 |
| | | KR 20120056297 A | 01-06-2012 |
| | | SG 149892 A1 | 27-02-2009 |
| | | SG 183683 A1 | 27-09-2012 |
| | | WO 2005075504 A1 | 18-08-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9304173 A
- WO 9704807 A
- WO 0034784 A
- EP 184187 A
- GB 2188638 A
- EP 239400 A
- EP 0120694 A
- EP 0125023 A
- WO 9201047 A
- US 5969108 A
- US 5565332 A
- US 5733743 A
- US 5858657 A
- US 5871907 A
- US 5872215 A
- US 5885793 A
- US 5962255 A
- US 6140471 A
- US 6172197 B
- US 6225447 B
- US 6291650 B
- US 6492160 B
- US 6521404 B
- WO 9109967 A
- US 5585089 A
- EP 592106 A
- WO 9317105 A
- WO 2004006955 A
- US 9209965 W
- WO 9413804 A
- US 4275149 A, Litman
- US 4318980 A, Boguslaski
- US 4424200 A, Crockford
- US 4479930 A, Hnatowich
- US 5185243 A, Ullman
- US 5814468 A


### Non-patent literature cited in the description


- **BURT et al.** *Eur. J. Immun,* 1987, vol. 17, 437-440
- **HELM et al.** *Nature,* 1988, vol. 331, 180-183
- **HELM et al.** *Proc. Natl. Acad. Sci.,* 1989, vol. 86, 9465-9469
- **VERCELLI et al.** *Nature,* 1989, vol. 338, 649-651
- **NIO et al.** *Peptide Chemistry,* 1990, 203-208
- **BURT et al.** *Molec. Immun.,* 1987, vol. 24, 379-389
- **ROBERTSON et al.** *Molec. Immun.,* 1988, vol. 25, 103-113
- **BANIYASH et al.** *Molec. Immun.,* 1988, vol. 25, 705-711
- **VAUGHAN et al.** *Nature Biotechnology,* 1996, vol. 14, 309-314
- **HUTCHINGS.** Antibody Engineering. Springer Laboratory Manuals, 2001, 93
- **OSBOURN.** *Immunotechnology,* 1996, vol. 2, 181-196
- **BANNISTER et al.** *Biotechnology and bioengineering,* 2006, vol. 94, 931-937
- **PERSIC, L. et al.** *Gene,* 1997, vol. 187, 9-18
- **MACH et al.** *Anal. Biochem.,* 1992, vol. 200 (1), 20-26
- **CLACKSON ; LOWMAN.** Phage Display A Practical Approach. Oxford University Press, 2004
- **THOMPSON.** *Journal of Molecular Biology,* 1996, vol. 256, 77-88
- **IKEYAMA.** *Molecular Immunology,* 1986, vol. 23 (2), 159-167
- **OSBOUM, JK et al.** *Immunotechnology,* 1996, vol. 2 (3), 181-96
- **HANES et al.** *Methods in Enzymology,* 2000, vol. 328, 404
- **TOMLINSON.** *Journal of Molecular biology,* 1997, vol. 224, 487-499
- **KARLSSON et al.** *Journal of Immunological Methods,* 1991, vol. 145 (1-2), 229-240
- **WURZBURG.** Structure of the Human IgE-Fc C3-C4 Reveals Conformational Flexibility. *Antibody Effector Domains,* 2000
- **IKEYAM.** *Mol Immunol,* 1986, vol. 23, 159-67
- **PERSIC et al.** *Gene,* 1997, vol. 187, 9-18
- **HAAN ; MAGGOS.** *BioCentury,* 2004, vol. 12 (5), A 1-A6
- **KOIDE et al.** *Journal of Molecular Biology,* 1998, vol. 284, 1141-1151
- **NYGREN et al.** *Current Opinion in Structural Biology,* 1997, vol. 7, 463-469
- **WESS, L.** *BioCentury, The Bernstein Report on BioBusiness,* 2004, vol. 12 (42), A1-A7

- **KABAT, E.A. et al.** Sequences of Proteins of Immunological Interest. US Department of Health and Human Services, 1987
- **KABAT, E.A. et al.** Sequences of Proteins of Immunological Interest. US Department of Health and Human Services, Public Service, NIH, 1991
- **SEGAL et al.** *PNAS,* 1974, vol. 71, 4298-4302
- **AMIT et al.** *Science,* 1986, vol. 233, 747-753
- **CHOTHIA et al.** *J. Mol. Biol.,* 1987, vol. 196, 901-917
- **CHOTHIA et al.** *Nature,* 1989, vol. 342, 877-883
- **CATON et al.** *J. Immunol.,* 1990, vol. 144, 1965-1968
- **SHARON et al.** *PNAS,* 1990, vol. 87, 4814-4817
- **SHARON et al.** *J. Immunol.,* 1990, vol. 144, 4863-4869
- **KABAT et al.** *J. Immunol.,* 1991, vol. 147, 1709-1719
- **HOLLIGER ; HUDSON.** *Nature Biotechnology,* 2005, vol. 23 (9), 1126-1136
- **KONTERMANN, R ; DUBEL, S.** Antibody Engineering. Springer-Verlag, 2001
- **MENDEZ, M. et al.** *Nature Genet,* 1997, vol. 15 (2), 146-156
- **KNAPPIK et al.** *J. Mol. Biol.,* 2000, vol. 296, 57-86
- **KREBS et al.** *Journal of Immunological Methods,* 2001, vol. 254, 67-84
- **WARD, E.S. et al.** *Nature,* 1989, vol. 341, 544-546
- **MCCAFFERTY et al.** *Nature,* 1990, vol. 348, 552-554
- **HOLT et al.** *Trends in Biotechnology,* 2003, vol. 21, 484-490
- **BIRD et al.** *Science,* 1988, vol. 242, 423-426
- **HUSTON et al.** *PNAS USA,* 1988, vol. 85, 5879-5883
- **HOLLIGER, P. et al.** *Proc. Natl. Acad. Sci. USA,* 1993, vol. 90, 6444-6448
- **REITER, Y. et al.** *Nature Biotech,* 1996, vol. 14, 1239-1245
- **HU, S. et al.** *Cancer Res.,* 1996, vol. 56, 3055-3061
- **HOLLIGER ; BOHLEN.** *Cancer and metastasis rev.,* 1999, vol. 18, 411-419
- **HOLLIGER, P. ; WINTER G.** *Current Opinion Biotechnol,* 1993, vol. 4, 446-449
- **GLENNIE M J et al.** *J. Immunol.,* 1987, vol. 139, 2367-2375
- **REPP R. et al.** *J. Hemat.,* 1995, 377-382
- **STAERZ U. D. ; BEVAN M. J.** *PNAS,* 1986, 83
- **SURESH M. R. et al.** *Method Enzymol.,* 1986, vol. 121, 210-228
- **MERCHAND et al.** *Nature Biotech.,* 1998, vol. 16, 677-681
- **RIDGEWAY, J. B. B. et al.** *Protein Eng.,* 1996, vol. 9, 616-621
- **HARLOW ; LANE.** Antibodies: A Laboratory Manual. Cold Spring Harbor Laboratory, 1988, 726
- **KÖHLER ; MILSTEIN.** *Nature,* 1975, vol. 256, 495-497
- Multivariate data analysis in chemistry. **WOLD et al.** Chemometrics -Mathematics and Statistics in Chemistry. D. Reidel Publishing Company, 1984
- **NORMAN et al.** Applied Regression Analysis. Wiley-Interscience, April 1998
- **KANDEL, ABRAHAM ; BACKER, ERIC.** Computer-Assisted Reasoning in Cluster Analysis. Prentice Hall PTR, 11 May 1995
- **KRZANOWSKI, WOJTEK.** Principles of Multivariate Analysis: A User's Perspective. Oxford University Press, December 2000
- **WITTEN, IAN H. ; FRANK, EIBE.** Data Mining: Practical Machine Learning Tools and Techniques with Java Implementations. Morgan Kaufmann, 11 October 1999
- **CHRISTOPHER C. HOLMES ; BANI K. MALLICK ; ADRIAN F. M. SMITH.** Bayesian Methods for Nonlinear Classification and Regression. John Wiley & Sons, July 2002
- **GHOSE, ARUP K. ; VISWANADHAN, VELLARKAD N.** *Combinatorial Library Design and Evaluation Principles, Software, Tools, and Applications in Drug Discovery,* ISBN 0-8247-0487-8
- **CHOTHIA C. et al.** *Journal Molecular Biology,* 1992, vol. 227, 799-817
- **AL-LAZIKANI et al.** *Journal Molecular Biology,* 1997, vol. 273 (4), 927-948
- **CHOTHIA et al.** *Science,* 1986, vol. 223, 755-758
- **WHITELEGG, N.R.U. ; REES, A.R.** *Prot. Eng.,* 2000, vol. 12, 815-824
- **GUEX, N. ; PEITSCH, M.C.** *Electrophoresis,* 1997, vol. 18, 2714-2723
- **ALTSCHUL et al.** *J. Mol. Biol.,* 1990, vol. 215, 405-410
- **PEARSON ; LIPMAN.** *PNAS USA,* 1988, vol. 85, 2444-2448
- **SMITH ; WATERMAN.** *J. Mol Biol.,* 1981, vol. 147, 195-197
- **VOET ; VOET.** Biochemistry. Wiley, 1995
- **GRAM et al.** *Proc. Natl. Acad. Sci., USA,* 1992, vol. 89, 3576-3580
- **BARBAS et al.** *Proc. Natl. Acad. Sci., USA,* 1994, vol. 91, 3809-3813
- **SCHIER et al.** *J. Mol. Biol.,* 1996, vol. 263, 551-567
- **MARKS et al.** *Bio/Technology,* 1992, vol. 10, 779-783
- **KAY, B.K. ; WINTER, J. ; MCCAFFERTY, J.** Phage Display of Peptides and Proteins: A Laboratory Manual. Academic Press, 1996

- **HUNTER W. M. ; GREENWOOD F. C.** *Nature,* 1962, vol. 194, 495
- **PLÜCKTHUN, A.** *Bio/Tcchnology,* 1991, vol. 9, 545-551
- **CHADD HE ; CHAMOW SM.** *Current Opinion in Biotechnology,* 2001, vol. 12, 188-194
- **ANDERSEN DC ; KRUMMEN L.** *Current Opinion in Biotechnology,* 2002, vol. 13, 117
- **LARRICK JW ; THOMAS DW.** *Current Opinion in Biotechnology,* 2001, vol. 12, 411-418
- **SAMBROOK ; RUSSELL.** Molecular Cloning: a Laboratory Manual. Cold Spring Harbor Laboratory Press, 2001
- Short Protocols in Molecular Biology: A Compendium of Methods from Current Protocols in Molecular Biology. John Wiley & Sons, 1999
- Sustained and Controlled Release Drug Delivery Systems. Marcel Dekker, Inc, 1978
- **LEDERMANN J.A. et al.** *Int. J. Cancer,* 1991, vol. 47, 659-664
- **BAGSHAWE K.D. et al.** *Antibody, Immunoconjugates and Radiopharmaceuticals,* 1991, vol. 4, 915-922
- **VAUGHAN, T. J. et al.** *Nature Biotechnology,* 1996, vol. 14, 309-314
- Generation of Naive Human Antibody Libraries. **HUTCHINGS, C.** Antibody Engineering. Springer Laboratory Manuals, 2001, 93